# EUROPEAN PATENT APPLICATION

(11) **EP 4 579 240 A1**
(43) Date of publication of application: **02.07.2025**
(21) Application number: 23814987.6
(22) Date of filing: 19.05.2023
(51) Int. Cl.: G01N 33/68

(54) **CF48 KIDNEY INJURY BIOMARKER AND USE THEREOF IN KIDNEY INJURY TREATMENT DRUG**

(30) Priority: 31.05.2022 CN 202210610636; 01.06.2022 CN 202210614388
(71) Applicant: Guangzhou Kangrun Biotechnology Co., Ltd., Guangzhou, Guangdong 510000 (CN)
(72) Inventor: LI, Jinhua, Guangzhou, Guangdong 510000 (CN); YANG, Jiayi, Guangzhou, Guangdong 510000 (CN); CHEN, Wei, Guangzhou, Guangdong 510000 (CN)
(74) Representative: Monteiro Alves, Inês
(86) International application number: PCT/CN2023/095186
(87) International publication number: WO 2023/231803

(57) **Abstract**

A use of a Cf48 small peptide, as a marker for kidney injury condition, in a diagnosis kit; a use of the Cf48 small peptide as a drug intervention target in the preparation of a drug for treating kidney injury; and a system for evaluating the kidney injury condition on the basis of Cf48. The sequence encoding the Cf48 small peptide is SEQ ID NO: 1. The Cf48 small peptide can be used as a marker for one or more of kidney disease diagnosis, kidney injury degree determination, prognosis prediction, renal fibrosis degree determination, and renal fibrosis activity determination. For a novel kidney disease injury diagnosis marker and a diagnosis kit, the kidney injury condition can be determined by detecting the content of Cf48 in kidney tissues and blood. The Cf48 small peptide has high specificity, the defect in the prior art that the kidney injury condition is basically determined by biopsy and pathology is overcome, the detection is convenient, and the result is accurate.

## Description

### TECHNICAL FIELD

This invention generally relates to the technical field of kidney disease treatment, and more particularly, to a C4orf48 (Cf48) small peptide used as a marker for kidney injury condition of a target object to be detected, a use of the Cf48 small peptide as a drug intervention target in the preparation of a drug for treating kidney injury, and a system for evaluating the kidney injury condition on the basis of Cf48.

### BACKGROUND

Kidney diseases, especially chronic kidney diseases (CKD), have increasingly affected the world's adult population. In China, the prevalence of CKD is 10.8%, and a considerable cases might deteriorate into end-stage renal disease (ESRD), resulting in the requirement of kidney replacement therapy.

Chronic kidney diseases (CKD) refer to the progressive declination of kidney function as well as structural and functional impairment of kidney caused by various reasons. It shows progressive and irreversible loss of nephrons, reduced ability of kidney regeneration, metabolic changes, oxidative stress and inflammation, which eventually leads to renal fibrosis. Renal fibrosis is the end result of various chronic kidney diseases and is also one of the leading causes of end-stage renal disease (ESRD). In fact, fibrosis is part of the normal repair process of the kidney. Under injury conditions, fibroblasts are capable of producing an appropriate amount of extracellular matrix (ECM) to maintain the integrity of tissue structure and function. However, when the injury exists chronically and the repair process is disrupted, the renal fibroblasts become activated or the epithelial cells trans-differentiate into myofibroblasts, which proliferate to produce excessive ECM to replace the original renal parenchymal tissues and finally lead to renal fibrosis. For example, podocyte loss replaced by ECM is called Glomerulosclerosis, and tubular cell injury and subsequent pathological deposition of ECM is called renal interstitial fibrosis (RIF). Compared with glomerulosclerosis, renal interstitial fibrosis is more strongly associated with a patient's proteinuria content and disease severity.

Acute kidney injury (AKI) refers to a rapid decline of kidney function over a short period of time, showing programmed cell death and excessive inflammatory response in the kidney. Patients suffering from CKD have an increased risk of being attacked by AKI, and an increasing number of epidemiological studies have shown that aging and CKD significantly increase the risk of being attacked by AKI. Similarly, severe or recurrent AKI may transition to CKD or even ESRD (known as AKI to CKD transition). Research reports show that there are over 5000 cases each year per million AKI patients who do not require dialysis, and a significant portion of them might deteriorate into to CKD or ESRD. The severity, frequency and duration of AKI are highly associated with subsequent incidence rate of CKD, and even mild AKI has the potential to deteriorate into renal fibrosis. Therefore, AKI is now recognized as an important risk factor for CKD and ESRD. However, in addition to supportive therapies, effective and specific treatment for delaying the deterioration of AKI diseases is now still in absence.

The progression of kidney diseases, especially chronic kidney diseases (CKD), is complex and heterogeneous. Presently, the clinical evaluation and diagnosis of kidney diseases primarily rely on the evaluation of glomerular function, namely, reflecting the kidney function through the evaluation of glomerular filtration condition. Over the past century, the diagnosis of kidney diseases has relied primarily on the detection of serum creatinine content. As a metabolite of creatine/creatine phosphate, serum creatinine is capable of being freely filtered by the glomerulus when the glomerular filtration function is normal. However, with the increasing sensitivity requirements for clinical diagnosis, using serum creatinine and other molecules as biomarkers of kidney injury for evaluating glomerular filtration function has been limited by the following: 1) poor diagnostic sensitivity: serum creatinine is an indirect marker of kidney injury, which may lead to delayed diagnosis; 2) poor diagnostic specificity: serum creatinine content may increase even when there is no injury to the glomeruli or tubules; 3) poor diagnostic directivity: whether the increase of serum creatinine content is caused by tubular injury or glomerular injury cannot be determined; (4) for patients having normal renal reserve function, serum creatinine content does not increase even if there is severe tubular injury.

To solve the prior problems, it is necessary to provide a biomarker in the blood or urine that is associated with kidney injury, especially a biomarker that is produced directly by the kidney or a biomarker having increased content in the blood or urine due to abnormal kidney function caused by kidney injury. Finding a biomarker having high specificity and potential therapeutic drug target during the occurrence and progression of kidney injury are of great significance.

### SUMMARY

The purpose of the present invention is to provide a use of a Cf48 small peptide as a marker for kidney injury condition in a diagnosis kit, a use of the Cf48 small peptide as a drug intervention target in the preparation of a drug for treating kidney injury and a system for evaluating the kidney injury condition on the basis of Cf48.

To achieve the above purposes, the present invention adopts the following technical solution: a use of a Cf48 small peptide as a marker for kidney injury condition, wherein the sequence encoding the the Cf48 small peptide is SEQ ID NO: 1.

In another preferred embodiment of the present invention, the Cf48 small peptide serves as a marker for kidney disease diagnosis, or the Cf48 small peptide serves as a marker for kidney injury degree determination, or the Cf48 small peptide serves as a marker for prognosis prediction, or the Cf48 small peptide serves as a marker for renal fibrosis degree determination, or the Cf48 small peptide serves as a marker for renal fibrosis activity determination, or the Cf48 small peptide serves as one or more markers for monitoring treatment effect on patients with kidney disease.

The present invention also provides a use of the Cf48 small peptide as a marker for kidney injury condition in a diagnosis reagent or diagnosis kit. The reagent or kit may be a reagent or kit that adopts mass spectrometry or immunodetection methods including but not limited to the enzyme-linked immunosorbent assay, chemiluminescence immunodetection, immunochip, fluorescence immunoassay, immunohistochemistry, immunochromatography, stress immunity and immunoturbidimetry, etc.

In another preferred embodiment of the present invention, by detecting the expression level (i.e. content) of the Cf48 small peptide in a biological sample, the expression level higher than a reference level indicates the presence of kidney injury. The sample may be kidney tissues, serum, plasma or whole blood.

In another preferred embodiment of the present invention, whether a target object has a kidney disease is determined by detecting the expression level of the Cf48 small peptide in the biological sample, or the kidney injury degree is determined by detecting the expression level of the Cf48 small peptide in the biological sample, or the prognosis prediction is performed by detecting the expression level of the Cf48 small peptide in the biological sample, or the renal fibrosis degree is determined by detecting the expression level of the Cf48 small peptide in the biological sample, or the renal fibrosis activity is determined by detecting the expression level of the Cf48 small peptide in the biological sample, or at least one method for monitoring the treatment effect on patients with kidney disease is performed by detecting the expression level of the Cf48 small peptide in the biological sample.

In another preferred embodiment of the present invention, whether there is renal tubular injury is determined based on the content of the Cf48 small peptide in a detected biological sample.

In another embodiment of the present invention, the higher the content of the Cf48 small peptide in the detected biological sample, the higher the kidney injury degree. The higher the content of the Cf48 small peptide in the detected biological sample, the more severe the prognosis prediction result, namely, the higher possibility that the kidney disease deteriorates. The higher the content of the Cf48 small peptide in the detected biological sample, the higher the renal fibrosis activity, namely, the higher the renal fibrosis degree.

In another preferred embodiment of the present invention, the content of the Cf48 small peptide in the detected biological sample is compared with a first threshold. When the content of the Cf48 small peptide in the detected biological sample is greater than or equal to the first threshold, it indicates that the renal fibrosis activity is high and an intervention is needed. When the content of the Cf48 small peptide in the detected biological sample is lower than the first threshold and greater than a second threshold, whether TGF-β1 exists in the biological sample is detected, and when TGF-β1 is found in the detected biological sample, it indicates that the renal fibrosis activity is high. When the content of the Cf48 small peptide in the detected biological sample is lower than the second threshold, it indicates that the renal fibrosis activity is low and the kidney fibrosis does not deteriorate, wherein the first threshold is greater than the second threshold.

The present invention also provide a use of the Cf48 small peptide as a drug intervention target in the preparation of a drug for treating kidney injury.

In another preferred embodiment of the present invention, the drug for treating kidney injury treats the kidney injury or delays the development of kidney injury by the targeted inhibition or elimination of the expression of the Cf48 small peptide.

In another preferred embodiment of the present invention, the drug for treating kidney injury inhibits the expression of the Cf48 small peptide in a targeted mode, thereby preventing the amino acid channel SLC3A in fibroblasts from being inhibited by the Cf48 small peptide such that the fibroblast activation is avoided.

In another preferred embodiment of the present invention, the drug for treating kidney injury inhibits the expression of the Cf48 small peptide in a targeted mode, thereby avoiding the induction of the EMT and promotion of the kidney fibrosis caused by Cf48 inhibiting the amino acid channel SLC3A2 and the energy metabolism in tubular cells.

In another preferred embodiment of the present invention, the drug for treating kidney injury inhibits the binding of the Cf48 small peptide and transferrin receptor protein 1 (TFRC), thereby avoiding the induction of EMT and promotion of renal fibrosis caused by Cf48 activating downstream signal channels of TFRC.

In another embodiment of the present invention, by means of the targeted inhibition of the expression of the Cf48 small peptide, the drug for treating kidney injury is capable of preventing the synergistic effect of the Cf48 and other factors that promote the formation of renal fibrosis.

The present invention also provides a system for evaluating the kidney injury condition on the basis of Cf48, comprising: a data acquisition module, configured to acquire at least a content of the Cf48 in a biological sample of a target object to be detected, and an evaluation module, configured to compare the content of the Cf48 in the biological sample with control parameters and give a level result corresponding to the kidney injury condition of the target object to be detected according to the comparison result.

In another preferred embodiment of the present invention, the evaluation module is provided with a parameter storage unit and an analysis unit, wherein the parameter storage unit stores a first reference value and a second reference value. The analysis unit compares the content of the Cf48 in the detected biological sample transmitted by the data acquisition module with the first reference value and the second reference value. When the content of the Cf48 small peptide in the detected biological sample is greater than or equal to the first reference value, the analysis unit gives a first-level result, and the first-level result indicates that the kidney injury condition is serious and needs to be intervened. When the content of the Cf48 small peptide in the detected biological sample is lower than the second reference value, the analysis unit gives a low-level result, and the low-level result indicates that the kidney injury condition is slight and an intervention is unnecessary, wherein the first reference value is greater than the second reference value.

In another preferred embodiment of the present invention, the data acquisition module is configured to acquire whether the TGF-β1 exists in the biological sample to be detected. The analysis unit compares the content of the Cf48 in the detected biological sample transmitted by the data acquisition module with the first reference value and the second reference value. When the content of the Cf48 small peptide in the detected biological sample is lower than the first reference value and greater than the second reference value, the analysis unit obtains a result of whether the TGF-β1 exists in the to-be-detected biological sample from the data acquisition module, and when the TGF-β1 exists in the biological sample to be detected, the analysis unit gives a second-level result, and the second-level result indicates that the kidney injury condition is serious and needs to be intervened.

Compared with the prior art, the present invention achieves the following benefits:

The present invention provides a novel kidney disease injury diagnosis marker and a diagnosis kit. The kidney injury condition can be determined by detecting the content of Cf48 in kidney tissues and blood. The Cf48 small peptide has high specificity. The defect in the prior art that the kidney injury condition is basically determined by biopsy and pathology is overcome. Meanwhile, the marker of the present invention is capable of identifying the variation degree of kidney injury, which compensates for the defect in the prior art that the variation of kidney injury condition cannot be determined. By using the Cf48 small peptide as a marker, the kidney injury condition can be determined without biopsy indicators, thereby breaking limitations of clinical diagnosis in the prior art. Moreover, the marker may be used as an indicator for physical examination.

The present invention also provides a drug for treating kidney injury using the Cf48 small peptide as a drug intervention target, which achieves inhibition or treatment of kidney injury by the targeted inhibition of the expression of the Cf48 small peptide.

The present invention also provides a system for evaluating kidney injury on the basis of Cf48. By acquiring the content of Cf48 in the biological sample of the target object, the system evaluates the kidney injury condition and gives a level result, achieving convenient detection and accurate results.

### BRIEF DESCRIPTION OF THE DRAWINGS

The present invention is further elaborated in combination with the drawings, but the content in the drawings does not constitute any limitation to the present invention.

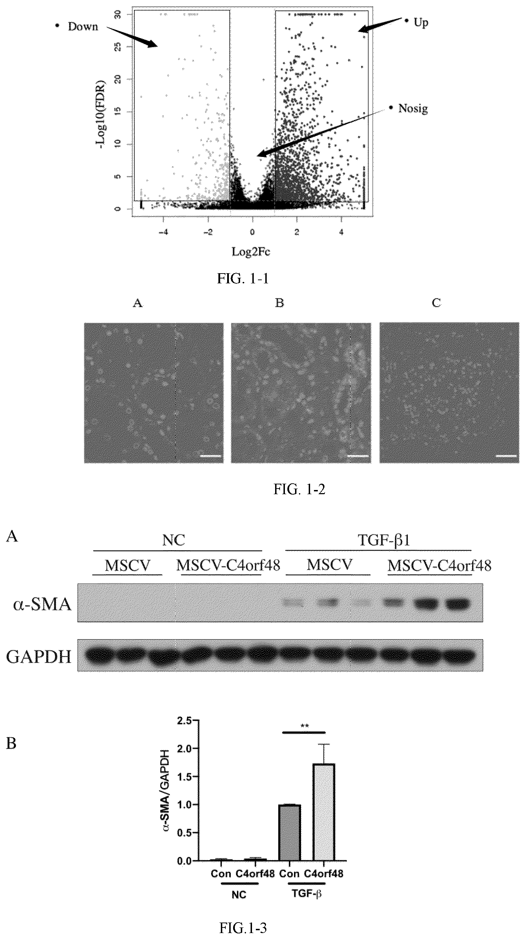

Figure 1-1 is a volcano plot illustrating differentially expressed genes in mice with progressive diabetic nephropathy (DN group) and normal controls (NC group);
Figure 1-2 is a schematic diagram illustrating the results of increased expression of Cf48mRNA in DN patients' renal tissues, wherein Figure 1-2A is a schematic diagram illustrating the expression of Cf48mRNA in normal renal tissues detected by using immunofluorescence in situ hybridization, Figure 1-2B is a schematic diagram illustrating the expression of Cf48mRNA in renal tubulointerstitium of patients with diabetic nephropathy, and Figure 1-2C is a schematic diagram illustrating the expression of Cf48mRNA in glomeruli of patients with diabetic nephropathy (plotting scale:50µm);
Figure 1-3 is a schematic diagram illustrating the results of Cf48 conditioned medium enhancing the activation response of TGF-β1-induced fibroblasts, wherein Figure 1-1-3A is a schematic diagram illustrating the results of western blot detection of the effects of the Cf48 conditioned medium (MSCV-Cf48) and control medium (MSCV) in the presence or absence of TGF-β1(1ng/mL) on the expression of α-SMA in NRK-49F cells, and Figure 1-3B is a schematic diagram illustrating the statistical analysis of the effect of the Cf48 conditioned medium on the expression of α-SMA in NRK-49F cells in three independent repeated experiments (n=3 and **P<0.01);

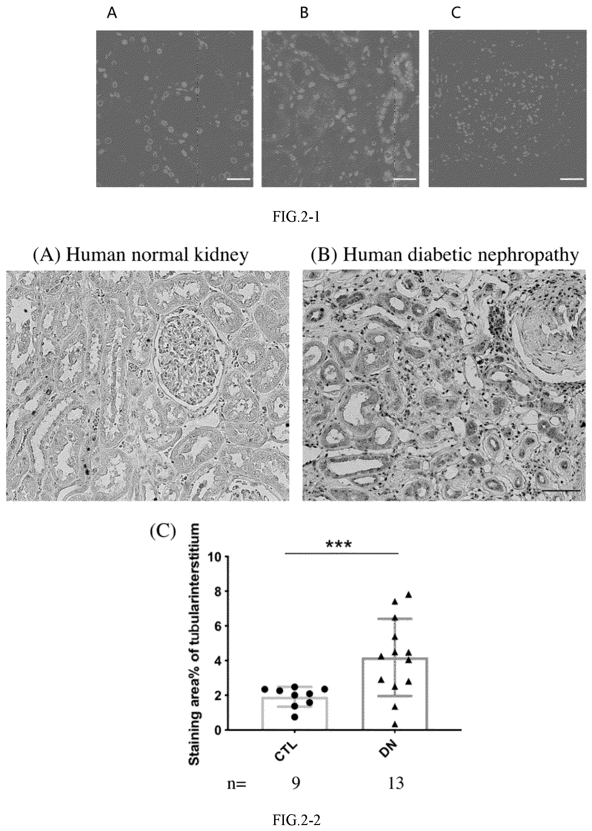

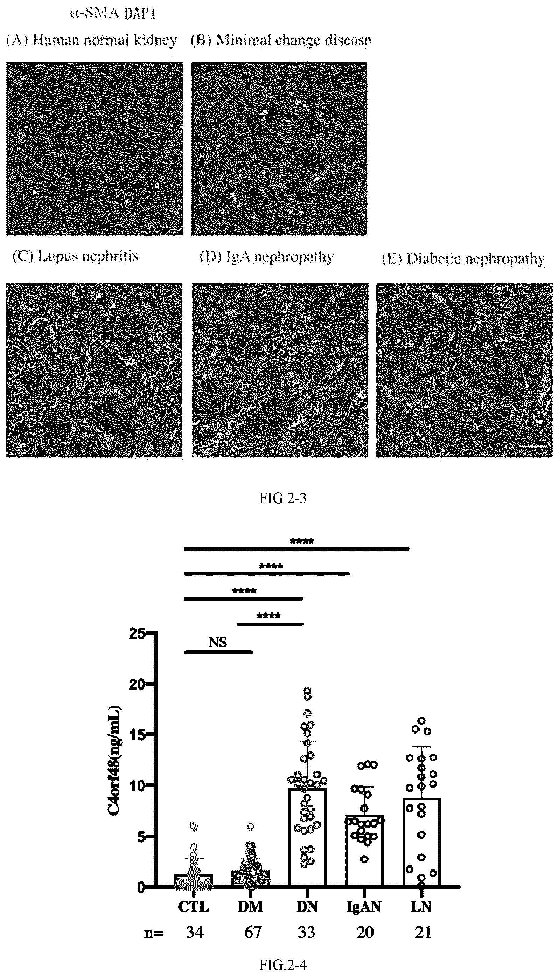

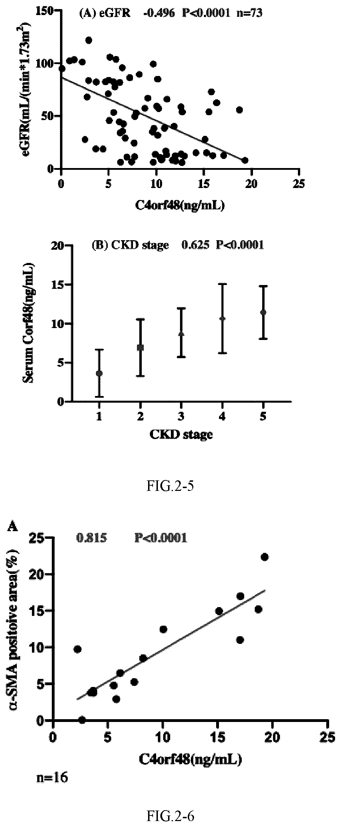

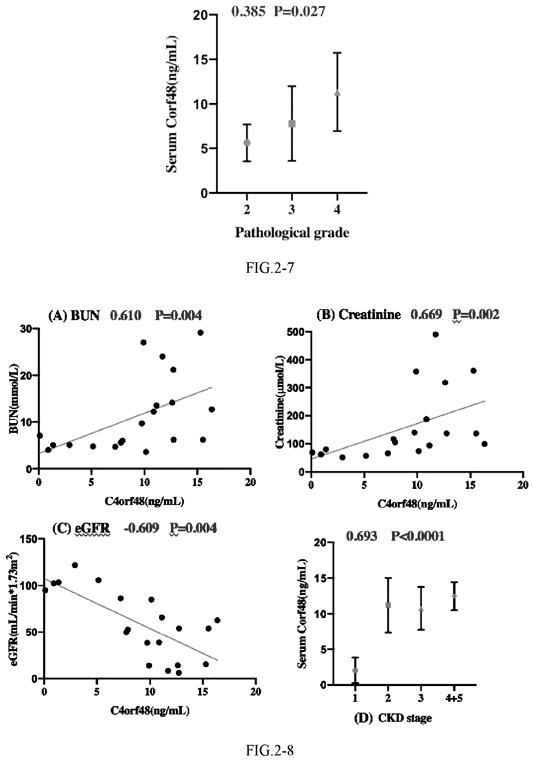

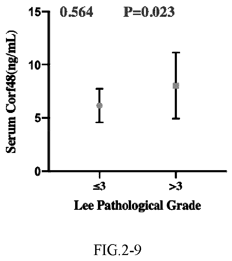

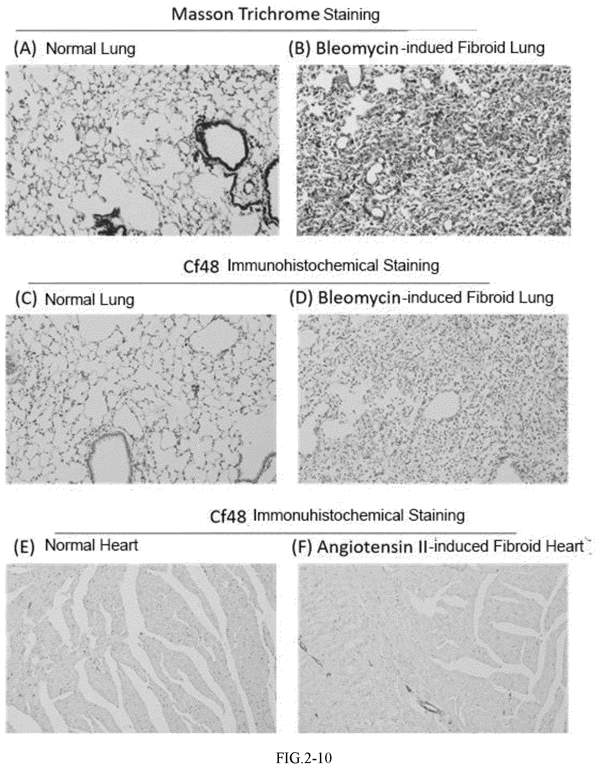

Figure 2-1 is a schematic diagram illustrating the results of increased expression of Cf48mRNA in DN patients' renal tissues, wherein Figures 2-1 A, 2-1B and 2-1C are schematic diagrams respectively illustrating the expression of Cf48mRNA detected by using immunofluorescence in situ hybridization in normal renal tissues, DN patients' tubulointerstitium and DN patients' glomeruli (plotting scale:50µm);
Figure 2-2 is a schematic diagram illustrating the results of increased expression of Cf48 in DN patients' renal tissues, wherein Figures 2-2A and 2-2B are schematic diagrams respectively illustrating the expression of Cf48 detected by using the immunohistochemical method in normal renal tissues and DN patients' renal tissues (plotting scale:500µm), and Figure 2-2C is a schematic diagram illustrating the expression intensity of Cf48 in renal tubulointerstitium analyzed by using immunohistochemical staining, wherein ***P<0.001, normal renal tissues (CTL, n=9), and DN patients' renal tissues (DN, n=13);

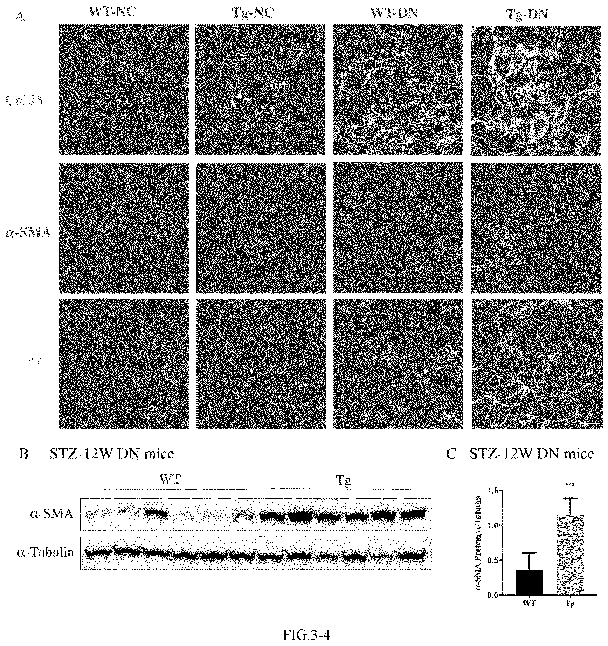

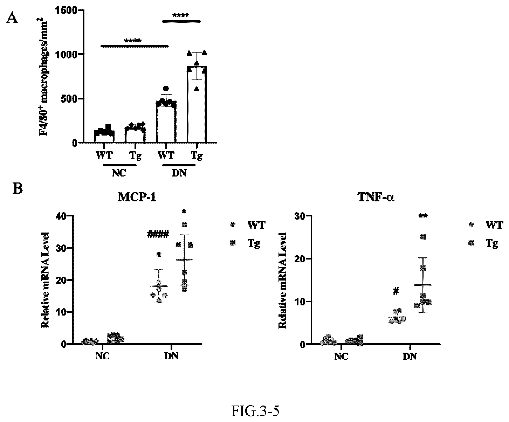

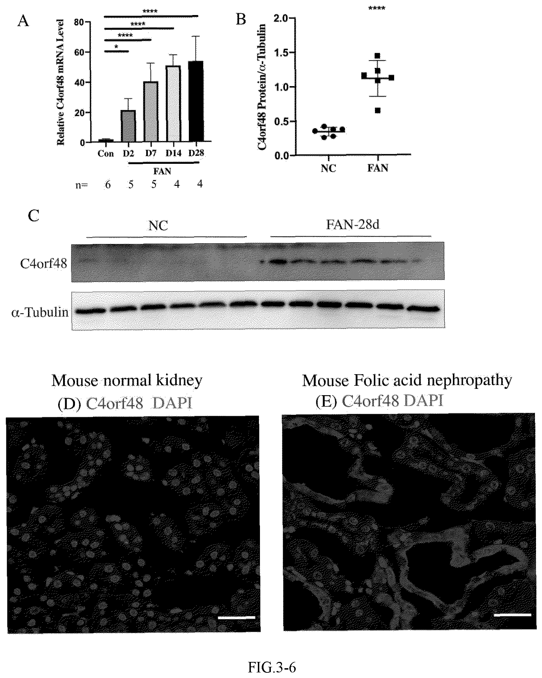

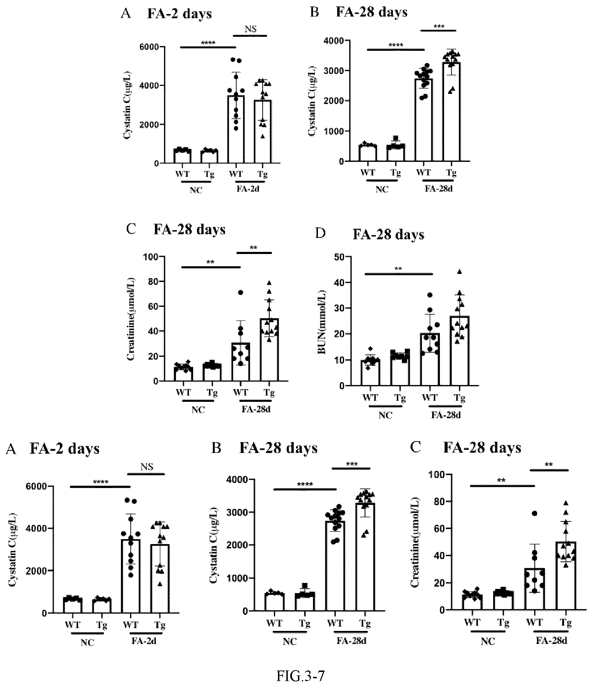

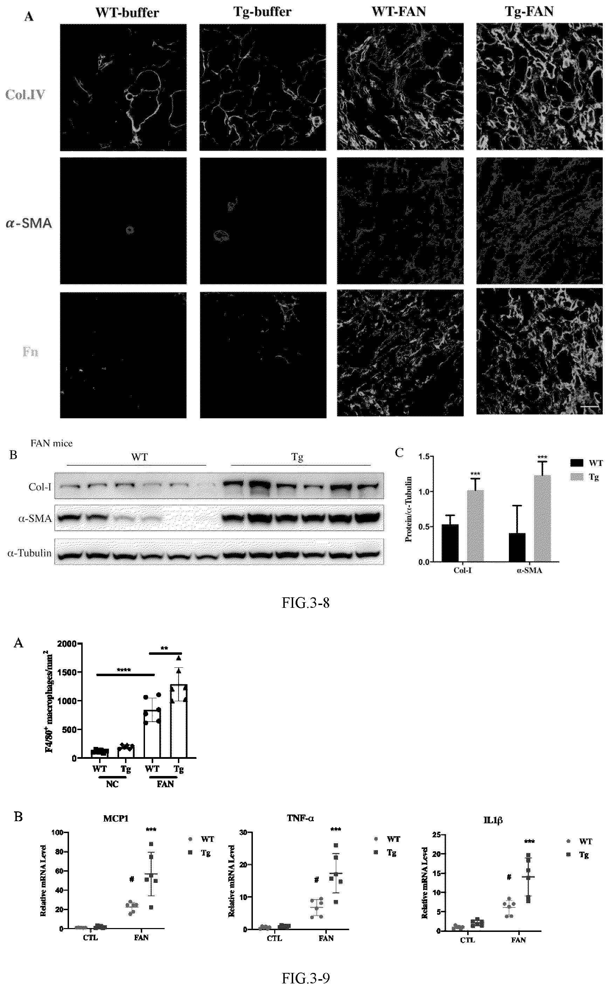

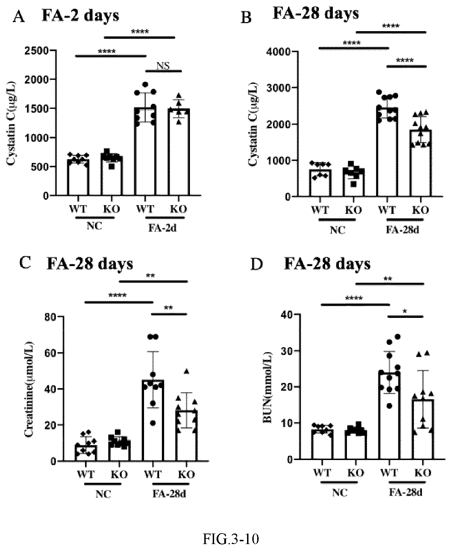

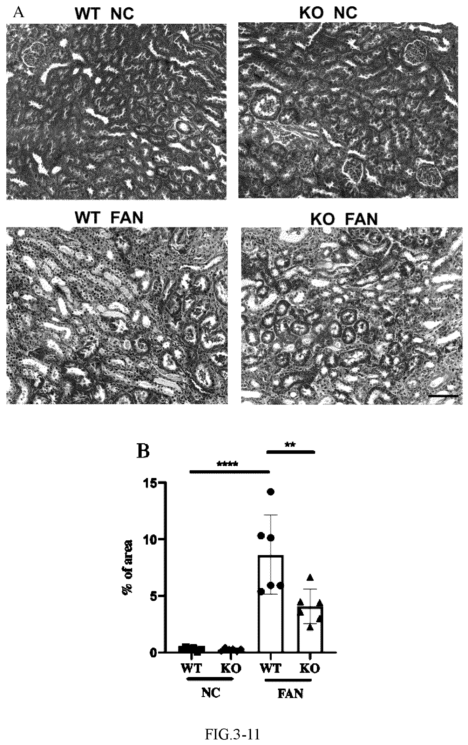

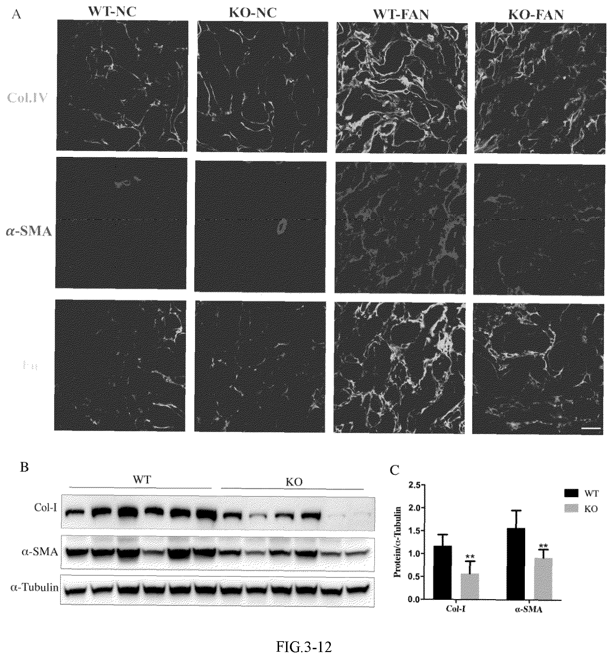

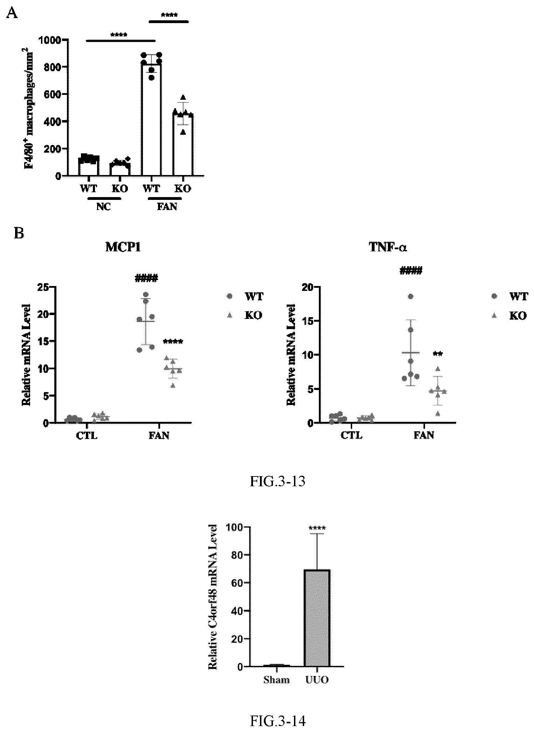

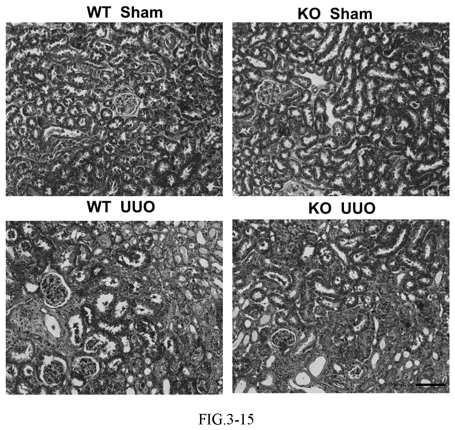

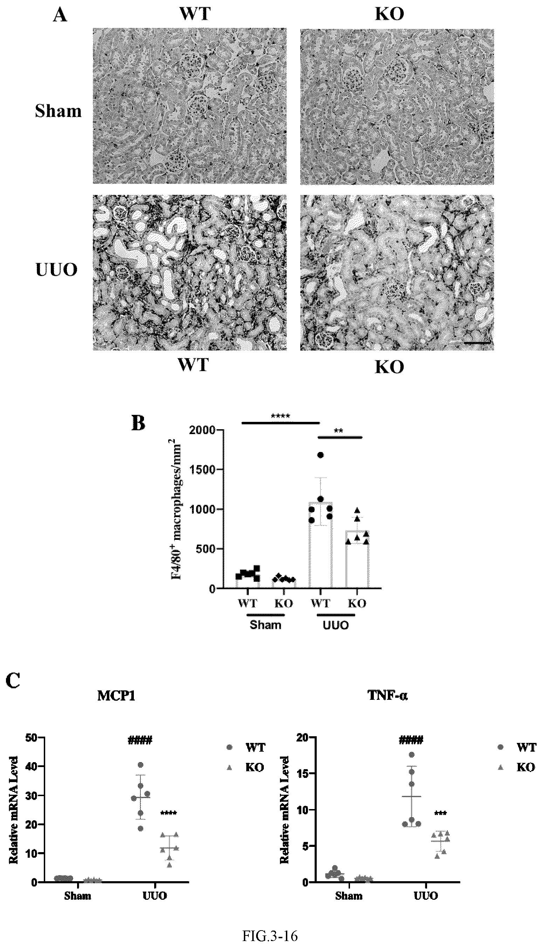

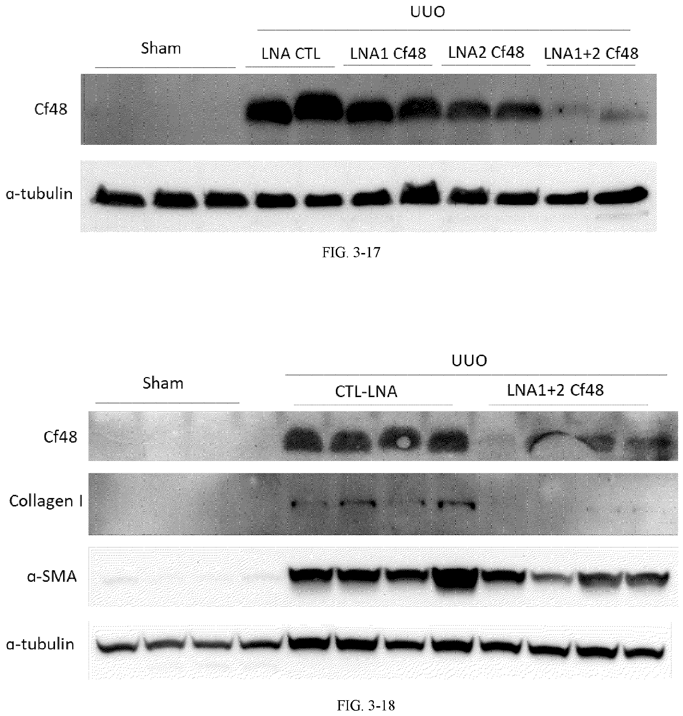

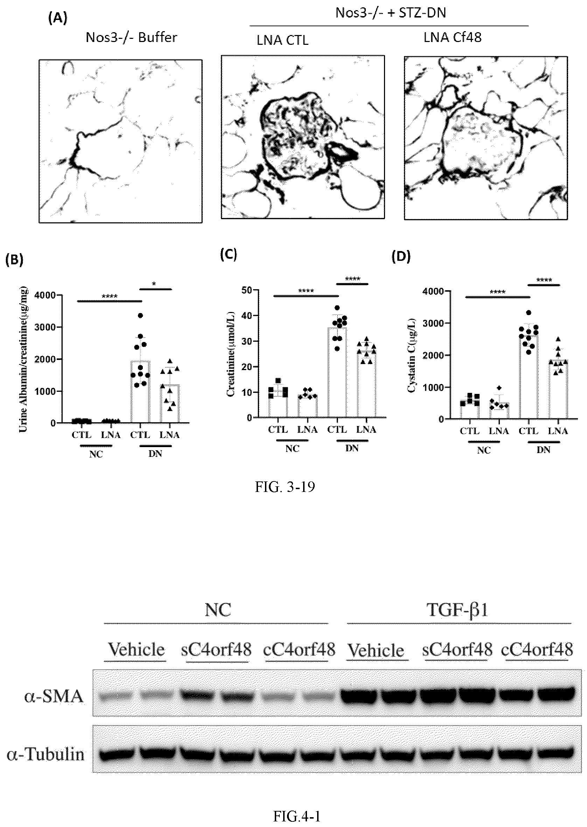

Figure 2-3 is a schematic diagram illustrating the results of the increased expression of Cf48 in CKD patients' renal tissues, wherein Figures 2-3A, B, C, D and E are diagrams respectively illustrating the expression of Cf48 in normal renal tissues and renal tissues of patients with minimal change disease, lupus nephritis, IgA nephropathy and diabetic nephropathy detected by using the immunofluorescence method (plotting scale:50µm);
Figure 2-4 is a schematic diagram illustrating the content of secretory Cf48 in the serum in peripheral blood of normal controls (CTL), disease controls with diabetic mellitus (DM) but without diabetic nephropathy, patients with diabetic nephropathy (DN), patients with IgA nephropathy (IgAN) and patients with lupus nephritis (LN), wherein NS=not significant; ****P<0.0001;
Figure 2-5 is a schematic diagram illustrating the analysis results of correlation between the Cf48 content in the CKD patients' serum and clinical indicators, wherein Figure 2-5A is a schematic diagram illustrating the correlation between the Cf48 content in CKD patients' serum and eGFR, and Figure 2-5B is a schematic diagram illustrating the correlation between the Cf48 content in CKD patients' serum and CKD staging, wherein n=73;
Figure 2-6 is a schematic diagram illustrating the correlation between the Cf48 content in DN patients' serum and α-SMA staining positive area of DN patients' renal tissues, wherein n=16;
Figure 2-7 is a schematic diagram illustrating the analysis on correlation between the Cf48 content in DN patients' serum and pathological grading, wherein n=33;
Figure 2-8 is a schematic diagram illustrating the analysis results of correlation between the Cf48 content in LN patients' serum and clinical indicators, wherein Figures 2-8A, B, C, and D are diagrams respectively illustrating the correlation between the Cf48 content in LN patients' serum and blood urea nitrogen, serum creatinine, eGFR and CKD grading, wherein n=20;
Figure 2-9 is a schematic diagram illustrating the analysis on correlation between the Cf48 content in IgAN patients' serum and the Lee pathological grading, wherein n=20;
Figure 2-10 is a schematic diagram illustrating the expression results of Cf48 in mice lungs and hearts, wherein Figures 2-10A and B are schematic diagrams respectively illustrating the normal mice lungs and fibrotic lungs induced by Belomycin for 4 weeks shown by the Masson Trichrome staining; Figures 2-10C, D, E, and F are diagrams respectively illustrating the expression of Cf48 in normal mice lungs, fibrotic lungs induced by Belomycin for 4 weeks, normal mice hearts (E), and fibrotic hearts induced by Angiotensin for 4 weeks by using the immunohistochemical method;

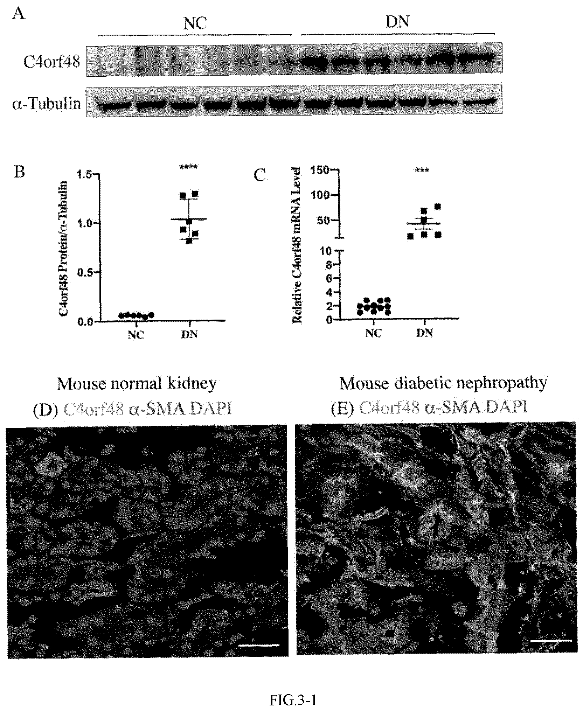

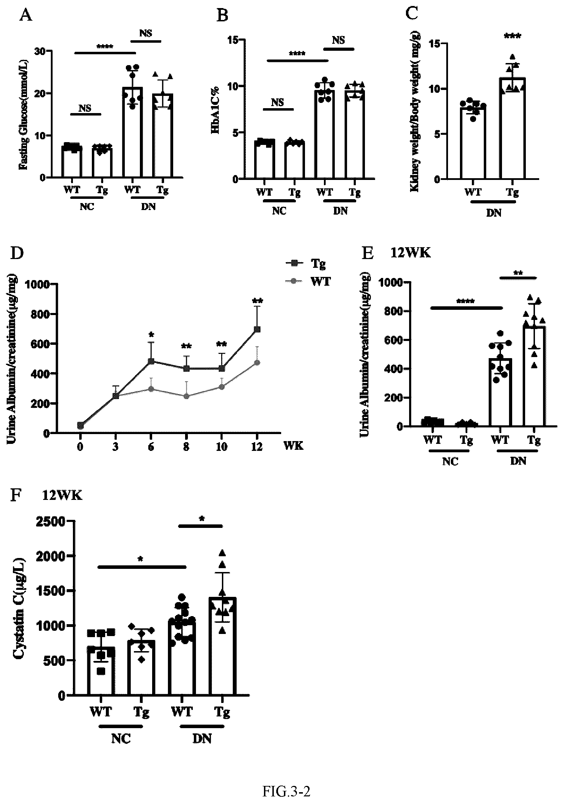

Figure 3-1 is a schematic diagram illustrating the up-regulation results of Cf48 in renal tissues of mice with diabetic nephropathy, wherein Figure 3-1A is a schematic diagram illustrating the western blot analysis (A) of Cf48 expression in renal tissues of normal control (NC) mice and mice with STZ-induced diabetic nephropathy (DN), Figure 3-1B is a schematic diagram illustrating the statistical analysis (A) of the Cf48 expression in renal tissues of mice in two groups ( *****P<0.0001,* n=6), Figure 3-1C is a schematic diagram illustrating the qRT PCR detection results of Cf48mRNA expression in renal tissues of NC and DN mice (****P*<0.001 , n=6), Figure 3-1D is a schematic diagram illustrating the expression of Cf48 and α-SMA in renal tissues of NC mice detected by using the immunofluorescence method, and Figure 3-2E is a schematic diagram illustrating the expression of Cf48 and α-SMA in renal tissues of DN mice detected by using the immunofluorescence method (plotting scale:50µm);
Figure 3-2 is a schematic diagram illustrating the declination of renal function of mice with Cf48 over-expression diabetic nephropathy, wherein Figure 3-2A is a schematic diagram illustrating the fasting blood glucose levels of mice in each group after 2 weeks of modeling (*****P*<0.0001, NS=not significant, n=6), Figure 3-2B is a schematic diagram illustrating the glycated hemoglobin levels of mice in each group after 4 weeks of modeling (*****P*<0.0001, NS=not significant, n=6), Figure 3-2C is a schematic diagram illustrating the kidney weight ratio of mice with diabetic nephropathy during the euthanasia (****P*<0.001, n=7), Figure 3-2D is a schematic diagram illustrating the random urinary albumin/creatinine ratio at each time point after successful modeling (0W) of DM mice ( **P*<0.05, ***P*<0.01*,* n=6), Figure 3-2E is a schematic diagram illustrating the random urinary albumin/creatinine ratio of mice in each group during the euthanasia (12W) (***P*<0.01, *****P<0.0001,* n=6), and Figure 3-2F is a schematic diagram illustrating the serum cystatin C levels of mice in each group during the euthanasia (12W) (**P*<0.05, n=6);

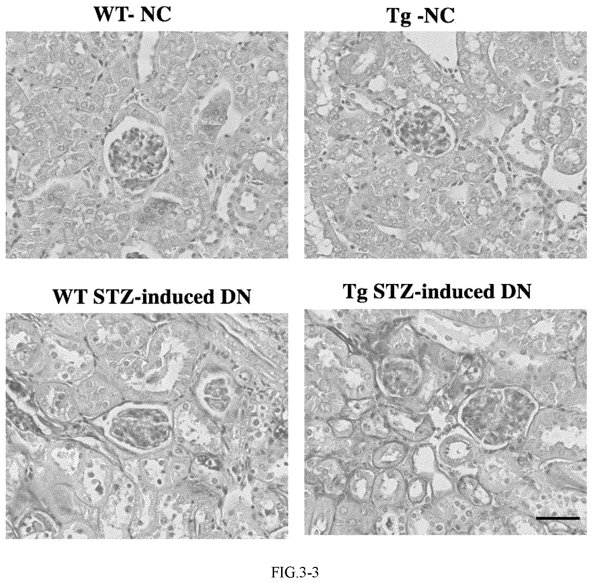

Figure 3-3 is a schematic diagram illustrating the deterioration of renal pathological injury in mice with Cf48 over-expression diabetic nephropathy, wherein in this figure, the kidney of mice in each group is stained using PAS (plotting scale: 50µm);
Figure 3-4 is a schematic diagram illustrating the deterioration of renal fibrosis in mice with Cf48 over-expression diabetic nephropathy, wherein Figure 3-4A is a schematic diagram illustrating the expression of fibrosis indicators in the renal tissues of mice in each group detected by using the immunofluorescence method (plotting scale: 50µm), Figure 3-4B is a schematic diagram illustrating the expression of α-SMA in renal tissues of mice with diabetic nephropathy analyzed by using the western blot, and Figure 3-4C is a schematic diagram illustrating the statistical analysis (B) of α-SMA expression in renal tissues of two groups of mice (****P*<0.001, n=6);
Figure 3-5 is a schematic diagram illustrating the exacerbation of renal inflammation in mice with Cf48 over-expression diabetic nephropathy, wherein Figure 3-5A is a schematic diagram illustrating the number of F4/80⁺ macrophages in the renal tissues of mice in each group ( *****P*<0.0001 , n=6), and Figure 3-5B is a schematic diagram illustrating the expression levels of inflammation indicators MCP-1 and TNF-α mRNA detected in the kidney tissues of mice in each group by using the qRT-PCR (^{####}*P*<0.0001 *vs* WT-NC mice, **P<0.05 vs* WT-DN mice, *^{#}P*<0.05 *vs* WT-NC mice, ***P<*0.01 *vs* WT-DN mice);
Figure 3-6 is a schematic diagram illustrating the up-regulation of Cf48 expression in renal tissues of mice with folic acid nephropathy, wherein Figure 3-6A is a schematic diagram illustrating the qRT-PCR detection results of Cf48mRNA expression levels in renal tissues of normal control mice (Con) and mice with folilc-acid- induced nephropathy at different time points (**P*<0.05, *****P*<0.0001), and Figure 3-6B is a schematic diagram illustrating the statistical analysis of Cf48 expression in renal tissues of mice with 28D folic acid nephropathy and normal control mice (*****P*<0.0001, n=6);
Figure 3-7 is a schematic diagram illustrating the declination of renal function of mice with Cf48 over-expression folic acid nephropathy, wherein Figure 3-7A is a schematic diagram illustrating the blood CysC levels of mice in each group after 2 days of the FA modeling (*****P*<0.0001, NS=not significant, n=6), Figure 3-7B is a schematic diagram illustrating the levels of CysC and (C) creatinine in the blood of mice in each group after 28 days of the FA modeling (***P*<0.01*,***P*<0.001*, ****P*<0.0001*,* n=6), Figure 3-7C is a schematic diagram illustrating the expression of Cf48 in the renal tissues of mice with 28d folic acid nephropathy and the normal control mice analyzed by using the western blot, and Figures 3-7D and 3-7E are schematic diagrams respectively illustrating the expression of Cf48 in renal tissues of the normal mice and the mice with folic acid nephropathy detected by the fluorescence method (plotting scale: 50µm);
Figure 3-8 is a schematic diagram illustrating the deterioration of renal fibrosis in mice with Cf48 over-expression folic acid nephropathy, wherein Figure 3-8A is a schematic diagram illustrating the expression of renal fibrosis indicators of mice in each group detected by using the immunofluorescence method (plotting scale: 50µm), Figure 3-8B is a schematic diagram illustrating the expression of ColI and α-SMA in renal tissues of mice with folic acid nephropathy analyzed by using the western blot, and Figure 3-8C is a schematic diagram illustrating the statistical analysis on the expression of ColI and α-SMA in renal tissues of two groups of mice (****P*<0.001, n=6);
Figure 3-9 is a schematic diagram illustrating the exacerbation of renal inflammation in mice with Cf48 over-expression folic acid nephropathy, wherein Figure 3-9A is a schematic diagram illustrating the statistical analysis of the number of F4/80⁺ macrophages in renal tissues of mice in each group (***P*<0.01, *****P<0.0001,* n=6), and Figure 3-9B is a schematic diagram illustrating the expression levels of inflammation indicators MCP-1, TNF-α and IL-1βmRNA detected by using qRT-PCR in the renal tissues of mice in each group (^{#}*P*<0.05 vs WT-NC mice, ****P*<0.001 *vs* WT-FA mice);
Figure 3-10 is a schematic diagram illustrating the results of reducing the degree of renal function impairment in mice with Cf48 knockout folic acid nephropathy, wherein Figure 3-10A is a schematic diagram illustrating the blood cystatin C levels of mice in each group after 2 days of the FA modeling (*****P<*0.0001, NS=not significant, n=6), and Figures 3-10B, C, and D are schematic diagrams respectively illustrating the levels of cystatin C, creatinine, and urea nitrogen in the blood of mice in each group after 28 days of the modeling *(*P<0.05, **P*<0.01, *****P*<0.0001, n=7);
Figure 3-11 is a schematic diagram illustrating the reduction of renal pathological injury in mice with Cf48 knockout folic acid nephropathy, wherein Figure 3-11A is a schematic diagram illustrating the Masson staining results of the kidneys of mice in each group (plotting scale: 500µm), and Figure 3-11B is a schematic diagram illustrating the proportion of collagen deposition in each group of mice by Masson staining (original magnification: ×400, ***P*<0.01*, ****P*<0.0001, n=6);
Figure 3-12 is a schematic diagram illustrating the results of the reduction of renal fibrosis degree in mice with Cf48 knockout folic acid nephropathy, wherein Figure 3-12A is a schematic diagram illustrating the expression levels of fibrosis indicators in renal tissues of mice in each group detected by using the immunofluorescence method (plotting scale: 500µm), Figure 3-12B is a schematic diagram illustrating the expression levels of ColI and α-SMA in renal tissues of mice with folic acid nephropathy analyzed by using the western blot, and Figure 3-12C is a schematic diagram illustrating the statistical analysis on the expression levels of ColI and α-SMA in renal tissues of two groups of mice (**P<0.01, n=6);
Figure 3-13 is a schematic diagram illustrating the results of the reduction of renal inflammation in mice with Cf48 knockout folic acid nephropathy, wherein Figure 3-13A is a schematic diagram illustrating the statistical analysis on the number of F4/80⁺ macrophages in renal tissues of mice in each group (*****P*<0.0001, n=6), and Figure 3-13B is a schematic diagram illustrating the expression levels of inflammation indicators MCP-1 and TNF-αmRNA in renal tissues of mice in each group detected by using the qRT-PCR (^{####}*P*<0.0001 *vs* WT-NC mice, ***P*<0.01, *****P<0.0001 vs* WT-FA mice);
Figure 3-14 is a schematic diagram illustrating the qRT-PCR detection results of the Cf48 mRNA expression levels in renal tissues of the sham mice (Sham) and UUO mice (*****P*<0.0001; n=6);
Figure 3-15 is a schematic diagram illustrating the Masson staining results of the kidneys of Cf48 knockout UUO mice in each group (plotting scale: 50µm);
Figure 3-16 is a schematic diagram illustrating the results of the reduction of renal inflammation in the Cf48 knockout UUO mice, wherein Figure 3-16A is a schematic diagram illustrating the expression of inflammatory cell marker F4/80 in renal tissues of each group of mice detected by using the immunohistochemical method (plotting scale: 50µm), Figure 3-16B is a schematic diagram illustrating the statistical analysis (A) on the number of F4/80⁺ macrophages in renal tissues of mice in each group (***P*<0.01 *, ****P<0.0001,* n=6), and Figure 3-16C is a scheamtic diagram illustrating the expression levels of inflammation indicators MCP-1 and TNF-αmRNA in renal tissues of mice in each group detected by using the qRT-PCR (^{####}*P*<0.0001 *vs* WT-Sham mice, ***P<0.001, *****P*<0.0001 *vs* WT-UUO mice);
Figure 3-17 is a schematic diagram illustrating the western blot results of the expression of Cf48 in renal tissues of mice in the sham group and UUO group after the LNA CTL or LNA Cf48 treatment;
Figure 3-18 is a schematic diagram illustrating the western blot results of the expression levels of Cf48, collagen I and α-SMA in renal tissues of mice in the sham group and UUO group after the LNA CTL or LNA Cf48 treatment;
Figure 3-19 is a schematic diagram illustrating the LNA Cf48 inhibiting the occurrence and progression of diabetic nephropathy, wherein Figure 3-19A is a schematic diagram illustrating the immunoflurescence results of the expression of collagen IV in renal tissues after 6 weeks of treatment with LNA CTL or LNA Cf48 immediately after 2 weeks of treatment with NOS3-/- Buffer (control group) and induction of DM (diabetes mellitus) with NOS3-/-+STZ, and Figures 3-19B, 3-19C, and 3-19D are schematic diagrams respectively illustrating the immunoflurescence results of the levels of proteinuria, blood Creatinine and blood Cystatin c after 6 weeks of treatment with LNA CTL or LNA Cf48 immediately after 2 weeks of treatment with NOS3-/- buffer (control group) and induction of DM (diabetes mellitus) with NOS3-/-+STZ (expression of data is Mean±SD. One-way ANOVA, *P<0.05;****P<0.0001);

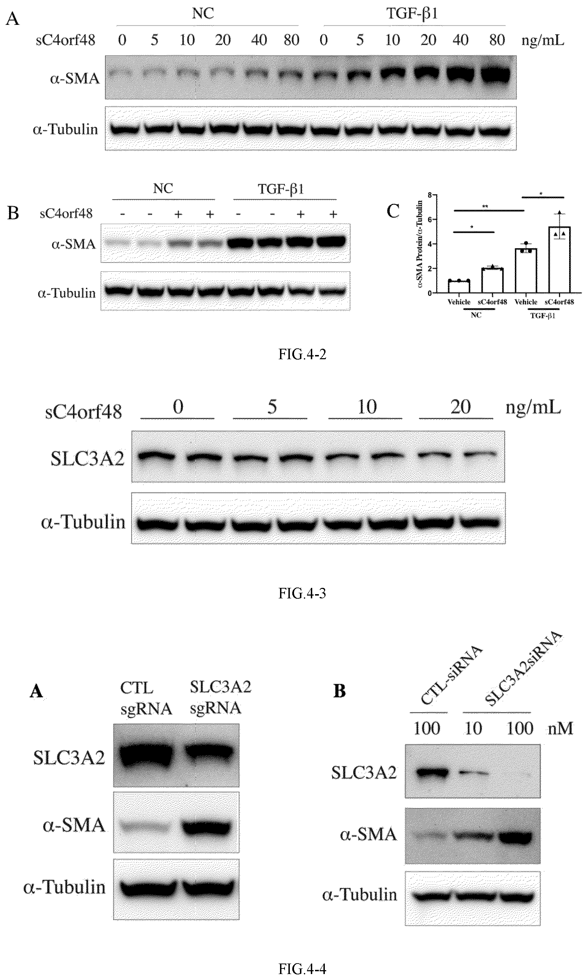

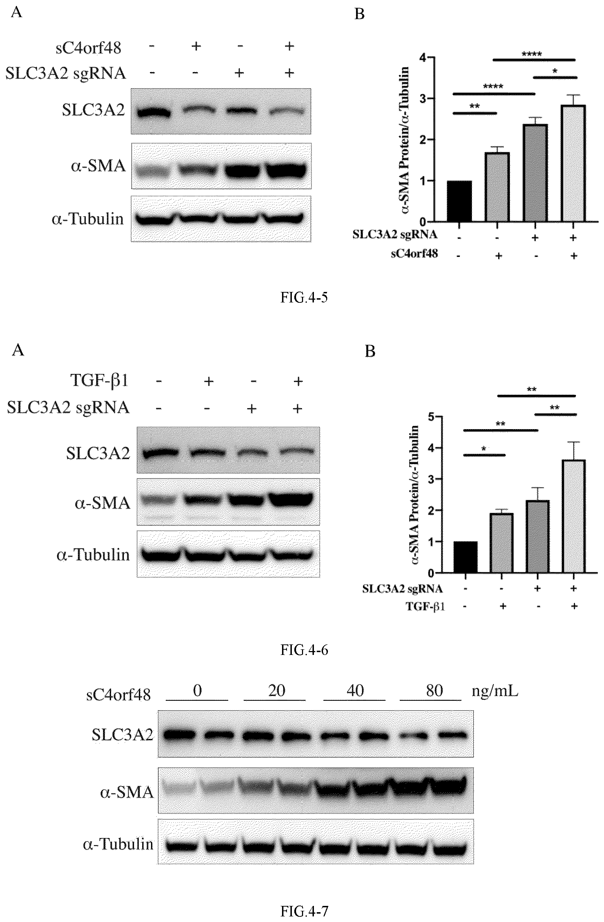

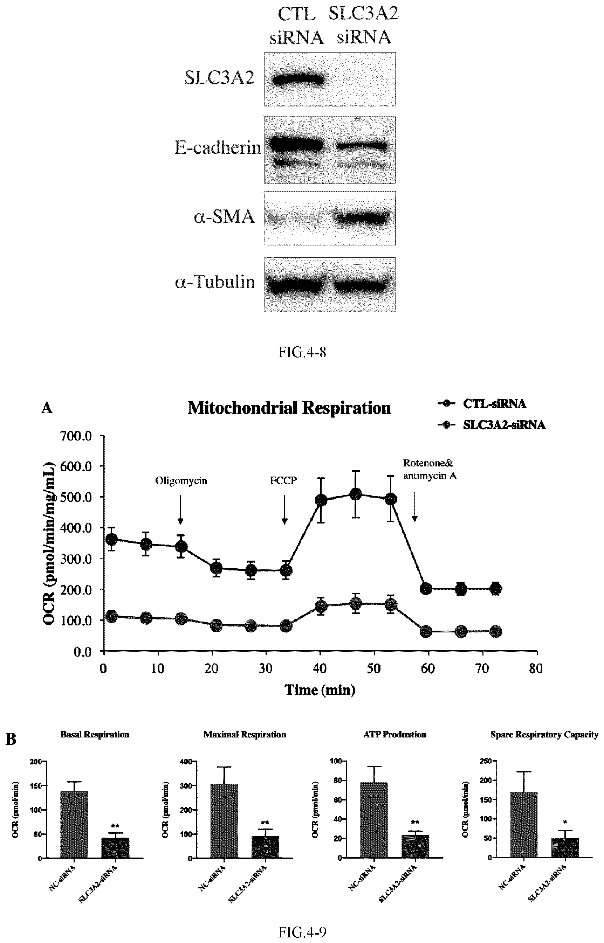

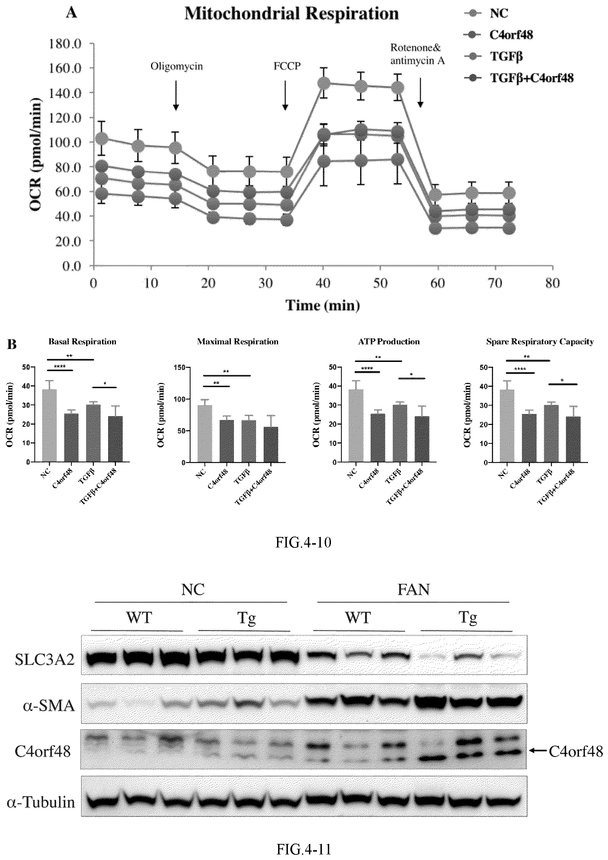

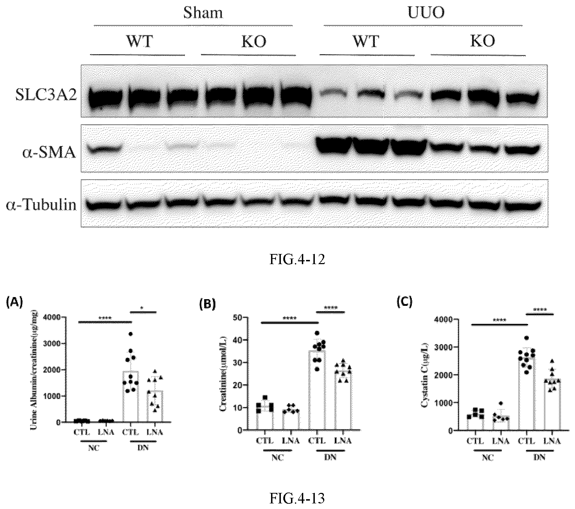

Figure 4-1 is a schematic diagram illustrating the expression of α-SMA in NRK-49F cells after 48 hours of stimulation using the secretory Cf48 (sCf48) and cellular Cf48 (cCf48) in the presence or absence of TGF-β1(1ng/mL);
Figure 4-2 is a schematic diagram illustrating the results of the concentration dependent promotion of fibroblast activation by the secretory Cf48, wherein Figure 4-2A is a schematic diagram illustrating the effect on the expression level of α-SMA when the secretory Cf48 (sCf48) with different concentrations is added to stimulate NRK-49F cells for 48 hours in the presence or absence of TGF-β1 (1ng/mL), Figure 4-2B is a schematic diagram illustrating the effect on the expression level of α-SMA when 40 ng/mL secretory Cf48 (sCf48) is added to stimulate NRK-49F cells for 48 hours in the presence or absence of TGF-β1 (1ng/mL), and Figure 4-2C is a schematic diagram illustrating the statistical analysis on the effect of 40 ng/mL secretory Cf48 (sCf48) on the expression of α-SMA in NRK-49F cells in three independent repeated experiments ( *P<0.05, **P<0.01, n=3);
Figure 4-3 is a schematic diagram illustrating the expression of SLC3A2 in NRK-49F cells after 48 hours of stimulation using the secretory Cf48 (sCf48) with different concentrations;
Figure 4-4 is a schematic diagram the results that the down-regulation of SLC3A2 expression promotes the fibroblast activation, wherein Figure 4-4A is a schematic diagram illustrating the protein levels of SLC3A2 and α-SMA in NRK-49F cells infected with CTL-sgRNA and SLC3A2-sgRNA detected by the western blot, and Figure 4-4B is a schematic diagram illustrating the protein levels of SLC3A2 and α-SMA in NRK-49F cells transfected with CTL-siRNA and SLC3A2-siRNA with different concentrations detected by the western blot;
Figure 4-5 is a schematic diagram illustrating the results that the down-regulation of SLC3A2 expression enhances the promoting effect of sCf48 on fibrosis, wherein Figure 4-5A is a schematic diagram illustrating the protein levels of SLC3A2 and α-SMA in NRK-49F cells transfected with CTL-sgRNA and SLC3A2-sgRNA detected by the western blot after 48 hours of stimulation with 40ng/mL sCf48, and Figure 4-5B is a schematic diagram illustrating the protein levels of α-SMA in three independent repeated experiments ( ***P*<0.05, ***P*<0.01, *****P*<0.0001, n=3);
Figure 4-6 is a schematic diagram illustrating the result that the down-regulation of SLC3A2 expression enhances the effect of TGF-β1 on the fibroblast activation, wherein Figure 4-6A is a schematic diagram illustrating the protein levels of SLC3A2 and α-SMA in NRK-49F cells transfected with CTL-sgRNA and SLC3A2-sgRNA detected by the western blot after 48 hours of stimulation with 1ng/mL TGF-β1, and Figure 4-6B is a schematic diagram illustrating the statistical analysis on the protein levels of α-SMA in three independent repeated experiments (**P*<0.05*, **P*<0.01*,* n=3);
Figure 4-7 is a schematic diagram illustrating the expression of SLC3A2 and α-SMA in NRK-52E cells stimulated by the secretory Cf48 (sCf48) at different concentrations for 5 days;
Figure 4-8 is a schematic diagram illustrating the protein levels of SLC3A2, E-cadherin and α-SMA in NRK-52E cells transfected with CTL-siRNA and SLC3A2-siRNA detected after 48 hours by using the western blot;
Figure 4-9 is a schematic diagram illustrating the down-regulation of SLC3A2 expression leading to a declination of tubullar cell mitochondrial function, wherein Figure 4-9A is a schematic diagram illustrating the real-time variation of oxygen consumption rate (OCR) of rat renal tubular cells treated with oligomycin, FCCP and rotenone after the CTL-siRNA and SLC3A2-siRNA treatment, and Figure 4-9B is a schematic diagram illustrating the results of quantitative analysis on the basal respiration rate, maximum respiration rate, ATP production capacity and reserve respiratory capacity of two groups of cells (*P<0.05, ***P*<0.01);
Figure 4-10 is a schematic diagram illustrating the declination of the mitochondrial function caused by the sCf48 in tubular cells, wherein Figure 4-10A is a schematic diagram illustrating the real-time variations of oxygen consumption rate (OCR) of rat renal tubular cells treated with oligomycin, FCCP and rotenone in the normal control (NC) group, sCf48 stimulation group, TGF-β1 stimulation group and TGF-β1+sCf48 stimulation group, and Figure 4-10B is a schematic diagram illustrating the quantitative analysis on the basal respiratory rate, maximum respiratory rate, ATP production capacity and reserve respiratory capacity of four groups of cells (**P*<0.05, ***P*<0.01, **** *P*<0.0001, n=6);
Figure 4-11 is a schematic diagram illustrating the expression levels of SLC3A2, α-SMA, and Cf48 in renal tissues of Cf48-Tg and WT mice in the normal control model and folic acid nephropathy model analyzed by using the western blot;
Figure 4-12 is a schematic diagram illustrating the expression levels of SLC3A2 and α-SMA in renal tissues of Cf48-KO and WT mice in the sham group model and UUO (unilateral ureteral obstruction) nephropathy model analyzed by using the western blot; and
Figure 4-13 is a schematic diagram illustrating the role of the TFRC in mediating the Cf48 fibrosis response, wherein the TFRC-siRNA is used to knock down the endogenous TFRC expression in NRK49F cells, and the results of the western blot show a decrease in the Cf48 induced α-SMA expression and the mediation of the TFRC in the Cf48 fibrosis response.

### DETAILED DESCRIPTION

Detailed embodiments are combined hereinafter to further elaborate the technical solution of the present invention.

### Embodiment 1

This embodiment provides a C4orf48 (Cf48) small peptide used as a marker for determining kidney injury condition of a target object to be detected. The sequence encoding the Cf48 small peptide is SEQ ID NO: 1.

The Cf48 small peptide is the abbreviation for C4orf48 small peptide. The human gene C4orf48 (NCBI Gene ID: 401115) is located in the 48th open reading frame of chromosome 4 (Chr4:2,035,610-2,043,970) and consists of 9 exons, which is capable of translating a peptide with an amino acid length of 90aa. The amino acid sequence is as follows:

The amino acid sequence of human gene C4orf48 is:

MAPPPACRSPMSPPPPPLLLLLLSLALLGARARAEPAGSAVPAQSRPCVDCH
AFEFMQRALQDLRKTACSLDARTETLLLQAERRALCACWPAGH (95aa).

The cDNA sequence of human gene C4orf48 is: 
ATGGCCCCCCCGCCCGCGTGCCGGTCCCCGATGTCACCGCCGCCGC
CGCTGCTGCTGCTGCTGCTGAGTCTGGCGCTGCTGGGCGCCCGGGCCCGCGCC
GAGCCCGCCGGGAGTGCCGTCCCCGCGCAGAGCCGCCCATGCGTGGACTGCCA
CGCCTTCGAGTTCATGCAGCGCGCCCTGCAGGACCTGCGGAAGACAGCCTGCA
GCCTGGACGCGCGGACGGAGACCCTACTGCTGCAGGCAGAGCGCCGTGCCCTG
TGTGCCTGCTGGCCAGCGGGGCACTGAG.
 (Note: underlined: signal peptides, other sequences: secretory peptides).

Experiments show that Cf48 small peptide is capable of serving as a marker for the diagnosis of kidney diseases. By detecting the content of the Cf48 small peptide in the sample of a target object, whether the target object suffers from a kidney disease is determined. Specifically, the expression of the Cf48 small peptide in the target object's tissues or blood allows whether the target object suffers from the kidney disease to be determined.

The Cf48 small peptide may also serve as a marker for determining the kidney injury degree. By detecting the expression of the Cf48 small peptide in the target object's tissues or blood, the kidney injury degree is determined based on the detected content of the Cf48 small peptide, including renal structural injury (e.g., structural changes), renal function injury (e.g., filtration function injury) and renal inflammation.

The Cf48 small peptide may also be used a marker for predicting the prognosis of the target object. The prognosis prediction of the target object represents the development ability of the current kidney injury. If the current development ability of kidney injury is high, it indicates a stronger ability to develop into a more severe stage. If the current development ability of kidney injury is low, it indicates that the current kidney injury does not have the ability to develop into a more severe stage. The current kidney injury condition is basically maintained or alleviated. By detecting the expression of the Cf48 small peptide in the target object's tissues or blood, the kidney injury degree is determined and the results of prognosis prediction are obtained.

The Cf48 small peptide also serves as a marker for the renal fibrosis degree, which is an important representation of kidney injury especially kidney diseases.

The Cf48 small peptide is also used as a marker for the renal fibrosis activity. The renal fibrosis activity is a representation of the current renal fibrosis degree. A high fibrosis activity indicates a high renal fibrosis degree, showing that the renal fibrosis rapidly occurs and a timely intervention is needed. A low fibrosis activity indicates a low development ability of renal fibrosis, showing that the renal fibrosis is less likely to occur. Under such circumstances, the current condition is maintained or the fibrosis is reduced, and there is no need to intervene.

The Cf48 small peptide may also serve as a marker for monitoring the therapeutic effect on patients with kidney diseases. By using the Cf48 small peptide, the therapeutic effect on patients is monitored.

The Cf48 small peptide is a novel kidney disease injury diagnosis marker. The kidney injury condition may be determined by detecting the content of Cf48 in kidney tissues and blood. The defect in the prior art that the kidney injury condition is basically determined by biopsy and pathology is overcome. Meanwhile, the novel marker may also be used to determine the variation degree of kidney injury, which compensates for the prior defect that the variation of kidney injury cannot be judged. By using the Cf48 small peptide as a marker to represent kidney injury, the kidney injury condition determination without biopsy indications is realized, which overcomes defects of clinical diagnosis in the prior art and is capable of being detected as an indicator for physical examination.

### Embodiment 2

This embodiment provides the Cf48 small peptide used as a marker for determining kidney injury condition in a diagnosis kit. By detecting the expression level of the Cf48 small peptide in biological samples, when the expression level is higher than the reference, the risk of kidney injury is indicated. The samples may be selected from kidney tissues, serum, plasma or whole blood.

More specifically, the Cf48 small peptide may be used as a marker for the kidney disease diagnosis, and the expression level of the Cf48 small peptide in biological samples may be detected to determine whether the target object suffers from a kidney disease; alternatively, the Cf48 small peptide may be used as a marker to evaluate the kidney injury degree by means of detecting the expression level of the Cf48 small peptide in biological samples; alternatively, the Cf48 small peptide may be used as a marker for predicting the prognosis of kidney injury by detecting the expression level of the Cf48 small peptide in biological samples; alternatively, the Cf48 small peptide may be used as a marker for renal fibrosis degree, and the expression level of the Cf48 small peptide in biological samples may be detected to determine the renal fibrosis degree; alternatively, the Cf48 small peptide may be used as a marker for renal fibrosis activity, and the expression level of the Cf48 small peptide in biological samples may be detected to determine the renal fibrosis activity; alternatively, the Cf48 small peptide may be used as a marker for monitoring the therapeutic effect on patients with kidney disease, and by detecting the expression level of the Cf48 small peptide in biological samples, the therapeutic effect on patients with kidney disease is monitored.

Specifically, the expression level of the Cf48 small peptide in biological samples may be determined based on the content of the Cf48 small peptide in the detected biological samples.

Experimental results show that, the higher the content of the Cf48 small peptide in the detected biological samples, the higher the kidney injury degree, and the greater the prognosis prediction value of the target object. Namely, the higher the possibility that the kidney disease deteriorates into a more severe stage. Meanwhile, the higher the content of the Cf48 small peptide in the detected biological samples, the higher the renal fibrosis activity, and the higher the renal fibrosis degree in the to-be-detected object's kidney. When the content of the Cf48 small peptide in the detected biological samples is high, there is injury to the renal tubules.

In practical use, corresponding threshold ranges may be set according to specific objects and detection requirements for determining the kidney injury condition. For example, the content of the Cf48 small peptide in the detected biological samples may be compared with a first threshold, and when the content of the Cf48 small peptide in the detected biological samples is greater than or equal to the first threshold, it indicates that the renal fibrosis activity is strong and the renal fibrosis degree is high such that an intervention is needed.

When the content of the Cf48 small peptide in the detected biological samples is lower than the first threshold and greater than the second threshold, the presence of TGF-β1 in the biological samples is detected, and when TGF-β1 is present in the detected biological samples, it indicates that the renal fibrosis activity is strong.

When the content of the Cf48 small peptide in the detected biological samples is lower than the second threshold, it indicates that the renal fibrosis activity is weak, the renal fibrosis degree is low, and the renal fibrosis does not deteriorate.

The first threshold is greater than the second threshold. It is worth mentioning that the first threshold and the second threshold may be set according to different purposes or requirements. For example, the range of values for the first threshold is not lower than 40ng/mL, and the range of values for the second threshold is from 0ng/mL to 6ng/mL.

By means of the kidney disease injury diagnosis marker and the diagnosis kit of the present invention, the kidney injury condition is determined by detecting the content of Cf48 in kidney tissues and blood. Thus, the defect in the prior art that the kidney injury condition is basically determined by biopsy and pathology is overcome. Meanwhile, the marker may also be used to determine the variation degree of kidney injury, which compensates for the prior defect that the variation of kidney injury cannot be judged. By using the Cf48 small peptide as a marker to represent kidney injury, the kidney injury condition determination without biopsy indications is realized, which overcomes defects of clinical diagnosis in the prior art and is capable of being detected as an indicator for physical examination. The diagnosis kit of the present invention may be used as a kit for various immunological diagnosis, such as immunohistochemmistry, chemiluminescent immunoassay, ELISA and western blot, etc.

### Embodiment 3

The present invention is further elaborated based on the specific experimental process.

### 1. Obtaining Cf48

### 1. 1 Research purpose

Translatomics sequencing is used to screen out novel small peptides having specificity and encoded by sORF in the progression of CKD.

### 1.2 Experimental materials

1) NRK-49F cell line and human embryonic kidney cell (293T), purchased from ATCC (American Type Culture Collection, USA); 2) streptozotocin, purchased from Sigma, USA; 3) recombinant TGF-β1 stimulating factor, purchased from Peprotech, USA; 4) serum-free expression medium, purchased from Thermo, USA; 5) RIPA cell lysate, purchased from Milipore, USA; 6) BCA kit, purchased from Thermo, USA; 7) antibodies: α-SMA antibodies purchased from Sigma Corporation (USA), GPADH antibodies purchased from SAB Corporation (China), and goat anti-rabbit antibodies purchased from Cell Signaling Technology Corporation (USA).

### 1.3 Research method

### 1.3.1 Experimental animals

*eNOS -*/*-* mice with C57BL/6 background, purchased from GemPharmatech (Jiangsu). The model of diabetic nephropathy is constructed in the Experimental Animal Center of Sun Yat-sen University (North Campus).

### 1.3.2 Preparation of animal model

The induced progressive diabetes mouse model: streptozotocin (STZ) is given to *eNOS -*/*-* mice to induce diabetic nephropathy. Partial damage to mice pancreatic islet cells is caused by continuous low-dose intraperitoneal injection for 5 days. After 12 weeks of successful modeling, mice with induced DN (DN group) are euthanized, and *eNOS -*/*-* mice are given the same dose of solvent, euthanized within the same period of time and are used as the normal control group (NC group).

### 1.3.3 Sequencing object

The renal tissues of 5 *eNOS -*/*-,* STZ induced mice with progressive diabetic nephropathy and 4 age matched *eNOS -*/*-* mice in the normal control group are taken for performing translatomics sequencing.

### 1.3.4 translatomics sequencing

The translatomics sequencing and relevant analysis are performed by Gene Denovo (Guangzhou). The main processes include translation inhibition fixation (actidione is added), RNAse digestion, ribosome removal, ribosome footprint extraction, reverse transcription, library construction, quality inspection and sequencing.

### 1.3.5 Steps of analysis

### 1) Calculating the ribosomal footprint translation abundance

### a) Comparing and identifying the ribosomal footprint (RFs):

Ribosomal footprint refers to the position where ribosome complexes slide and stay on RNA during translation. When the translation is stopped, only the mRNA fragments covered by ribosomes are retained when the ribosome newborn peptide chain complexes are treated using low concentration RNAse. After removing the ribosomes, these small RNA fragments having a length of about 30bp and protected by ribosomes are called ribosome footprints. Each RFs represents a ribosome position, representing the transcript being translated.

RFs are sequenced directly by second-generation sequencing to obtain RF reads. The RF reads are compared with the mice genome by using the bowtie2 software, and only the RF reads that are uniquely matched to a gene are retained for subsequent quantitative analysis.

### b) Calculating the open reading frame (ORF) translation expression:

Based on the comparison results of RFs, the abundance of RF reads falling within each ORF (the number of RF reads that overlap with ORFs) are further counted, and the expression of ORFs is represented by using RPKM (Reads Per Kilobase per Million mapped reads) values.

### 2) Analyzing the ORF Inter-Group Difference

After calculating the abundance (RPKM) of the RF Reads in various ORFs, the edgeR software is used to compare ORFs with expression difference between the DN group and the NC group, and a fold change (FC) is calculated. Differentially translted genes (DTG) are screened out by using FDR and log2FC, wherein the screening condition is |log2FC|> and FDR<0.05.

### 3) Evaluating the sORF coding potential

The sORF coding potential is comprehensively evaluated by the following three standards:

### a) ORFscore

RFs conform to the characteristic of a three-base rhythm, and ribosomes pause on RNA in a unit of codon composed of three bases during translation. Therefore, RFs signals with translation potential are concentrated within the main reading frame of an ORF sequence (also known as Frame1). The ORF score is given based on the aforesaid characteristic of RFs with translation potential.

### b) RRSscore

Ribosomes are released from RNA when they encounter a stop codon, so that the abundance of RFs with translation potential after the stop codon is significantly lower than that in an upstream ORF region. The RRS score is given based on the aforesaid characteristic of RFs with translation potential.

### c) Fickett score

The coding sequence and non-coding sequence of each species differ in sequence characteristics, so that the known coding sequence of a species may be used as a reference database to evaluate whether a non-coding sequence has coding ability. The Fickett score is given by using this method, and normally, Fickett score 0.74 is used as the standard for evaluating RFs with coding potential.

sORFs that may have translation potential are screened out based on the standards including ORFscore greater than the threshold, RRSscore greater than the threshold, and Fickett score=0.74.

### 4) ORF annotation

The ORFs having translation potential and exhibiting a translation difference fold greater than 2 between the two groups are annotated, and the protein sequences potentially encoded by sORF are compared to the pfam database (version 26.0) to find the potential protein domain contained by sORF.

### 1.3.6 Stimulating the secretory small peptide into fibroblasts using over-expression conditioned medium

The vector used for the screened secretory small peptide over-expression and control plasmid is PMSCV-IRES-GFP. The plasmid synthesis is performed by Genewiz Biotechnology Co., Ltd. The synthesized secretory small peptide over-expression plasmid is Cf48-pMSCV-IRES-GFP, Hilpda-pMSCV-IRES-GFP and Apoc1-pMSCY-IRES-GFP, and the synthesized control plasmid is pMSCV-IRES-GFP.
1)Transfection of the secretory small peptide over-expression and control plasmid is performed according to the operation process of Lipofectamine 3000, comprising the steps of:
   a) Evenly planting 293T tool cells in a six-well cell plate using a cell growth medium (DMEM/F12+10%FBS);
   b) After the cells are attached to the wall and grow steadily, replacing the cell growth medium with a serum-free medium, wherein each well is added with 0.5mL of serum-free medium;
   c) Preparing a reaction solution in the amount of 500µL Opti-MEM +2.5µg DNA +3.75µL Lipofetamine 3000 +5 µL P3000 for each well, mixing at room temperature and letting it sit for 15 minutes, and then adding 500µL of reaction solution containing the over-expression or control plasmid to each well;
   d) After 12 hours of reaction, replacing the reaction solution with the cell growth medium (DMEM/F12+10%FBS) and culturing in a cell culture box;
2) Collecting the secretory small peptide over-expression and control conditioned medium: after 24 hours of transfection with plasmids, observing the cells under a fluorescent microscope, wherein the cells give a bright green fluorescence, and the density of cells giving green fluorescence is about 70%; subsequently, replacing the cell growth medium with serum-free expression medium; after 36 hours of reaction, collecting the secretory small peptide over-expression and blank control conditioned medium, centrifuging and removing the cell debris;
3) Evenly planting NRK-49F cells in a six-well cell plate using the cell growth medium (DMEM/F12+10%FBS);
4) Adding the collected secretory small peptide over-expression and control conditioned medium in the presence or absence of 1ng/mL TGF-β1 into the NRK-49F cells, collecting the cell protein after 48 hours of reaction, and observing the expression quantity of α-SMA by using western blot;

### 1.3.7 Extracting cell protein

1) Performing protein lysis: placing the six-well cell plate on ice, extracting the supernatant of the culture medium, removing cell debris using the pre-cooled clean PBS, adding 60µL of a cell lysate to each well, and reacting on ice for 15 minutes;
2) Using a cell scraper to scrape off the protein in a certain direction in each well, and transferring the cell lysate to a 1.5mL EP tube;
(3) Centrifuging at a speed of 15000rpm for 30 minutes, and extracting the supernatant from the lysate for later use;

### 1.3.8 Measuring protein concentration using BCA method

Measuring the protein concentration according to the operating process of the BCA kit (Thermo), comprising the steps of:
1) After diluting the protein stock solution by the corresponding multiple, adding 5µL of protein and 5µL of protein standard to each well of a corresponding 96-well plate;
2) Preparing a working solution (solution A: solution B=50:1), and adding 200µL of the working solution to the protein and standard solution in each well;
3) Reacting for 30 minutes at a temperature of 37°C;
4) Measuring the absorbance at a wavelength of 562nm using a molecular device, and calculating the protein concentration by drawing a standard curve based on the standard.

### 1.3.9 Measuring using the western blot method

### 1) Preparing a PAGE gel

According to the operating process of the PAGE gel rapid preparation kit (Epizyme), preparing a 10% separation gel and a spacer gel, comprising the steps of:
a) Cleaning a 1.5mm glass plate, clamping it onto a glue preparation rack, fixing it onto a filled gel strip, and adding ddH20 to check if there is water leakage of the glass plate and if the liquid level is horizontal;
b) Preparing a lower-layer gel buffer solution following the ratio of the operating process: 4mL of the lower-layer gel solution+4mL of lower-layer gel buffer solution; after thorough mixing, adding 80µ L of a coagulant, and after mixing again, slowly adding anhydrous ethanol to the glass plate and flattening the lower-layer gel, wherein after letting it sit for about 15 minutes, a clear boundary between the lower-layer gel and anhydrous ethanol is seen, namely, the solidified gel; subsequently, pouring the anhydrous ethanol out, evaporating, and drying at room temperature;
c) Preparing an upper-layer gel buffer solution following the ratio of the operating process: 2mL of the upper-layer gel solution+2mL of upper-layer gel buffer solution; after thorough mixing, adding 40µL of coagulant, and after mixing again, adding a glass plate, slowly tilting, and inserting a 1.5mm 15-hole comb; subsequently, letting it sit about 15 minutes until the upper-layer gel congeals, and storing the prepared PAGE gel in a wet state for later use.

### 2) Performing SDS-PAGE electrophoresis

Clamping the prepared PAGE gel in a vertical electrophoresis tank using an electrophoresis clip, taking out the denatured protein, heating it at a temeprature of 100°C using the air bath method for 5 minutes, and then taking it out for later use after the protein is completely dissolved; filling the electrophoresis tank with a prepared 1x electrophoresis solution, slowly and gently pulling out the comb, adding 30µL of protein into each hole, and respectively adding 5µL of protein molecular weight indicator at both ends of the PAGE gel; performing electrophoresis, running the gel slowly under a constant voltage of 90V, and after all the protein samples enter the separation gel and the protein strip exhibits a narrow horizontal line, adjusting the constant voltage to 120V, and stopping electrophoresis until all the protein indicators enter the electrophoresis solution.

### 3) Performing membrane transfer

Immersing and activating a PVDF membrane in methanol; preparing a membrane transfer sandwich device: sequentially arranging the thick filter paper, separation gel, PVDF membrane and thick filter paper from the negative electrode to positive electrode; using a roller to remove bubbles, covering using an electrode cover, and placing in an ice bath device; performing membrane transfer for 120 minutes under a constant voltage of 100V;
4) Sealing: preparing 5% milk (skimmed milk powder in TBST), cleaning the PVDF membrane in ddH20 after the membrane transfer is completed, and sealing it at room temperature in 5% milk for 1 hour;
5) Incubating a primary antibody: diluting the primary antibody with 5% milk (1:1000) and incubating overnight on a shaker at a temperature of 4°C;
6) Washing membrane: after recovering the primary antibody, washing it on the TBST shaker for 3 times for 10 minutes;
7) Incubating a secondary antibody: diluting the secondary antibody with 5% milk (1:5000) and incubating at room temperature for 1 hour;
8) Washing membrane: after recovering the secondary antibody, washing it on the TBST shaker for 3 times for 10 minutes;
9) Developing: evenly dropwise adding luminescent liquid onto the PVDF membrane in a dark environment, reacting at room temperature for 3 minutes; subsequently, placing the PVDF membrane into a fully automatic chemiluminescence imaging device, and developing in an automatic exposure mode; when the signal is too strong or too weak, changing to a manual exposure mode for development;
10) Performing protein stripping: adding protein stripping buffer to the PVDF membrane, washing on a shaker for 15 minutes, and washing using TBST for 5 minutes; subsequently, adding 5% milk and sealing at room temperature for 1 hour, and then incubating a next antibody;
11) Analyzing results: quantitatively analyzing the grayscale values of the target protein strips using a fully automatic chemiluminescence imaging system, and then calculating the relative content of the target protein by using the internal reference protein as an internal control for statistical analysis.

### 1.4 Experimental results

### 1.4.1 Differentially expressed genes in mice with progressive diabetes nephropathy and normal control mice

In ORFs with coding potential, differentially expressed genes are screened between mice with progressive diabetes nephropathy (DN) and normal control mice (NC) by screening conditions (FDR<0.05, | log2FC|>1). The results show that there are 1981 significantly different genes between the two groups, of which 1622 are significantly up-regulated genes and 359 are significantly down-regulated genes. There are significant differences in the overall expression patterns of genes between the DN and NC groups of mice. As shown in Figure 1-1, in the DN group, most genes show an up-regulated expression pattern, while in the NC group, most genes show a down-regulated expression pattern. The expression patterns of the samples belonging to the NC group (NC1-NC4) and the samples belonging to the DN group (DN1-DN5) are similar, indicating that the NC mice samples and DN mice samples have high repeatability.

### 1.4.2 Screening conditions for target genes

Screening target genes based on the following screening conditions:
1) The target genes need to have good conservation: as the sequencing is performed in kidney tissues of mice, to achieve higher clinical application value, the research targets need to have good conservation and are capable of being simultaneously expressed in both humans and mice. By consulting the database, 39 out of 1981 differentially expressed genes show good conservation and can be expressed simultaneously in both humans and mice (39/1981).
2) The target genes are capable of translating small peptides: based on the advantages of translatomics sequencing, it is expected to screen out novel small peptides related to diseases. Among the 39 genes having good conservation, 11 genes are capable of translating small peptides (11/39) with an amino acid length of <100aa.
3) The target genes should be secretory small peptides: further prediction is made using the software (SignalP-5.0), and 3 out of 11 peptides are secretory peptides (3/11).
4) The target genes have potential biological functions: among the three differentially expressed secretory peptides, preliminary in-vitro experiments are conducted to verify whether the secretory small peptides have biological functions.

### 1.4.3 Verification of the fibrosis-promoting effect of secretory small peptide

The expression of Cf48 secretory small peptide obtained by the screening conditions in 1.4.2 is increased in the kidney tissues of mice with diabetic nephropathy:

### 1) Cf48 (MGI:2686519, DN/NC: Log2(FC)=2.96, FDR=0.046, 90aa).

Taking into account the characteristic of the secretory small peptide, the following in-vitro experiments are designed. By transfecting the over-expression plasmid of Cf48 in the secretory small peptide with increased expression in mice with diabetic nephropathy into tool cells, blank vectors and small peptide over-expression conditioned medium supernatant are collected after sufficient expression. Fibroblasts (NRK-49F) are stimulated in the presence or absence of TGF-β1, and the expression of α-SMA in fibroblasts is observed. The results are shown in Figures 1-2 and 1-3. According to Figures 1-2 and 1-3, compared with the conditioned medium of the blank control vector, the supernatant of the conditioned medium containing Cf48 over-expression is capable of enhancing the fibroblast activating response induced by TGF-β1, and the expression of α-SMA is increased.

### 1.4.4 The Cf48 small peptide exhibits high conservation

The mice gene Cf48 (Gene name: Gm1673 in mice, MGI:2686519, NCBI Gene ID: 381633) is located on chromosome 5 (Chr5:34,140,777-34,142,353) and consists of four exons. It is capable of translating a small peptide with an amino acid length of 90aa, and the amino acid sequence is SEQ: 2. The human gene Cf48 (NCBI Gene ID: 401115) is located in the 48th open reading frame of chromosome 4 (Chr4:2,035,610-2,043,970) and consists of 9 exons. It is capable of translating a small peptide with an amino acid length of 90aa, and the amino acid sequence is SEQ:1. Comparing the gene structures of Cf48 in mice and humans, it is found that the gene structures of Cf48 in humans and mice are similar, and the gene homology is high. According to the multiple sequence alignment results of the protein sequence of Cf48 among species, it is found that the Cf48 small peptide exhibits high evolutionary conservation among species.

The amino acid sequence of Cf48 in mice is:

MAPALRSLLSPRTLLLLLLSLALLGARAEPATGSAVPAQSRPCVDCHAFEFM
QRALQDLRKTAYSLDARTETLLLQAERRALCACWPAGR (90aa).

The cDNA sequence of Cf48 in mice is: 
ATGGCCCCCGCGCTCAGGTCCTTGCTGTCGCCACGGACTTTACTGCTGCT
GCTGCTGAGCCTGGCGCTGCTGGGCGCCCGCGCCGAGCCCGCCACCGGAGCG
CTGTCCCCGCTCAGAGCCGCCCGTGCGTGGATTGCCACGCATTTGAATTCATGC
AGCGCGCCCTTCAGGACCTACGGAAAACCGCCTACAGCCTGGACGCACGGACG
GAGACCCTCCTGCTGCAGGCTGAGCGCCGGGCTCTGTGTGCCTGCTGGCCAGC
TGGACGCTGA.
 (Note: underlined: signal peptides, other sequences: secretory peptides).

### 1.4.5 The small peptide Cf48 is a secretory small peptide

The secretory potential of Cf48 small peptide is predicted through SignalP-5.0 (http://www.cbs.dtu.dk/services/SignalP-5.0/), and the following results are obtained: the Cf48 small peptide is a secretory peptide in both mice and humans, and the sites of breakage at both the signal end and secretory end are the same. The lysis site of mice is between amino acids 28 and 29, while that of humans is between amino acids 34 and 35. It is capable of being lysed into signal peptides located inside the cells and secretory peptides outside the cells.

### 1.4.6 Cf48 over-expression in conditioned medium enhances the activation response of TGF-β1-induced fibroblasts

As mentioned earlier, according to the results shown in Figures 1-3, compared with the blank control, the Cf48 ove-expression in the conditioned medium is capable of enhancing the expression level of TGF-β1-induced α-SMA while promoting the activation of fibroblasts. The secretory Cf48 has a biological function of promoting fibrosis.

### 1.4.7 Conclusion from the above experiment:

Through the translatomics sequencing performed on mice with diabetic nephropathy, it is found that the expression level of the Cf48 small peptide is significantly increased in the kidney tissues of mice with progressive diabetic nephropathy, and the over-expression of secretory Cf48 in the conditioned medium is capable of enhancing the activation response of TGF-β1-induced fibroblasts and promoting the activation of fibroblasts.

### 2. Expression of Cf48 small peptide in patients with chronic kidney disease

### 2.1 Research purpose

The research purpose is to analyze the expression of Cf48 in renal tissues and serum of CKD patients with different causes of disease and explore the correlation between Cf48 and disease activity as well as severity of renal injury.

### 2.2 Experimental materials

### 2.2.1 Main experimental apparatuses and devices

1) Molecular devices (USA); 2) Fully automatic microplate washer (Biorad, USA); 3) vortex mixer XW-80A (Shanghai Medical University Instrument Co., Ltd., China).

### 2.2.2 Main experimental consumables

1) PAP pen (ZSGB-BIO, China); (2) positively charged adhesive glass slide (Thermo, USA); (3) cover slip (Guangzhou Whiga Technology Co., Ltd., China).

### 2.2.3 Main experimental reagents

1) PBS powder (Whiga Technology, Guangzhou); 2) BSA powder (Whiga Technology, Guangzhou); 3) 30% hydrogen peroxide (Whiga Technology, Guangzhou); 4) immunohistochemical kit (Dako, Denmark); 5) mayer hematoxylin purchased (Abcam, USA); 6) neutral resin (Hesi Biotech, China); 7) DAPI (Thermo, USA); 8) mounting medium (Dako, Denmark); 9) 50X antigen repair solution (Whiga Technology, Guangzhou); 10) Cf48 ELISA Kit (CUSABIO, China); 11) antibodies: anti-Cf48 antibody, purchased from Abcam Corporation (USA); directly labeled α-SMA-cy3 antibody and anti-α-SMA antibody, purchased from Sigma Corporation (USA); and Alexa Fluro 488-marked goat anti-rabbit IgG, purchased from Life Technology (USA).

### 2.3 Research method

### 2.3.1 Research object

### 2.3.1.1 Clinical research ethics

This research has been approved by the Ethics Committee of the First Affiliated Hospital of Sun Yat-sen University, and all patients, as well as disease and healthy controls have signed the informed consent forms. The approval number is "Ethic Review (2016) No. 215 of the First Affiliated Hospital of Sun Yat-sen University".

### 2.3.1.2 Inclusion and exclusion criteria for CKD patients

The renal tissue Cf48 expression level and free Cf48 content in serum of CKD patients confirmed through renal biopsy by the Nephrology Department of the First Affiliated Hospital of Sun Yat-sen University are detected. The selected CKD diseases include: diabetic nephropathy (DN), lupus nephritis (LN) and IgA nephropathy (IgAN). The inclusion criteria for patients: patients who have underwent renal biopsy at the Nephrology Department of the First Affiliated Hospital of Sun Yat-sen University from September 2018 to September 2021; the renal puncture result conforms to one of DN, LN or IgAN; age ranges from 18 to 65, male or female, CKD1-5 stage, and are willing to sign an informed consent form.

Exclusion criteria for patients: patients having severe complications such as malignant tumors, heart failure, malignant hypertension, or cerebrovascular accidents, etc.

### 2.3.1.3 Inclusion criteria for disease control group and healthy control group

Inclusion criteria for diabetic nephropathy disease control group (DM): age ranges from 18 to 65, the diagnosis result meets the diagnostic criteria for diabetes revised by the American Diabetes Association (ADA) in 2020, have no any degree of renal injury, and are willing to sign an informed consent form.

Inclusion criteria for the healthy control group: age ranges from 18-65, have normal renal function, have no microscopic hematuria or proteinuria, have no recent infection, have no history of major diseases, and are willing to sign an informed consent form.

### 2.3.1.4 Sample collection

5ml of peripheral venous blood from all CKD patients and controls are collected, and after being isolated by using centrifuging, the serum is obtained and frozen at a temperature of -80°C for later use. The kidney tissue slides of CKD patients are obtained from paraffin slides of kidney biopsy, while the normal control slides are obtained from paraffin slides of normal kidney tissues adjacent to tumors in renal cancer patients.

### 2.3.1.5 Clinical information collection

1) Demographic data: demographic data such as gender and age of included CKD patients, DM disease controls and healthy controls are collected;
2) Disease severity indicators: the original disease course, hypertension history, diabetes history, and cardiovascular disease history of CKD patients are collected; serum urea nitrogen (BUN), serum creatinine, blood albumin, blood uric acid, fasting blood glucose, glycated hemoglobin, and 24-hour urine protein indicators of CKD patients are collected; the eGFR calculation adopts a simplified MRDR calculation formula.
3) Pathological indicators:
   a) Patients with diabetic nephropathy: based on the histopathologic diagnosis of renal biopsy, the DN patients are divided into grades 2, 3 and 4 according to the pathological classification proposed by Tervaet et al.
   b) Patients with lupus nephritis: based on the pathological diagnosis of renal biopsy, ISN/RPS pathological classification is adopted.
   c) Patients with IgAnephropathy: based on the pathological diagnosis of renal biopsy, patients with IgA nephropathy are divided into a group having Lee's grading≤3 and a group having Lee's grading>3. The Lee's grading standards mainly refer to the 1982 Lee's grading standards.

### 2.3.2 Fluorescence in situ hybridization staining(Cf48)

Fluorescence in situ hybridization staining is performed on paraffin slides (5µm) of DN patients and normal kidney tissues adjacent to tumors in renal caner patients. Probe synthesis and in situ hybridization are performed by Guangzhou Sevier Biotechnology Co., Ltd.

### 2.3.3 Immunohistochemical staining (Cf48, α-SMA)

1) Baking the slides: placing paraffin slides with a thickness of 5µm into an oven, baking at a temperature of 60°C for 1 hour until the paraffin on the slides is completely removed;
2) Performing deparaffinage and hydration: performing in the following sequence: xylene (20 minutes, 2 times), anhydrous ethanol (10 minutes, 2 times), 95% ethanol (10 minutes, 2 times), 75% ethanol (10 minutes, 2 times), and PBS buffer (5 minutes, 1 time);
3) Performing antigen repair: preparing a 1X antigen repair solution (sodium citrate, 0.01mol/L, pH=6), and repairing in a pressure cooker; when reaching the highest boiling point of the cooker, cooling naturally to room temperature, wherein the entire process takes about 3 hours; subsequently, washing off the antigen repair solution using the PBS buffer for 5 minutes;
4) Drawing circles using a PAP pen: wiping off the moisture around the tissues, and gently drawing circles using a PAP pen; after the tissues are completely circled, placing them on a wet box, wherein the tissues are protected from being dried out during the process;
5) Inactivating catalase: dropwise adding 50µL of a 3% hydrogen peroxide solution into each histochemical circle, incubating at room temperature for 15 minutes in a dark environment; subsequently, placing into the PBS buffer for 5 minutes on the shaker, and washing for 3 times;
6) Sealing: dropwise adding 50µL of a 5% BSA (prepared using PBS) solution into each histochemical circle, and sealing at room temperature for 10 minutes;
7) Incubating the primary antibodies: diluting the primary antibodies at the corresponding concentration (Cf48, 1:100, α-SMA, 1:1000) in the PBS buffer, and incubating at room temperature in the dark environment for 1 hour;
8) Incubating a secondary antibody: placing in PBS bufwfer on the shaker for 10 minutes, washing for 3 times, dropwise adding 50µL of a solution A (mouse rabbit combined secondary antibody) in an immunohistochemical kit to each histochemical circle, and incubating at room temperature in the dark environment for 1 hour;
9) Developing color using DAB: placing in the PBS buffer on the shaker for 10 minutes, and washing for 3 times; subsequently, preparing a DAB reaction working solution in a ratio of 50:1 of a B solution and a C solution in the immunohistochemical kit, dropwise adding the DAB reaction working solution into the histochemical circles, and observing the reaction speed under a microscope; after the reaction is completed, rinsing with DDH 20;
10) Counter-staining using hematoxylin: immersing the slides in mayer hematoxylin for 3 minutes and washing with ddH20;
11) Performing routine dehydration and sealing: performing in the following sequence: 75% ethanol (5 minutes, 2 times), 95% ethanol (5 minutes, 2 times), anhydrous ethanol (5 minutes, 2 times), xylene (5 minutes, 2 times), and sealing using neutral resin;
12) Observing and photographing under an upright microscope

### 2.3.4 Performing immunofluoresent staining (Cf48, α-SMA)

1) Baking paraffin slides having a thickness of 5µm, deparaffinating, hydrating, repairing the antigen, and drawings circles using the PAP pen following the steps in 2.3.3 1)-4);
2) Sealing: dropwise adding 50µL of the 1% BSA (prepared using PBS) solution to each histochemical circle, and sealing at room temperature for 1 hour;
3) Incubating the primary antibodies: diluting the primary antibodies at the corresponding concentration (Cf48, 1:50, α-SMA, 1:5000) in the 1% BSA solution, and incubating overnight at a temperature of 4°C in the dark environment;
4) Incubating the secondary antibody: placing in the PBS buffer on the shaker for 10 minutes, washing for 3 times, diluting the corresponding secondary antibody in the 1% BSA solution at a ratio of 1:1000, and incubating at room temperature for 1 hour;
5) Counter-staining using DAPI: placing in the PBS buffer for 10 minutes on a shaker, washing for 3 times, and incubating using DAPI at a concentration of 1:100 for 5 minutes at room temperature;
6) Placing in the PBS buffer on the shaker for 10 minutes, washing for 3 times, shaking off water, dropwise adding a sealing agent, covering with a cover slip, and drying overnight at room temperature in the dark environment;
7) Observing and photographing under a laser scanning confocal microscope.

### 2.3.5 Immunohistochemical staining area measurement

Scanning a certain number of glass slides in a fully automatic scanner, and randomly selecting 10 renal cortical fields of view under high power microscope (×200) using a single blind method; through Image j (http://www.imagej.nih.gov/ij/), quantifying the expression intensity of positive signals, taking the percentage of positive expression intensity in each field of view as the target molecule expression level, and taking the average expression level of 10 fields of view as the expression level of the target molecules (Cf48, α-SMA).

### 2.3.6 Detection of Cf48 content in serum

Detecting the content of Cf48 in serum according to the operating process of the Cf48ELISA kit, comprising the steps of:
1) Defrosting all reagents to room temperature 30 minutes in advance;
2) Preparing standards: dissolving a standard in a sample diluent to obtain a mother liquor at a concentration of 40ng/mL; after performing a two-fold dilution, respectively obtaining standards with concentrations of 40, 20, 10, 5, 2.5, 0.625, and 0 ng/mL;
3) Incubating using a microplate: respectively adding 100µL of the standard and serum of each experimental group into each well, gently shaking up after a film is applied, and incubating at a temperature of 37°C for 2 hours;
4) Directly discarding the supernatant, slightly beating the paper to remove the residual liquid, adding 100µL of biotin-antibody to each well, gently shaking up after a film is applied, and incubating at a temperature of 37°C for 1 hour;
5) Washing the microplate: adding 300µL of washing liquid to each well, and removing the washing liquid by using an automatic plate washer; after repeating for 3 times, slightly beating the paper to remove the residual liquid;
6) Adding 100µL of HRP-avidin to each well, gently shaking up after a film is applied, and incubating at a temperature of 37°C for 1 hour;
7) Washing the microplate: performing step 6), and after repeating for 5 times, slightly beating the paper to remove the residual liquid;
8) Adding 90µL of a TMB substrate colorimetric solution to each well and incubating at a temperature of 37°C in an incubator in the dark environment for 30 minutes until the liquid in the sample and standard wells gradually turns blue;
9) Adding 50µL of a termination solution to each well, and gently mixing until the liquid turns from blue to yellow;
10) Reading the absorbance (OD value) at a wavelength of 450nm by using the molecular device;
11) Drawing a standard curve and calculating the concentration of sample.

### 2.3.7 Statistical analysis

The data is presented as mean ± standard deviation and is analyzed using the SPSS 26.0 software (GraphPad Prism 8.0, developed by GraphPad Software, San Diego, CA). The Student's t-test is used for comparison between two groups, and the ANOVA is used for comparison among a plurality of groups. Subsequently, the Tukey's Post test is used for multiple comparisons among three or more groups. The correlation analysis adopts the Sperman correlation analysis, and the P-value<0.05 is defined as having a statistical significance.

### 2.4 Experimental result

### 2.4.1 Increased expression of Cf48 mRNA in DN patients' renal tissues

The fluorescence in situ hybridization (FISH) staining is performed on DN patients' renal puncture tissues and normal tissues adjacent to tumors to determine the location and expression of Cf48mRNA in DN patients. As shown in Figure 2-1, compared with normal renal tissues, the expression level of Cf48mRNA is significantly increased in DN patients' renal tubules. Moreover, higher expression is observed in significantly injured and dilated tubules, while no expression of Cf48mRNA is observed in the glomeruli. The expression of Cf48mRNA is significantly increased in the injured renal tubules of patients with diabetic nephropathy, and no expression of Cf48mRNA is observed in the glomeruli and normal renal tissues of patients with diabetic nephropathy, proving that the Cf48 small peptide is produced by injured tubular cells.

### 2.4.2 Increased expression of Cf48 in DN patients' renal tissues

The expression of Cf48 in DN patients and normal renal tissues is detected by means of the immunohistochemical staining. As shown in Figure 2-2, the Cf48 is significantly increased in DN patients' renal tissues, and the positive signals are concentrated in the renal tubulointerstitium. Further statistical analysis is performed on the expression intensity of Cf48 in DN patients' renal tubulointerstitial region. As shown in Figure 2-2C, the results show that the expression intensity of Cf48 in DN patients' tubulointerstitial region is significantly higher than that in normal renal tissues. The difference is statistically significant (P<0.001).

### 2.4.3 Increased expression of Cf48 in CKD patients' renal tissues

The expression of Cf48 in renal tissues of CKD patients with other causes of disease is further detected to determine whether the increased expression of the Cf48 small peptide in renal tissues is specific or non-specific for diabetic nephropathy. Normal renal tissues adjacent to tumors in renal cancer patients is taken as the normal control, renal puncture tissues from patients with minimal change disease (MCD) is taken as the disease control, and renal puncture tissues from CKD patients (including LN, IgAN, and DN patients) are selected to detect the expression of Cf48 through immunofluorescence detection. According to the results shown in Figure 2-3, the expression level of Cf48 is low in normal controls' and MCD patients' renal tissues, and no significant expression of Cf48 is observed. Meanwhile, there is also no increase in α-SMA staining. The expression level of Cf48 in CKD patients' (including LN, IgAN, and DN patients') renal tissues is significantly increased. The signals are mainly concentrated in tubular epithelial cells, and the increase is more significant in regions with higher expression of α-SMA. It can be seen that the expression level of the Cf48 small peptide is increased in renal tissues of CKD patients with different causes of disease and is associated with the expression of fibrosis index α-SMA. The Cf48 small peptide is capable of serving as a marker for kidney disease to represent the degree of kidney disease and fibrosis.

### 2.4.4 Increased content of secretory Cf48 in CKD patients' serum

Because Cf48 is a small secretory peptide, the content of Cf48 in the serum of normal controls (CTL), disease controls with diabetic mellitus (DM) but without diabetic nephropathy, and patients with diabetic nephropathy (DN) are detected by using the ELISA kits first. As shown in Figure 2-4, the content of Cf48 in DN patients' serum is significantly higher than that in normal controls' serum (9.678±4.661 *vs* 1.243±1.566, P<0.0001) and is also significantly higher than that in DM patients' serum (9.678±4.661 *vs* 1.626±1.150,

P<0.0001). However, there is no significant difference of Cf48 content in serum between normal controls and DM patients. This result indicates that the Cf48 secretory small peptide is a key factor in promoting the progression of kidney disease, and the Cf48 level is not associated with the patients' glucose tolerance level. Therefore, the Cf48 content in the serum of CKD patients without DN cause is further detected, and the results show that the Cf48 content in the serum of IgAN and LN patients are respectively 7.131ng/mL and 8.752ng/mL, which are significantly higher than those in normal controls (P<0.0001). The above results show that the content of the Cf48 small peptide in the serum of CKD patients with different causes of disease is increased, and its up-regulation of expression level is associated with the progression of kidney disease rather than its cause of disease. Therefore, it may participate in the common mechanism of the progression of CKD.

### 2.4.5 Significant negative correlation between secretory Cf48 content in CKD patients' serum and eGFR

The analysis on correlation between the secretory Cf48 content in CKD patients' serum and eGFR is shown in Figure 2-5A. As shown in Figure 2-5A, it can be seen that the secretory Cf48 content in CKD patients' serum is significantly negatively correlated with eGFR (correlation coefficient r=-0.496, P<0.0001, n=73). Figure 2-5B shows the correlation between the secretory Cf48 content in CKD patients' serum and CKD grading. This shows that the secretory Cf48 content in CKD patients' serum is capable of reflecting the patients' CKD stage, exhibiting a significant positive correlation (correlation coefficient r=0.625, P<0.0001, b=73). The higher the secretory Cf48 content in the serum, the higher the CKD stage. It can be learned that the secretory Cf48 content in CKD patients' serum is capable of representing the patients' kidney disease degree and the CKD stage.

### 2.4.6 Significant positive correlation between the secretory Cf48 content in the serum and the renal fibrosis degree

To explore the correlation between the content of the secretory Cf48 in the serum and the renal fibrosis degree, renal puncture slides of DN patients who have underwent the renal biopsy at the Department of Nephrology in the First Affiliated Hospital of Sun Yat-sen University are obtained. The renal fibrosis degree is reflected by using the α-SMA immunohistochemical staining and the analysis on correlation between the secretory Cf48 content in the serum and the renal fibrosis degree is performed. As shown in Figure 2-6, the content of Cf48 in DN patients' serum is significantly positively correlated with the expression intensity of α-SMA in patients' renal tissues (correlation coefficient r=0.815, P<0.0001, n=16). Taking two patients as examples, the content of Cf48 in the first patient's serum is 15.128ng/mL and the α-SMA staining positive area is 14.94%, while the content of Cf48 in the second patient's serum is 3.718ng/mL and the α-SMA staining positive area is 3.78%. It shows that the content of Cf48 in the serum is capable of representing the renal fibrosis degree and serving as a marker for representing the degree of fibrosis in renal tissues.

### 2.4.7 Analysis on correlation between secretory Cf48 content in DN patients' serum and clinical indicators

In the following, a stratified analysis is performed on the CKD patients included in this research according to their disease types, and the correlation between the secretory Cf48 content in DN patients' serum and clinical indicators is further analyzed. The analysis results are shown in Table 2-1. Table 2-1 shows that the content of secretory Cf48 in DN patients' serum is significantly positively correlated with their blood urea nitrogen and creatinine levels (correlation coefficient r=0.514, P=0.002; correlation coefficient r=0.446, P=0.009), is significantly negatively correlated with DN patients' eGFR (correlation coefficient r=-0.446, P=0.009), and is significantly positively correlated with the CKD staging (correlation coefficient r=0.669, P<0.0001). The higher the content of secretory Cf48 in the serum, the higher the DN patients' CKD stage. As shown in Figure 2-7, the correlation between the content of Cf48 in DN patients' serum and their pathological grading is further analyzed. It is found that the higher the content of Cf48 in DN patients' serum, the more severe their renal tissue pathological grading results. The aforesaid two are significantly positively correlated (correlation coefficient r=0.385, P=0.027).

**Table 2-1 Analysis on correlation between secretory Cf48 content in DN patients' serum and clinical indicators**

| Clinical Indicators | Spearman r | *P value* |
|---|---|---|
| CKD staging | 0.669 | **<0.0001****** |
| Pathological grading | 0.385 | **0.027*** |
| BUN | 0.514 | **0.002**** |
| Creatinine | 0.446 | **0.009**** |
| Serum albumin | 0.042 | 0.812 |
| FBG(fasting blood-glucose) | -0.031 | 0.867 |
| HbAC1 | -0.041 | 0.834 |
| Uric acid | 0.306 | 0.094 |
| 24h Urine protein | -0.076 | 0.678 |
| eGFR | -0.446 | **0.009**** |

| | | |
|---|---|---|
| Note: n=33, * p<0.05, ** p<0.01, ****p<0.0001. | | |

### 2.4.8 Analysis on correlation between secretory Cf48 content in LN patients' serum and clinical indicators

The correlation between the secretory Cf48 content in LN patients' serum and clinical indicators is further analyzed. The analysis results are shown in Tables 2-2 and 2-8. Table 2-8 shows that the content of secretory Cf48 in LN patients' serum is significantly positively correlated with their blood urea nitrogen and creatinine levels (correlation coefficient r=0.610,P=0.004; correlation coefficient r=0.669, P=0.002), is significantly negatively correlated with LN patients' eGFR (correlation coefficient r=-0.609,P=0.004), and is significantly positively correlated with the CKD staging (correlation coefficient r=0.693,P<0.0001). The correlation between the content of Cf48 in LN patients' serum and their pathological grading is further analyzed, and no significant correlation (correlation coefficient r=-0.097, P=0.731) between the aforesaid two is found.

**Table 2-2 Analysis on correlation between secretory Cf48 content in LN patients' serum and clinical indicators**

| Clinical Indicators | Spearman r | *P Value* |
|---|---|---|
| CKD staging | 0.693 | **<0.0001****** |
| ISN/RPS pathological grading | -0.097 | 0.731 |
| BUN | 0.610 | **0.004**** |
| Creatinine | 0.669 | **0.002**** |
| Serum albumin | -0.195 | 0.424 |
| FBG(fasting blood-glucose) | 0.030 | 0.903 |
| HbAC1 | -0.154 | 0.805 |
| Uric acid | 0.286 | 0.493 |
| 24h Urine protein | 0.414 | 0.098 |
| eGFR | -0.609 | **0.004**** |

| | | |
|---|---|---|
| Note: n=20, ** p<0.01, ****p<0.0001. | | |

### 2.4.9 Analysis on correlation between secretory Cf48 content in IgAN patients' serum and clinical indicators

The correlation between the secretory Cf48 content in IgAN patients' serum and clinical indicators is further analyzed. The analysis results are shown in Tables 2-3. The results show that the content of secretory Cf48 in LN patients' serum is significantly positively correlated with the Lee's grading of renal biopsy results (correlation coefficient r=0.564, P=0.023). As shown in Figure 2-9, the higher the Cf48 content in the patient's serum, the higher the Lee's grading of the patient's renal biopsy results.

**Table 2-3 Analysis on correlation between secretory Cf48 content in IgAN patients' serum and clinical indicators**

| Clinical Indicators | Spearman r | *P Value* |
|---|---|---|
| CKD staging | 0.320 | 0.169 |
| Lee pathological grading | 0.564 | **0.023*** |
| BUN | 0.211 | 0.371 |
| Creatinine | 0.280 | 0.232 |
| Serum albumin | 0.090 | 0.705 |
| FBG(fasting blood-glucose) | 0.078 | 0.743 |
| HbAC1 | 0.543 | 0.266 |
| Uric acid | 0.143 | 0.583 |
| 24h Urine protein | 0.074 | 0.758 |
| eGFR | -0.355 | 0.125 |

| | | |
|---|---|---|
| Note: n=20, * p<0.05. | | |

### 2.4.10 Expression of Cf48 in fibrotic tissues of mice lungs and hearts

To further determine the expression of Cf48 in other fibrotic organ tissues, the expression of Cf48 is detected in mice pulmonary fibrosis tissues induced by using Bleomycin for 4 weeks. The Masson Chrome staining shows the normal lungs (Figure 2-10A) and fibrotic lungs (Figure 2-10B). As shown in Figure 2-10C, the immunohistochemical results show that the Cf48 expression is not found in normal mice lungs, and as shown in Figure 2-10D, the Cf48 expression exhibits only a small amount of scattered dots in fibrotic lung tissues. As shown in Figure 2-10E, the immunohistochemical results show that the Cf48 expression is not found in normal mice hearts, and as shown in Figure 2-10F, the expression of Cf48 in fibrotic hearts is also very low and the expression exhibits only a small amount of scattered dots.

### 2.4.11 The expression level of Cf48 in peripheral blood of patients with lung diseases

To further determine whether Cf48 is increased in the peripheral blood of patients with pulmonary fibrosis, the Cf48 ELISA kits are used to detect samples with KL-6 values higher than 800 (KL-6 is an indicator for interstitial lung disease, which represents the proliferation of type 2 alveolar epithelial cells and is usually lower than 500 in normal people). The ELISA results show that the level of Cf48 in peripheral blood of patients with KL-6 values higher than 800 is 1.07±0.64 ng/ml (mean±SD, n=57), while the level of Cf48 in other controls with lung diseases (including pneumonia, pulmonary embolism and lung cancer, etc.) is 0.72±0.52 ng/ml (mean±SD, n=32). It provides strong evidence to suggest that the level of Cf48 in the peripheral blood of patients with renal fibrosis is significantly higher than that of patients with lung diseases (including pulmonary fibrosis and non-pulmonary fibrosis), indicating that the increased level of Cf48 may be used as a specific indicator for renal fibrosis.

### 2.5 Conclusion

The expression level of the Cf48 small peptide is significantly increased in renal tissues of CKD patients with different causes of disease. The content of the Cf48 secretory small peptide is significantly increased in CKD patients' serum, which is significantly negatively correlated with the eGFR and is significantly positively correlated with the renal fibrosis degree and renal pathological severity. In normal mice lungs and hearts, the expression level of Cf48 is almost undetectable by using the immunohistochemical method. The expression of Cf47 detected in the Bleomycin-induced pulmonary fibrosis tissues is almost negligible, and the expression of Cf48 in the angiotensin-II-induced cardiac fibrosis tissues is low. The expression level of Cf48 in the peripheral blood of patients with lung diseases does not increase. The increase of Cf48 may be used as a specific indicator for renal fibrosis.

### 3. The role and mechanism of Cf48 small peptide in mice renal fibrosis and inflammation

3.1.1 Research purpose: to explore the role of Cf48 over-expression in renal fibrosis and inflammation during mice disease progression
3.1.2 Experimental materials
   3.1.2.1 Experimental animals: C57BL/6 mice are purchased from GemPharmatech (Jiangsu), and the breeding of Cf48 transgenic parent mice are performed by Cyagen Biosciences (Guangzhou).
   3.1.2.2 Main experimental reagents
      1) Streptozotocin (Sigma, USA); 2) folic acid (Sigma, USA); 3) mice genotype rapid identification kit (Vazyme, Guangzhou); 4) 50X TAE solution (Jingxin Biotech, Guangzhou); 5) agarose (Sigma, USA); 6) nucleic acid staining agent (Vazyme, Guangzhou); 7) PAGE gel rapid preparation kit (Epizyme, China); 8) 10X electrophoresis solution: 144g glycine+30.2g Tris base+10g SDS in 1000mL ddH20; 9) 10X membrane transfer solution: 144g glycine+30.2g Tris base in 1000mL ddH20; 10) 1X TBST solution: TBST powder (Whiga Biotech, Guangzhou) in 2000mL ddH20+2mL Tween-20; 11) Tween-20 (Sigma, USA); 12) protein stripping buffer (Thermo, USA); 13) skimmed milk powder (BD, USA); 14) BSA powder (Whiga Biotech, Guangzhou); 15) 2X SDS loading buffer (Sigma, USA); 16) pre-stained protein molecular weight marker (Thermo, USA); 17) RIPA protein lysate, (Millipore, USA); 18) protease inhibitor (Cell Signaling Technology, USA); 19) ECL luminescent liquid (Millipore, USA); 20) horseradish peroxidase (HRP) labeled donkey anti-mouse IgG (Cell Signaling Technology, USA); 21) horseradish peroxidase (HRP) labeled sheep anti-rabbit IgG (Cell Signaling Technology, USA); 22) PVDF membrane (Millipore, USA); 23) Trizol reagent (Invitrogen, USA); 24) DEPC water (Whiga Biotech, Guangzhou); 25) chemical reagents: chloroform, isopropanol, anhydrous ethanol, 95% ethanol, 75% ethanol, methanol and xylene, etc. (all analytical reagents are purchased from Guangzhou Chemical Reagent No.2 Factory); 26) transcriptor first strand cDNA synthesis kit (Roche, Switzerland); 27) Sybrgreen real-time PCR kit (Roche, Switzerland); 28) Alexa Fluro 488 labeled goat anti-mouse IgG (Life Technology, USA); 29) Alexa Fluro 488 labeled goat anti-rabbit IgG (Life Technology, USA); 30) Alexa Fluro 488 labeled donkey anti-goat IgG (Life Technology, USA); 31) Alexa Fluro 555 labeled goat anti-rabbit IgG (Life Technology, USA); 32) Alexa Fluro 647 labeled goat anti-rabbit IgG (Life Technology, USA); 33) DAPI (Thermo, USA); 34) fluorescent sealing agent (Dako, Denmark); 35) immunohistochemical kit (Dako, Denmark); 36) neutral resin (Hesi Biotech, China); 37) Cystatin-C ELISA kit (Abcam, USA); 38) Albumin ELISA kit (Abcam, USA); 39) Cretanine ELISA kit (R&D, USA); 40) Masson staining kit (Sigma, USA); 41) PAS staining kit (Powerful Biology, Wuhan); 42) antibodies: anti-ColIV antibody (Southern Biotech, USA), anti-ColI antibody (Boster Corporation, China), anti-Fn antibody (Abcam Corporation, USA), anti-F4/80 antibody and anti-α-Tubulin antibody (Cell Signaling Technology, USA), and directly labeled anti-α-SMA-Cy3 antibody (Sigma Corporation, USA); 43) The rabbit anti-mouse Cf48 antibody used for western blot experiment is prepared by Mingyan Biotech (Hangzhou). The identified antigen sequence is RALQDLRKTAYSLDARTETLLLQAE.
3.1.3 Research method
   3.1.3.1 Breeding of transgenic mice

The breeding of a pair of transgenic mice (Cf48-Tg, one male and one female) with the C57BL/6 background is performed by Syagen Biosciences. After the vector is constructed and purified, it is injected into fertilized eggs along with an infection reagent, and the fertilized eggs are transplanted into the fallopian tubes of surrogate mother mice. Positive founders are obtained after the PCR identification. The breeding is performed at the Experimental Animal Center of Sun Yat-sen University. After the mating of Cf48-Tg and Cf48-WT mice, the obtained offspring are identified by PCR to obtain Cf48-Tg and Cf48-WT mice suitable for the experiment.

The Tg and WT mice used for the experiment are male mice aged 8-10 weeks and weighed 25-30g, which are housed at the Experimental Animal Center of Sun Yat-sen University. The experimental environment meets the requirement of the SPF (specific pathogen free) animal grade, and the mice are fed with standard feed and clean water in a 12h/12h day-night cycle. The environmental temperature is maintained at 20-25°C. During the experiment, mice are free to eat and drink. This experiment has been approved by the Experimental Animal Ethics Committee of Sun Yat-sen University, and the approval number is SYSU-IACUC-2022-000134.

### 3.1.3.2 Genotype identification of transgenic mice

After the tail tissues of 4-week-old weaned mice are obtained, performing the genetype identification according to the operating process of the mouse genotype rapid identification kit (Vazyme, Guangzhou), comprising the steps of:
1) Extracting the tissue DNA: placing the tail tissues of each mouse into 100µL of lysate, adding 2µL of proteinase K, and boiling at a temperature of 56°C for 30 minutes by using the hot water bath method; after the tissues are lysed, boiling at a temperature of 98°C for 5 minutes by using the hot water bath method, thereby inactivating the proteinase K; subsequently, extracting the supernatant and storing at a temperature of -20°C for later use;

### 2) Configuring the PCR system

According to the following system, each mouse' tail tissues need:

| Reagents | Dosage |
|---|---|
| 2X Mg²⁺ mixture | 10 µL |
| Primer F | 0.8 µL |
| Primer R | 0.8 µL |
| DEPC water | 8.4 µL |
| DNA | 4uL |

The PCR primer sequences for Tg mice identification is as follows: the expected length of transgenic mice PCR product: 232bp;
Primer F: GGCAACGTGCTGGTTATTGTG;
Primer R: GTATTGCCTCTGAAAGCATGTTC.

### 3) Performing amplification following PCR procedure:

Performing the amplification according to the following PCR procedure:

| Steps | Temperature °C | Duration | Note |
|---|---|---|---|
| 1 | 94 | 2 minutes | |
| 2 | 94 | 20 seconds | |
| 3 | 65 | 15 seconds | Reducing 0.5°C per cycle |
| 4 | 68 | 10 seconds | |
| 5 | | | Repeating steps 2-4 for 10 cycles (touchdown) |
| 6 | 94 | 15 seconds | |
| 7 | 60 | 15 seconds | |
| 8 | 72 | 10 seconds | |
| 9 | | | Repeating steps 6-8 for 28 cycles |
| 10 | 72 | 2 minutes | |
| 11 | 4 | | Maintaining |

### 4) Preparing an agarose gel using horizontal electrophoresis

Placing a mold on a horizontal table, and inserting a 25-hole comb; preparing a 1XTAE solution, wherein each medium-sized mold requires 200mL of slution, adding 2% of agarose, and mixing well; heating the solution in a microwave at high temperature until it boils, and then taking out and shaking well; heating again in the microwave until it boils, and then taking out and shaking well until the solution becomes transparent and the agarose is completely dissolved; subsequently, adding 6µL of nucleic acid staining agent, mixing well, and pouring into the mold; after the solution cools down and turns into a gel, storing for later use;

### 5) Performing the horizontal electrophoresis

Placing the agarose gel in a horizontal electrophoresis tank, and adding the 1XTAE solution; mixing the PCR product well, and adding 10µL of agarose gel into each hole; after running at a constant voltage of 120V for 20 minutes, observing and photographing under the ultraviolet light.

### 3.1.3.3 Animal model preparation and grouping

### 3.1.3.3.1 Establishment and grouping of mice with streptozotocin-induced diabetic nephropathy (DN)

The method of inducing DN with streptozotocin (STZ) is the same as 1.3.2. After 55mg/kg STZ is dissolved into the 0.1 mmol/L and pH 4.5 sodium citrate solution, it is injected into the abdominal cavities of C57BL/6 background Cf48-Tg and WT mice that have undergone a 4-hour fasting. The injection lasts for 5 consecutive days, and two weeks after the STZ induction, the fasting blood glucose is measured. Mice with fasting blood glucose greater than 16.7 mmol/L are considered successfully modeled, namely, the diabetes mellitus (DM) model being successfully induced. The mice whose fasting blood glucose does not reach the standard are euthanized. Two weeks after the successful DM modeling, the glycated hemoglobin is measured. Mice with glycated hemoglobin greater than 7% are considered successfully modeled, and mice whose glycated hemoglobin does not meet the standard are euthanized. The remaining mice are successfully modeled and are euthanized 12 weeks after the DM modeling (DN group). The control mice given the same dose of sodium citrate solution is taken as the normal control group (NC group). After the DM modeling, the 4-hour random urine is respectively collected by using a metabolic cage at weeks 3, 6, 8, 10, and 12 for the detection of urinary albumin.

The Tg and WT mice are randomly divided into 4 groups using a random number table method and each group has 8-15 mice:
1) Cf48 wild-type, normal control group (WT-NC group);
2) Cf48 wild type, diabetic nephropathy group (WT-DN group);
3) Cf48 transgenic, normal control group (Tg-NC group);
4) Cf48 transgenic, diabetic nephropathy (Tg-DN group).

### 3.1.3.2.2 Construction and grouping of folic acid nephropathy mice model (FAN)

The process of inducing folic acid nephropathy (FAN) in mice using folic acid (FA) is: inducing a toxic tubular necrosis folilc acid nephropathy model by using folic acid through adopting a high-dose single injection method; dissolving 0.25mg/g FA in 0.3mol/L sodium bicarbonate solution and injecting intraperitoneally for one time; weighing 2 days after the folic acid induction; after anesthetizing, collecting a small amount of blood from intraorbital angular veins 2 days after the folic acid induction; centrifuging to obtain the serum for measuring the blood CysC two days after the folic acid induction, wherein if the CysC content in the serum is greater than 1500µg/L, the modeling is successful, and the mice that are not successfully modeled are euthanized.

The Tg and WT mice are randomly divided into 4 groups using a random number table method and each group has 8-15 mice:
1) Cf48 wild-type, normal control group (WT-NC group); Cf48 wild-type, folic acid nephropathy group (WT-FAN group); Cf48 transgenic, normal control group (Tg-NC group); and Cf48 transgenic, folic acid nephropathy group (Tg-FAN group).

### 3.1.3.4 Extraction of total protein from renal tissues

1) Cutting approximately 3mg of renal tissues from each group of mice on a cold table and placing into 1.5mL EP tubes; adding 200µL of protein lysate (1xRIPA+protease inhibitor) to each EP tube;
2) Placing a grinding bead with a diameter of 3mm to each EP tube, and grinding for two times in a grinder at a temperature of 0°C and a frequency of 60HZ for 15 seconds, wherein during the two times of grinding, there is an interval of 8 seconds;
3) After placing on ice for 30 minutes, centrifuging at a speed of 15000rpm and a temperature of 4°C for 30 minutes;
4) Extracting the protein supernatant, diluting for 20 times with ddH20, and measuring the protein concentration by using the BCA method;
5) Making the protein content in renal tissues of mice in each group consistent, wherein in each time electrophoresis, the protein content in the renal tissues of mice in each group is 50ug and the total volume is 20ul;
6) Adding a 2x loading buffer, performing air bath at a temperature of 98°C for 10 minutes to lyse the denatured protein, and storing the denatured protein at a temperature of -20°C for later use.

### 3.1.3.5 BCA method for measuring protein concentration: following the same steps in 1.3.8.

### 3.1.3.6 Protein western blot

The SDS-PAGE electrophoresis is performed on indicators with small molecular weight such as Cf48 by using the PAGE gel with a concentration of 15%, and the membrane transfer is performed by adopting the semi-drying transfer method. A semi-drying transfer buffer is prepared according to the instructions, wherein each gel block requires 100mL, and the formula is: 20mL of 5x semi-drying transfer buffer, 20mL of anhydrous ethanol, and 60mL of ddH20. Subsequently, the thick filter paper is immersed in the semi-drying transfer buffer for 30 minutes in advance, thereby enabling the filter paper to be saturated with the transfer buffer. The PVDF membrane is activated by immersing in methanol. The thick filter paper, PVDF membrane, separation gel and thick filter paper are sequentially arranged from the negative electrode to the positive electrode. A roller is used to remove bubbles, and an electrode cover is used for covering. The membrane transfer is performed at a constant current of 2.5A and a voltage of 25V for 7 minutes. The remaining steps are the same as 1.3.9.

### 3.1.3.7 Extraction of total RNA from renal tissues

1) Cutting approximately 3mg of renal tissues from each group of mice on a cold table and placing into 1.5mL EP tubes; subsequently, add ing 500µL Trizol solution to each EP tube; 2) adding a grinding bead with a diameter of 3mm to each EP tube, and grinding for 4 times in a grinder at a temperature of 4°C and a frequency of 60HZ for 15 seconds, wherein there is an interval of 8 seconds after each time of grinding; 3) after re-adding 500µL of Trizol solution to each EP tube, adding 200µL of chloroform, shaking vigorously up and down for 30 seconds to mixing up, and letting the mixture sit for 10 minutes until it is clearly divided into aqueous phase (RNA, DNA) and chloroform phase (protein); 4) centrifuging at a speed of 15000rpm at a temperature of 4°C for 30 minutes; 5) adding 500µL of isopropanol to each EP tube, extracting the top aqueous phase from the centrifuged mixture, and adding it into isopropanol; subsequently, turning upside down and mixing well, and letting it sit on ice for 2 hours; 6) centrifuging at a speed of 15000rpm at a temperature of 4°C for 30 minutes; 7) after the centrifugation is completed and when a sediment is seen, discarding the supernatant; 8) preparing 70% ethanol with anhydrous ethanol and DEPC water, adding 1mL of enzyme-free 70% ethanol to each tube, and blowing and beating; 9) centrifuging at a speed of 15000rpm at a temperature of 4°C for 5 minutes; 10) repeating steps 7-9; 11) discarding the supernatant, ensuring there is no residual ethanol, and letting it sit on ice to become dry; 12) after the periphery of the sediment becomes transparent, adding 200µL of DEPC water to each tube to dissolve the RNA; 13) measuring the RNA concentration using a spectrophotometer, freezing at a temperature of -80°C for later use or reversely transcribing to cDNA.

### 3.1.3.8 Reverse transcription reaction

Performing the reverse transcription following the operating instructions of the reverse transcription kit (Roche) by using a two-step method, comprising the steps of:
1) Preparing the reagents for the first step reaction, and the following dosage is required for each RNA sample:

| Reagents | Dosage |
|---|---|
| Oligonucleotide primer | 1µL |
| Random primer | 2µL |
| Total RNA | 2µg |
| DEPC water | Adding to 13 µL |

2) Reacting in a PCR instrument: heating at a temperature of 65°C and reacting for 10 minutes;
3) Preparing reagents for the second step reaction, adding 7µL into each well in the already reacted system, and then mixing well:

| Reagents | Dosage |
|---|---|
| 5x reverse transcription reagent | 4µL |
| RNA inhibitor | 0.5µL |
| dNTP | 2µL |
| Reverse transcriptase | 0.5µL |

4) Reacting in the PCR instrument: reacting according to the following process:

| Steps | Temperature °C | Duration |
|---|---|---|
| 1 | 25 | 10 minutes |
| 2 | 50 | 60 minutes |
| 3 | 85 | 5 minutes |

5)After the reverse transcription is completed, measuring the cDNA concentration using a spectrophotometer and freezing at a temperature of -20°C for later use.

### 3.1.3.9 Real-time fluorescence quantitative PCR (qRT PCR)

Performing the real-time fluorescence quantitative PCR according to the operating instructions of the real-time fluorescence quantitative PCR kit (Roche), comprising the steps of:
1)Diluting the cDNA: diluting the cDNA for 20 times using DEPC water, thereby allowing the total amount of cDNA in the final system to be 20ng;
2) Preparing a reaction mixture, wherein the amount of mixture used in each well is as follows:

| Reagents | Dosage |
|---|---|
| Forward primer | 0.2µL |
| Reverse primer | 0.2µL |
| SYBR Green | 5µL |
| DEPC water | 0.6 µL |

3) Adding into microplate: adding 4µL of cDNA and 6µL of reaction mixture into each well of a 96-well or 384-well microplate;
4) Reacting in the real-time fluorescence quantitative PCR instrument, wherein the reaction system is as follows:

| Step | Temperature °C | Duration | Note |
|---|---|---|---|
| 1 | 95 | 10 minutes | |
| 2 | 95 | 15 seconds | |
| 3 | 58 | 30 seconds | Reading plate |
| 4 | 72 | 30 seconds | |
| 5 | | | Repeating steps 2-5 for 40 cycles |
| 6 | 95 | 10 seconds | |
| 7 | | | Analyzing melting curve+reading plate |

The qRT-PCR primer synthesis is performed by BGI (Shenzhen), and the specific sequences are as follows:

| Gene(Mouse) | | Primer sequence |
|---|---|---|
| Cf48 | Forward primer | TGTCGCCACGGACTTTACTG |
| | Reverse primer | TAGGCGGTTTTCCGTAGGTC |
| TNF-α | Forward primer | TCGTAGCAAACCACCAAGTG |
| | Reverse primer | CCTTGAAGAGAACCTGGGAG |
| MCP-1 | Forward primer | CCCACTCACCTGCTGCTAC |
| | Reverse primer | TTCTTGGGGTCAGCACAGA |
| IL-1β | Forward primer | ACTGTGAAATGCCACCTTTTG |
| | Reverse primer | TGTTGATGTGCTGCTGTGAG |
| β-Actin | Forward primer | GACATGGAGAAGATCTGGCA |
| | Reverse primer | GGTCTTTACGGATGTCAACG |

### 3.3.3.10 Mice blood glucose and glycated hemoglobin measurement

After the mice undergo a fasting for 6 hours, a small portion of tail tissues is cut off in a quiet environment.

The fasting blood glucose measurement: inserting a blood glucose test strip into a blood glucose meter; after the glucose meter prompts a test, squeezing a drop of blood from the tail tip, dropwise adding it into the blood glucose test strip, and waiting for 3 seconds to read the recorded result;

Measuring the glycated hemoglobin following the operating instructions of the glycated hemoglobin analyzer (Sinocare), comprising the steps of:
1) Re-warming the reagents for 30 minutes;
2) Turning on: inserting a password card attached to the reagent kit into a glycated hemoglobin analyzer and verifying the information on the password card;
3) Collecting a sample: squeezing the blood out from the tail tip until the blood reaches the top of the capillary tube of a sampler; subsequently, inserting the sampler into a R1 reagent container, vigorously shaking the blood sample in the capillary tube into the R1 reagent, shaking well, and letting it sit for 2 minutes;
4) Adding the sample: dropwise adding the R1 mixture onto the glycated hemoglobin test strip while avoiding the formation of bubbles, then vertically dropwise adding the R2 reagent onto the test strip, and letting it sit for 15 seconds, thereby ensuring that the R2 reagent fully penetrates the membrane of the test strip;
5)Reading test results: inserting the test strip into the glycated hemoglobin analyzer and waiting for 30 seconds to read the recorded results.

### 3.1.3.11 Mice renal function experiment

During the experiment, 200µL of mice whole blood is collected from the angular vein two days after the FA induction and is let sit at room temperature for 1 hour. Subsequently, it is centrifuged at a speed of 3000rpm at room temperature for 10 minutes, and the upper-layer serum is extracted for mice blood CysC detection;

The whole blood is collected from the abdominal aorta of mice after anesthesia during the euthanasia. It is centrifuged at room temperature for 1 hour at a speed of 3000rpm for 10 minutes. Subsequently, the upper-layer serum is extracted for the detection of creatinine, blood urea nitrogen and blood CysC at the disease endpoint of mice. The blood creatinine and blood urea nitrogen of mice are detected by using a fully automatic biochemical analyzer, and the mice blood CysC is detected by using ELISA (Abcam, USA). The serum is diluted for 2000 times following the operating instructions, and the specific steps are the same as 2.3.6.

### 3.1.3.12 Mice urine albumin detection

The 4-hour random urine of DN mice and NC mice are respectively collected by using a metabolic cage at weeks 3, 6, 8, 10, and 12. The mice urine albumin (Albumin, Abcam, USA) and urine creatinine (Cretanine, R&D, USA) are detected by using ELISA. The urine is diluted for 2000 times according to the operating instructions. The detection of urine creatinine adopts the original urine, and the specific steps are the same as 2.3.6. The ratio of urine albumin/creatinine is used to reflect the urine albumin level of mice.

### 3.1.3.13 Mice renal tissue pathological staining

1) PAS staining: according to the operating instructions, performing the PAS staining, comprising the steps of: a) deparaffinating and hydrating paraffin slides with a thickness of 5µm, wherein the steps are the same as 2.3.3 1)-2); b) washing for 1-2 minutes using distilled water; c) oxidating using a periodic acid solution for 10 minutes; d) washing using distilled water to remove the floating colors; e) staining using the Schiff reagent for 30 minutes; f) washing using distilled water to remove floating colors; g) performing Mayer hematoxylin nuclear staining for 3 minutes; h) washing using distilled water to remove floating colors; i) performing routine dehydration and clearing, and then mounting using neutral resin, wherein the steps are the same as 2.3.3 11); k) observing and photographing under an upright microscope or scanning using a fully automatic slide scanner;
2) Masson staining: performing the masson staining following the operation instructions, comprising the steps of: a) deparaffinating and hydrating the paraffin slides with a thickness of 5µm, wherein the steps are the same as 2.3.3 1) -2); b) preparing a 1% acetic acid solution (200mL inddH20), a solution containing hematoxylin (100mL A solution+100mL B solution), and a phosphate solution (50mL C solution+50mL D solution+100mL ddH20); e) staining using the solution containing hematoxylin for 5 minutes; f) washing using running water for 5 minutes to remove floating colors, then washing with ddH20; g) staining using a magenta solution for 5 minutes; h) washing using ddH20 to remove floating colors; i) staining using the phosphate solution for 5 minutes; j) washing using ddH20 to remove floating colors; k) staining using an anline blue solution for 20 minutes; l) washing using ddH20 to remove floating colors; m) washing up and down for two times using the 1% acetic acid solution, and removing the floating colors; n) performing routine dehydration and clearing, and mounting using the neutral resin, wherein the steps are the same as 2.3.3 11); o) observing and photographing under an upright microscope or scanning using a fully automatic slide scanner.

### 3.1.3.14 Mice renal tissue immunohistochemical/immunofluorescence staining

Paraffin slides with a thickness of 5µm are taken for use following the same steps as 2.3.3 and 2.3.4.

### 3.1.3.15 Immunohistochemical F4/80⁺ macrophage quantification

Ten renal cortex fields of view are randomly selected from the slides scanned in the fully automatic slide scanner under a high power microscope (x400) by using a single blind method. The selected fields of view should avoid glomeruli and blood vessels. The number of F4/80⁺ macrophages in each field of view should be quantified, and the average number of macrophages in 10 fields of view is taken as the number of F4/80⁺ macrophages in the renal tubulointerstitial region of the mice.

### 3.1.3.16 Statistical method: the method for statistical analysis is the same as 2.3.7.

### 3.1.4 Experimental results

### 3.1.4.1 Identification of Cf48 transgenic mice

The gene identification is performed on newborn mice. Based on the genotype identification of the mice, PCR results show that the Cf48Tg mice have an expression product with a length of 232bp. The western blot results show that the expression of Cf48 protein in the renal tissues of Cf48Tg mice is significantly increased compared with the WT group.

### 3.1.4.2 Increased expression of Cf48 in renal tissues of DN mice

Through western blot, qRT-PCR, and immunofluorescence detection of Cf48 expression in renal tissues of DN mice, the results show that the mRNA expression and protein expression of Cf48 in renal tissues of DN mice are significantly increased compared to those in renal tissues of DN mice. The immunofluorescence results show that the expression of Cf48 in the renal tissues of DN mice is high and the expression is mainly concentrated in the renal tubules and interstitium. As shown in Figure 3-1, by means of co-staining, it is found that Cf48 has co-localization with α-SMA positive myofibroblasts.

### 3.1.4.3 Declination of renal function of mice with Cf48 over-expression diabetic nephropathy

The results of blood glucose measurement show that the blood glucose and glycosylated hemoglobin of DN mice are significantly higher than those of NC mice and have reached the levels of diabetes. There is no significant difference in the fasting blood glucose and glycated hemoglobin levels between the WT and Tg groups of mice. As shown in Figures 3-2A and B, the above results indicate that the over-expression of Cf48 does not change the glucose tolerance of mice. As shown in Figure 3-2C, the kidney weight/body weight ratio of Tg-DN mice is significantly higher than that of WT-DN mice, indicating that the renal ultrafiltration capacity of mice with Cf48 over-expression diabetic nephropathy is higher.

The urine albumin of WT-DN and Tg-DN mice during the progression of diabetes nephropathy is dynamically observed. The results show that the excretion of urine albumin of DN mice gradually increases along with the progress of diabetes nephropathy, indicating that the modeling of diabetes nephropathy is successful. The urine albumin/creatinine ratio of the two groups of mice in the early stage (3 weeks) of diabetes nephropathy has no significant difference. As shown in Figures 3-2D and 3-2E, in the middle stage (6 weeks) of diabetic nephropathy, the urine albumin of Tg mice is significantly higher than that of WT mice, and the high urine albumin excretion of Tg mice continues to the late stage (12 weeks) of diabetes nephropathy at the time of euthanasia.

The renal function of mice is reflected by detecting the level of cystatinC (CysC) in their blood. As shown in Figure 3-2F, during the euthanasia, the CysC in the serum of DN mice is significantly higher than that of normal control mice, indicating severely declined renal function of mice in this model. The difference of CysC levels in the serum of Tg mice and WT mice in the NC group has no statistical significance, while the CysC levels in the serum of Tg mice in the DN group is significantly higher than those of WT mice in the DN group. These results show that the over-expression of Cf48 accelerates the declination of renal function of mice with diabetic nephropathy under the condition that the glucose tolerance is not changed.

### 3.1.4.4 Aggravation of renal pathological injury of mice with Cf48 over-expression diabetic nephropathy

PAS results show that, compared with NC mice, DN mice have thickened glomerular basement membrane, increased number of glomerular mesangial cells and fibrously thickened mesangial area, which are consistent with the features of diabetic nephropathy. As shown in Figure 3-3, compared with WT mice, Tg mice show more severe injury by diabetic nephropathy, more significant thickening of renal tubular basement membrane and more significant increase of extracellular tubule interstitium.

### 3.1.4.5 Deterioration of renal fibrosis in mice with Cf48 over-expression diabetic nephropathy

The immunofluorescence results show that the extracellular matrix CollagenIV (ColIV), fibronectin (Fn) and myofibroblast marker α-smooth muscle actin (α-SMA) in DN mice are significantly increased compared to NC mice. In the DN model, the Tg mice show a more significant increase in ColIV, Fn and α-SMA compared to the WT mice, indicating a deterioration of renal interstitial fibrosis in the Tg-DN mice. Compared with WT mice, Tg-DN mice show significantly increased deposition of ColIV in their glomeruli, indicating that the over-expression of Cf48 also leads to the deterioration of glomerulosclerosis in the DN mice. As shown in Figure 3-4, the western blot results show that the Tg mice has significantly increased α-SMA compared to the WT mice, which is consistent with the immunofluorescence results.

### 3.1.4.6 Exacerbation of inflammation in mice with Cf48 over-expression diabetic nephropathy

F4/80 is a marker for macrophages. To observe the renal inflammation in mice, the number of F4/80⁺ macrophages in the renal cortex of each group of mice is counted by using the immunohistochemical method. The results show that there is only a small number of macrophages in the normal mice without modeling, and there is no significant difference in the number of macrophages between the WT and Tg groups of mice. As shown in Figure 3-5A, the number of F4/80⁺ macrophages in the renal cortex of the DN mice is significantly increased compared to the NC mice, and the number of F4/80⁺ macrophages in the Tg-DN mice is significantly increased compared to the WT-DN mice. It shows a statistical difference.

To further observe the renal inflammatory response of mice in each group, the qRT-PCR is used to detect the expression levels of inflammation indicators MCP-1 and TNF-α mRNA in renal tissues. As shown in Figure 3-5B, the results show that the expression levels of MCP-1 and TNF-α mRNA are significantly increased in the DN mice compared to the NC mice, and the expression levels of inflammation indicators are further increased in the Tg-DN mice compared to the WT-DN mice.

### 3.1.4.7 Increased expression of Cf48 in renal tissues of FAN mice

The QRT-PCR is used to respectively detect the mRNA expression of Cf48 in renal tissues on the 2, 7, 14, and 28^{th} day after the injection of folic acid. The experimental results show that, the mRNA level of Cf48 is significantly increased on the 2^{nd} day after the induction of folic acid nephropathy (FAN), and along with the progression of the disease, the mRNA level of Cf48 is gradually increased. The expression level of Cf48 is the highest on the 28^{th} day after the induction of FAN. The FAN-28d (days) model is chosen as the research object.

Western blot results show that the protein expression level of Cf48 in the renal tissues of FAN-28d is significantly increased compared to normal renal tissues. As shown in Figure 3-6, the immunofluorescence results show a significant increase in the expression of Cf48 in the FAN model. The expression of Cf48 is mainly concentrated in the renal tubules and is more significant in severely dilated and injured renal tubules.

### 3.1.4.8 Declination of renal function of mice with Cf48 over-expression folic acid nephropathy

On the 2^{nd} day after the folic acid modeling, the blood CysC of the FA mice is significantly increased, which is consistent with the features of the acute kidney injury. On the 28^{th} day after the folic acid modeling, the blood CysC, Creatinine (Cr), and blood urea nitrogen (BUN) levels in the FA mice are significantly increased compared to NC mice, indicating the successful modeling of the folic acid nephropathy. In the FA group, compared with the WT mice, the Tg mice show significant increase in blood CysC and blood Cr, indicating a statistical difference. As shown in Figure 3-7, the blood urea nitrogen shows an upward trend, indicating that the over-expression of Cf48 leads to the declination of renal function of mice on the 28^{th} day of the FA induction.

### 3.1.4.9 Aggravation of renal pathological injury of mice with Cf48 over-expression folic acid nephropathy

On the 28^{th} day after the FA induction, a significant shrinkage of mice kidneys is observed. The kidney surface is obviously granular and uneven. The Masson staining results show that the renal tubules of mice with folic acid nephropathy are significantly dilated. The arrangement of renal tubules is disordered, the tubule necrosis occurs, and part of the necrotic tubules fall off into the lumen to form cellular casts. The renal interstitial edema is significant and accompanied by inflammatory cell infiltration. The Masson staining also shows a significant increase in collagen deposition and significant renal tissue fibrosis in the FA mice. In the FA group, compared with the WT mice, the Tg mice show more severe tubular injury, and the necrosis of a large number of tubular cells, increased number of cellular casts and significantly increased collagen deposition are observed. The Masson staining shows that the collagen deposition ratio in the kidneys of the FA mice is significantly higher than that of the NC mice. In the FA group, the collagen deposition ratio in the Tg mice is significantly higher than that in the WT mice, and the renal fibrosis degree of the Tg mice is more severe.

### 3.1.4.10 Deterioration of renal fibrosis in mice with Cf48 over-expression folic acid nephropathy

The immunofluorescence results show that the expression of ColIV and Fn in the NC mice are mainly concentrated in their basement membrane and the morphology of the membrane is regular, while the expression of α-SMA is mainly concentrated in blood vessels. The staining results show that the expression levels of the ColIV, Fn, and α-SMA in renal tissues of the FA mice are significantly increased, the membrane is significantly thickened, the arrangement is disordered, and the expression of ColIV, Fn, and α-SMA is mainly distributed in the renal interstitium. In the FA model, the increase of renal fibrosis factors is more significant in the Tg mice compared to the WT mice. As shown in Figure 3-8, the western blot results show that, in the FA group, the expression levels of ColI and α-SMA in the Tg mice are significantly increased, which are consistent with the immunofluorescence results.

### 3.1.4.11 Exacerbation of inflammation in mice with Cf48 over-expression folic acid nephropathy

The immunohistochemical results show that the number of F4/80⁺ macrophages in the FA mice is significantly increased compared to the NC mice, and the inflammation of renal tissues of mice with folic acid nephropathy becomes more severe. In the FA model, the number of F4/80⁺ macrophages in the Tg mice is significantly increased compared to WT mice, indicating a statistical difference. As shown in Figure 3-9, the qRT-PCR results show that the mRNA expression levels of inflammation indicators MCP-1, TNF-α and IL-1 β in the FA mice are significantly higher than those in the NC mice, and the expression levels of inflammation indicators in the Tg mice are further increased compared to the WT mice.

### 3.2 The effect of Cf48 gene knockout on models of mice with folic acid nephropathy and obstructive nephropathy

### 3.2.1 Research purpose: to verify the effect of Cf48 gene knockout on the progression of renal inflammation and fibrosis in mice

### 3.2.2 Experimental Materials: the C57BL/6 mice are purchased from GemPharmatech (Jiangsu), and the breeding of the Cf48 gene knockout parent mice is performed by Cyagen Bioscience.

### 3.2.3 Research method

### 3.2.3.1 Breeding of Cf48 gene knockout mice

The breeding of a pair of Cf48 +/- mice (one male and one female) with the C57BL/6 background is performed by Syagen Biosciences. The design of sgRNA adopts the CRISPR/Cas9 technology. After performing the high-throughput electroporation on fertilized eggs and identifying using PCR, the Cf48 +/- founder mice are obtained. The breeding is performed at the Experimental Animal Center of Sun Yat-sen University (Normal Campus). After the mating of the male and female Cf48-HZ mice, the obtained offspring are identified by PCR to obtain Cf48 -/-(KO) and Cf48 +/+(WT) mice suitable for the experiment.

The KO and WT mice used for the experiment are male mice aged 8-10 weeks and weighed 25-30g, which are housed at the Experimental Animal Center of Sun Yat-sen University. This experiment has been approved by the Experimental Animal Ethics Committee of Sun Yat-sen University, and the approval number is SYSU-IACUC-2022-000134.

### 3.2.3.2 Identification of genotype of Cf48 knockout mice

The steps of performing the mice tail DNA extraction, PCR amplification, gel preparation and horizontal electrophoresis are the same as 3.1.3.2.

The PCR primer sequences for the identification of KO mice is as follows:
The expected length of the PCR product of the KO mice: 246bp;
Primer F1: TTGGTCTCCCACACGAATGG,
Primer R1: CCAGAGCTCAGCTTCCACAA.

The PCR primer sequences for the identification of WT mice is as follows:
The expected length of the PCR product of the WT mice: 265bp;
Primer F2: GAGCAGGCATCCACTCCTTT,
Primer R2: AGAGCTCTCCTCCTTCCCAG.

The PCR procedure is as follows:

| Step | Temperature°C | Duration | Note |
|---|---|---|---|
| 1 | 94 | 2 minutes | |
| 2 | 94 | 30 seconds | |
| 3 | 60 | 30 seconds | |
| 4 | 72 | 45 seconds | |
| 5 | | | Repeating step 2-4 for 35 cycles |
| 6 | 72 | 10 minutes | |
| 7 | 4 | | Maintaining |

### 3.2.3.3 Animal model preparation, grouping, and research plan

### 3.2.3.3.1 Establishment of model and grouping of mice with folic acid nephropathy (FAN)

The method for folic acid nephropathy modeling is the same as 3.1.3.2.2. The KO and WT mice are randomly divided into four groups using a random number table method, and each group has 8-15 mice: 1) Cf48 wild-type, normal control group (WT-NC group); 2) Cf48 wild-type, folic acid nephropathy group (WT-FAN group); 3) Cf48 gene knockout, normal control group (KO-NC group); 4) Cf48 gene knockout, folic acid nephropathy group (KO-FAN group).

### 3.2.3.3.2 Establishment of model and grouping of mice experienced unilateral ureteral obstruction (UUO)

The modeling of mice experienced unilateral ureteral obstruction (UUO), comprising the steps of: performing the anesthesia using pentobarbital (45mg/kg) on the UUO group of mice (UUO), and performing routine surgical disinfection and skin preparation; cutting an opening near the abdominal line at the root of the right thigh, separating the skin and muscle tissues, thereby exposing the abdominal cavity; finding the slanted ureter along the posterior abdominal wall; subsequently, separating the ureter, ligating at the proximal and distal ends of the ureter, and then cutting off the ureter; returning the organs to the abdominal cavity and sequentially suturing the muscle layer and skin layer. For the sham group of mice, the ligation is not performed after the ureter is separated, and the remaining steps are the same as the UUO group. Seven days after the surgery, the mice are euthanized for testing.

The KO and WT mice are randomly divided into four groups using a random number table method, and each group has 8-15 mice: 1) Cf48 wild-type, Sham group (WT-Sham group); 2) Cf48 wild-type, UUO group (WT-UUO group); 3) Cf48 gene knockout, Sham group (KO-Sham group); 4) Cf48 gene knockout, UUO group (KO-UUO group).

### 3.2.3.4 Other research methods are the same as those in 3.1.3

### 3.2.4 Experimental results

### 3.2.4.1 Identification of Cf48 gene knockout mice

The results of the genotype gel electrophoretogram of newborn mice show that, the Cf48-/- mice are able to amplify a target product with a length of 246bp by using the P1F1 (KO) primer but unable to amplify by using the P2F2 (WT) primer, the Cf48+/+ mice are unable to amplify the product by using the P1F1 (KO) primer but able to amplify a product with a length of 265bp by using the P2F2 (WT) primer, and the Cf48+/- mice are able to amplify the corresponding products by using both the P1F1 (KO) primer and P2F2 (WT) primer. According to the aforesaid, the identification of the genotype of the mice is achieved.

### 3.2.4.2 Reduction of kidney injury degree of mice with Cf48 knockout folic acid nephropathy

On the 2^{nd} day after the folic acid modeling, the blood CysC of the FA mice is significantly increased, which conforms with the features of an acute kidney injury and is consistent with the previous experimental results. On the 28^{th} day after the folic acid modeling, the blood CysC, blood Cr and blood BUN of the FA mice are significantly increased compared to the NC mice. In the FA group, compared with the WT mice, the KO mice show a significant decrease in the increased blood CysC, blood Cr, and blood BUN levels, indicating a statistical difference. However, as shown in Figure 3-10, the decreased levels do not return to normal renal function levels.

### 3.2.4.3 Reduction of renal pathological injury in mice with Cf48 knockout folic acid nephropathy

By means of the Masson staining, it is observed under a light microscope that the renal morphology of the NC mice is basically normal and the arrangement of tubular cells is regular. On the 28^{th} day after the folic acid modeling, the Masson staining results show that the changes of the renal morphology of mice are the same as previous results. Under the light microscope, obvious renal interstitial fibrosis, severe inflammatory cell infiltration, necrosis of tubular epithelial cells, collapse of tubules, and cell casts are observed. As shown in Figure 3-11, the severity of tubular injury in the KO-FA mice is reduced compared to WT-FA mice, and the area of renal interstitial fibrosis is significantly reduced.

### 3.2.4.4 Reduction of renal fibrosis degree of mice with Cf48 knockout folic acid nephropathy

The immunofluorescence staining results are consistent with the previous results, indicating that the expression of extracellular matrix in the NC mice is mainly concentrated in the basement membrane and the renal morphology is normal, while the expression of α-SMA is mainly concentrated in blood vessels. The staining results show that the expression levels of ColIV, Fn and α-SMA in the renal tissues of the FA mice are significantly increased, the arrangement of tubular cells is disordered, and the expression of ColIV, Fn and α-SMA is mainly distributed in the renal interstitium. Compared with the WT mice, the KO mice show reduced staining intensity and area of fibrosis indicators. As shown in Figure 3-12, the western blot results show that, in the FA model, the expression levels of ColI and α-SMA are significantly reduced in the KO mice compared to the WT mice, which are consistent with the immunofluorescence results.

### 3.2.4.5 Reduction of inflammation in mice with Cf48 knockout folic acid nephropathy

The immunohistochemical results show a significant increase in the number of F4/80⁺macrophages in the FA mice and a significant inflammatory infiltration of renal cortex in mice with folic acid nephropathy. There is no statistically significant difference in the proportion of F4/80⁺ macrophages between the KO mice and the WT mice in the NC group. In the FA model, the proportion of F4/80⁺ macrophages in the KO mice is lower than that in the WT mice, and the difference has a statistical significance. As shown in Figure 3-13, the qRT-PCR results show that the expression levels of inflammation indicators MCP-1 and TNF-α in the FA mice are significantly higher than those in the NC mice, while the mRNA expression levels of inflammation indicators in the KO mice are significantly lower than those in the WT mice.

### 3.2.4.6 Increased expression of Cf48 in renal tissues of UUO mice

The Cf48 expression in the renal tissues of the UUO mice is detected by using the qRT-PCR. As shown in Figure 3-14, the results show that the mRNA expression level of Cf48 in the UUO mice is significantly higher than that in the renal tissues of the Sham mice.

### 3.2.4.7 Reduction of renal pathological injury in Cf48 knockout UUO mice

By means of the Masson staining, it is observed under the light microscope that the tubular cells in the Sham group of mice are arranged regularly, the basement membrane is intact, no edema exists in the renal interstitium, and no deposition of extracellular matrix exists. The renal tubules of the UUO mice are highly dilated, the necrosis and exfoliation of some cells occur, the extracellular matrix is significantly increased, a large amount of collagen deposition exists, and obvious renal tissue fibrosis occurs. As shown in Figure 3-15, in the Sham group, there is no significant difference in renal morphology between the WT and KO mice, and in the UUO group, the deposition of renal interstitial collagen is significantly reduced in the KO mice compared to the WT mice.

### 3.2.4.8 Reduction of renal fibrosis in Cf48 knockout UUO mice

Through the immunofluorescence staining, it is found that the level of extracellular matrix in the Sham mice is normal, which is mainly distributed in the basement membrane and blood vessels. The expression levels of ColIV, Fn and α-SMA in the renal interstitium in the UUO mice are significantly increased, the basement membrane is significantly thickened, and the renal interstitial fibrosis is obvious. Compared with the WT mice, the KO mice show a significant decrease in the expression of extracellular matrix and a reduced degree of fibrosis.

### 3.2.4.9 Reduction of renal inflammation in Cf48 knockout UUO mice

The immunohistochemical results show that the morphology of renal interstitium of the Sham mice is normal, and only the infiltration of a small mount of F4/80⁺ macrophages around the blood vessels is observed. The UUO mice show significant dilation of tubules and significant increase in inflammatory cells, and the aggregated inflammatory cells are sheet-shaped. The inflammatory cells in the KO mice are significantly reduced compared to the WT mice, indicating a statistical difference. The qRT-PCR results show that the expression levels of inflammation indicators MCP-1 and TNF-α in the UUO mice are significantly higher than those in the NC mice. As shown in Figure 3-16, in the UUO group, the expression levels of inflammation indicators in the KO mice are significantly reduced compared to the WT mice.

### 3.3.1 Research purpose

To explore whether intervening in the expression of C4orf48 in kidney by injecting LNA C4orf48 can alleviate the renal fibrosis and delay the progression of DKD in UUO and STZ-DKD models (Nos3-/- mice).

In this research, mice fibrosis models including the UUO and progressive STZ-DKD models (Nos3-/- mice) are constructed first, and then the mice are injected with the locked nuclei acid (LNA) anti-sense oligonucleotide C4orf48(LNA C4orf48) to reduce the expression of endogenous C4orf48 in the mice kidneys. By intervening the expression of C4orf48, whether the renal fibrosis of the UUO and STZ-DKD mice is alleviated and the progression of STZ-DKD is delayed can be observed.

### 3.3.2 Experimental materials

Control LNA CTL: 5'-AACACGTCTATACGC-3', wherein the first "A" and the last "C" are both LNAs, and the entire sequence contains a phosphorothioate skeleton. LNA1 C4orf48: 5'-CGCTGCATGAATTCAA-3', wherein the first "A" and the last "C" are both LNAs, and the entire sequence contains a phosphorothioate skeleton. LNA2 C4orf48: 5'-CCGTAGGTCCTGAAGG-3', wherein the first "A" and the last "C" are both LNAs, and the entire sequence contains a phosphorothioate skeleton. The LNAs are synthesized by GeneBiogist (Shanghai).

### 3.3.3 Research method

The sham surgery (sham) or UUO surgery is performed on 8-12 week old C57BL6/J male mice. The UUO group of mice are randomly divided into three groups: intraperitoneal injection of LNA CTL (10mg/kg) group, LNA1 C4orf48 10mg/kg group, LNA2 C4orf48 10mg/kg group, and LNA1 C4orf48 10mg/kg+LNA2 C4orf48 10mg/kg group, wherein each group consists of 8 mice. On the 6^{th} day after the surgery, the same dosage of solvent is intraperitoneally injected into the mice again. On the 7^{th} day after the surgery, the mice are euthanized and their kidney tissues are collected for various analyses. The WB results show that the expression of UUO kidney C4orf48 is significantly inhibited in mice of the LNA1 C4orf48 10mg/kg+LNA2 C4orf48 10mg/kg group. The mice is intraperitoneally injected with LNA1 C4orf48 10mg/kg+LNA2 C4orf48 10mg/kg once a week as a treatment for STZ-DKD. The 10-12 week old Nos3-/- male mice are intraperitoneally injected with STZ 55mg/kg for 5 consecutive days. After 2 weeks, the fasting blood glucose of the mice is measured, and the fasting blood glucose greater than 250mg/dl is diagnosed as diabetes. Mice with diabetes are randomly divided into two groups: one group is given intraperitoneal injection of LNA CTL (20mg/Kg) once a week, and the other group is given intraperitoneal injection of 10mg/kg of LNA1 C4orf48 and 10mg/kg of LNA2 C4orf48 once a week for 6 consecutive weeks. The mice are euthanized at week 7. In addition, the 10-12 week old Nos3-/- male mice are given intraperitoneal injection of the same dosage of solvent for 5 consecutive days as the control group, wherein each group consists of 12-14 mice. The material collection, such as the collection of blood and urine, as well as the collection of tissues of kidney, liver, spleen, heart and other organs is the same as described in 3.1.3.

### 3.3.4 Experimental results

### 3.3.4.1 Injection of LNA Cf48 reduces the expression of UUO kidney Cf48

The western blot results show that the expression level of Cf48 in the sham group of mice is very low, while in the 7-day UUO group, the expression level of Cf48 is increased significantly. The injection of LNA1 Cf48 or LNA2 Cf48 is capable of reducing the expression of Cf48. As shown in Figure 3-17, the combined injection of LNA1 Cf48 and LNA2 Cf48 further inhibits the expression of Cf48, and the inhibition rate reaches over 90%. The weekly combined injection of LNA1 Cf48 and LNA2 Cf48 sufficiently inhibits the expression of endogenous Cf48 in the mice kidneys.

### 3.3.4.2 Injection of LNA Cf48 alleviates the UUO renal fibrosis

The western blot results show that the combined injection of LNA1 Cf48 and LNA2 Cf48 reduces the production of extracellular matrix α-SMA and Collagen I in UUO mice, indicating that the injection of LNA1 Cf48 and LNA2 Cf48 is capable of alleviating the renal fibrosis. As shown in Figure 3-18, it suggests that intervening in the expression of Cf48 achieves the goal of inhibiting the renal fibrosis.

### 3.3.4.3 Injection of LNA Cf48 reduces the production of STZ-DKD proteinuria, alleviates the renal fibrosis and delays the declination of renal function

There is no difference in the measured fasting blood glucose and glycated hemoglobin between the STZ-DKD+LNA CTL group and the STZ-DKD+LNA Cf48 group. The immunohistochemical results of Collagen IV show that the treatment with LNA Cf48 alleviates the STZ-DKD renal fibrosis. As shown in Figure 3-19, compared with the STZ-DKD+LNA CTL group, the STZ-DKD+LNA Cf48 group has reduced production of proteinuria. The LNA Cf48 treatment also reduces the levels of STZ-DKD blood Cr and Cystatin-c, indicating that the LNA Cf48 treatment is capable of delaying the STZ-DKD renal function declination.

In conclusion, the injection of LNA Cf48 alleviates the renal fibrosis and delays the occurrence and progression of STZ-DKD. It proves that the Cf48 is capable of serving as a target, and the drugs with controlled content of Cf48 may be used for controlling kidney disease and renal fibrosis.

### 4. Molecular mechanism of Cf48 small peptide in promoting renal fibrosis

### 4.1 Research purpose

To explore the direct effects of the Cf48 small peptide on fibroblasts and tubular cells in vitro and screen downstream molecules of Cf48 for investigating the molecular mechanism of Cf48 in promoting fibroblast activation and EMT of tubular cells.

### 4.2 Experimental materials

### 4.2.1 Experimental cells

The mice renal fibroblasts (NRK-49F), mice renal tubular cells (NRK-52E) and human embryonic renal cells (293T) are purchased from ATCC (American Type Culture Collection, USA).

### 4.2.2 Main experimental apparatuses and devices

1) Clean bench (Esco, Singapore); 2) 5% CO₂ incubator (ThermoClectron Corporation, USA); 3) cell counter (Thermo, USA); 4) high pressure sterilization pot, (Hirayama, Japan); 5) electric constant temperature drying oven (Guangzhou Dongfang Electric Drying Equipment); 6) reverse mixing reaction holder (Thermo, USA); 7) ultrasonic cell disruptor (Helser UP200S, Germany); 8) SeahorseXFe/XF analyzer (Agilent, USA); 9) others: same as 3.1.2.2.

### 4.2.3. Main experimental consumables

1) 75cm² cell culture bottle (Corning, USA); 2) cell culture dish (Corning, USA); 3) 96-well cell plate and 9-well cell plate (Corning, USA); 4) cell counting plate (Thermo, USA); 5) 22µm cell sieve (Corning, USA); 6) 2mL cryovial (Corning, USA); 7) chamber slide (Thermo, USA); 8) others: same as 3.1.2.3.

### 4.2.4 Main experimental reagents

1) Fetal bovine serum (GIBCO, USA); 2) DMEM/F12 medium (GIBCO, USA); 3) 0.25% trypsin (GIBCO, USA); 4) penicillin/streptomycin, (GIBCO, USA); 5) TGF-β1 recombinant protein (R&D, USA); 6) lipofectamine 3000 (Thermo, USA); 7) Opti-MEM medium (GIBCO, USA); 8) protein A/G agarose (Santa Cruz, USA); 9) blasticidin S (Solarbio, China); 10) puromycin (Sigma, USA); 11) polybrene transfection reagent (Genomeditech, China); 12) seahorse related reagents: RPMI medium, pyruvic acid, glutamine, glucose, oligomycin, FCCP, and rotenone/antimycin A purchased from Agilent (USA); 13) antibodies: directly labeled anti-α-SMA-Cy3 antibody and anti-α-SMA antibody purchased from Sigma Corporation (USA), anti-His antibody and anti-Pax8 antibody purchased from Abcam (USA), anti-α-Tubulin antibody purchased from Cell Signaling Technology (USA), and anti-SLC3A2 antibodies respectively purchased from Thermo (USA) and Cell Signaling Technology (USA); 14) SLC3A2-sgRNA-CCRISPR/Cas9 virus and control virus synthesized by Genomeditech (Shanghai); 15) SLC3A2-siRNA and CTL-siRNA synthesized by Genebio (Shanghai); 16) Cf48 recombinant protein synthesized by Atagenix (Wuhan); 17) HCf48-his-GFP plasmid synthesized by Genewiz; 18) others: same as 3.1.2.4.

### 4.3 Research method

### 4.3.1 Synthesis of Cf48 recombinant protein

The synthesis of the Cf48 recombinant protein is performed by Atagenix (Wuhan). The specific process comprising the steps of:
1) Performing gene synthesis and expression vector cloning
   a) Synthesizing the Fc-labeled Fc-Cf48 cDNA sequence and connecting it to the expression vector pATX1 (cloning site: EcoRI/NotI);
   b) Transforming the expression vector Fc-Cf48-pATX1 into CHO cells (tool cells: Chinese hamster ovary cells) and performing CHO cell expansion to obtain as many produced amino acids as possible, wherein the expected produced amino acid sequence is as follows:

MKHLWFFLLLVAAPRWVLSCPAPELLGGPSVFLFPPKPKDQLMISRTPEVTC
VVVDVSHEDPEVKFNWYVDGVEVHNAKTKPREEQYNSTYRVVSVLTVLHQDWL
NGKEYKCKVSNKALPAPIEKTISKAKGQPREPQVYTLPPSREEMTKNQVSLTCLVK

GFYPSDIAVEWESNGQPENNYKTTPPVLDSDGSFFLYSKLTVDKSRWQQGNVFSCS
VLHEALHNHYTQKSLSLSPGKDDDDKSREPATGSAVPAQSRPCVDCHAFEFMQR
ALQDLRKTAYSLDARTETLLLQAERRALCACWPAGR*.

Note: Cf48 signal peptide [1:19], Fc label [20:238], enzyme cutting site [239:245], and Cf48 secretory peptide [246:307];

### (2) Performing affinity purification on produced amino acids

Performing affinity purification on the Fc-labeled Cf48 by using the protein resin A; as the synthesized Cf48 is at the secretory end, collecting the cell lysate (CLB) and cell culture medium for affinity purification, thereby respectively obtaining the cellular Fc-Cf48 (cFc-Cf48, isolated from cell lysate) and extracellular secretory Cf48 (sFc-Cf48, isolated from cell culture medium); subsequently, verifying the obtained proteins by using the SDS-PAGE gel and Coomassie staining;

### (3) Performing protease digestion and removing Fc labels

Using enterokinase to digest and lyse the enzyme cutting sites, and removing free Fc labels by means of the adhesion of the protein resin A, thereby obtaining the c-Cf48 and s-Cf48 without Fc lables; subsequently, verifying the obtained proteins by using the SDS-PAGE gel and Coomassie staining.

### 4.3.2 Cell culture

The NRK-49F and NRK-52E cell strains are cultured in the DMEM/F12 medium containing 10% fetal bovine serum under the conditions of 95% air and 5% CO₂ at a temperature of 37°C.

### 4.3.3 Stimulating fibroblasts using Cf48 recombinant protein

1) The NRK-49F cells are uniformly planted in a six-well cell plate by using the growth medium (DMEM/F12+10% FBS);
2) When cells adhere to the plate wall and grow steadily to reach a density of 70%, different types of Cf48 recombinant proteins (s-Cf48 and c-Cf48) are diluted with the DMEM/F12 medium containing 0.5% FBS, or the Cf48 at different concentrations is prepared and added to the NRK-49F cells. The NC group is not given TGF-β1, while the TGF-β1 stimulation group is given TGF-β1 at a total concentration of 1ng/mL. The cells are stimulated for 48 hours.

### 4.3.4 Extraction of cell protein: same as 1.3.7.

### 4.3.5 Western blot detection of cell protein: same as 1.3.9.

### 4.3.6 Immunofluorescence detection of cells

1) Planting the NRK-52E cells in a chamber slide at a rate of 5000 cells per well by using the growth medium (DMEM/F12+10% FBS); 2) stimulating the cells: after the cells adhere to the wall of the chamber slide, using the s-Cf48 at concentrations of 0ng/mL, 20ng/mL, 40ng/mL and 80ng/mL to stimulate the NRK-52E cells in the DMEM/F12 medium containing 0.5% FBS in the presence or absence of TGF-β1(1ng/mL) for 48 hours; 3) removing the culture medium from the chamber slide and washing for three times using the pre-cooled PBS; 4) fixing: fixing cells using 4% paraformaldehyde at room temperature for 5 minutes; 5) placing in the PBS buffer on the shaker for 10 minutes and washing for three times; 6) drawing circles using a pap pen: removing the cell partition plates from the chamber slide, wiping off the moisture around the cells, gently drawing circles with the pap pen until the cells are completely surrounded, and placing them on a wet box, wherein the tissues should be protected from being dried during the process; 7) sealing: dropwise adding 50µL of 1% BSA (in PBS) solution to each circle and sealing at room temperature for 1 hour; 8) incubating the primary antibody: diluting the primary antibody at a corresponding concentration (α-SMA-Cy3, 1:5000) in 1% BSA solution and incubating overnight in the dark; 9) placing in the PBS buffer on the shaker for 10 minutes and washing for three times; 10) performing DAPI counter-staining: incubating at room temperature for 5 minutes using the DAPI at a concentration of 1:100; 11) mounting: placing in the PBS buffer on the shaker for 10 minutes, washing for 3 times, drying, dropwise adding the mounting medium, covering with a cover slip, and drying overnight at room temperature in the dark; 12) observing and photographing under the laser scanning confocal microscope.

### 4.3.7 Identification of co-immunoprecipitation and protein mixtures

### (1) hCf48-his-IRES-GFP plasmid transfection and protein expression

The synthesis of hCf48-his-IRES-GFP plasmid is performed by Genewiz. The specific synthesis method comprising the steps of:
a) Evenly planting 293T cells in three 10cm cell culture dishes, and when the cell density reaches 80%, replacing the culture medium with a serum-free medium;
b) Preparing a transfection mixture in a ratio of 67.5 µgDNA, 33.75uL Lipofectamine 3000 and 45uLP3000 per dish; mixing well at room temperature, letting it sit for 15 minutes and then adding cells; after performing the transfection for 12 hours, replacing the used medium with the cell culture medium containing 10% FBS and culturing for another 36 hours; observing the cells under the upright fluorescence microscope, wherein it is observed that the cells are in good condition, the morphology is normal, and about 70% of the cells fluoresce;

### 2) Lysis protein

After washing each dish with the clean pre-cooled PBS for three times, adding 300uL of RIPA protein lysate containing a protease inhibitor; after reacting on ice for 15 minutes, scraping off the cells with a cell scraper, extracting the protein lysate and adding it into a 1.5mL EP tube; after crushing the protein with an ultrasonic cell crusher, centrifuging at high speed (15000rpm, 4°C) for 30 minutes, extracting the protein supernatant, and removing the precipitate;

### 3) Reaction using specific primary antibody and agarose

Adding 400uL of PBS to every 100uL of protein lysate to achieve a total reaction system of 500uL; adding the anti-His antibody to each mixture at a ratio of 1:100, turning upside down and mixing at a temperature of 4°C for 1 hour; subsequently, adding 50uL of protein A/G agarose to each mixture, turning upside down, and mixing overnight at a temperature of 4°C;

### 4) Precipitate of agarose

Centrifuging the mixture reacting overnight at high speed (10000rpm, 4°C) for 1 minute such that the precipitate of agarose is seen; discarding the supernatant and washing for three times with the clean PBS; centrifuging at high speed (10000rpm, 4°C) for 1 minute and then removing the supernatant.

### 5) Protein denaturation

Directly adding 2XLoading buffer to the precipitate after removing the supernatant, boiling at a temperature of 100°C for 15 minutes, centrifuging at high speed (15000rpm, 4°C) for 30 minutes, and extracting the supernatant protein lysate for gel eletrophoresis;

### (6) Protein gel eletrophoresis and Coomassie staining

Taking 100uL of boiled denatured protein lysate and performing electrophoresis in the SDS-PAGE gel; after the protein running into the separation gel is about 1.5cm, stopping performing electrophoresis and performing Coomassie staining; after the protein bands are clearly seen, cutting the gel with an area about 1cm^{2;}

### (7) Protein mixture identification

The cut gel samples are sent to PTM BIO for identification, and the mass spectrum identification of the protein mixture is performed by PTM BIO.

### 4.3.8 Construction of SLC3A2 knockdown cell line by lentivirus transfection

1) The SLC3A2 target gene knockout adopts the CRISPR/Cas9 system. The gene editing of CAS9 selects a BlasticidinS resistant vector and the gene editing of gRNA selects a puromycin resistant vector (PGMLV-GM1: U6-gRNA-EF1a-Puro). The synthesis of the two lentiviruses and a control lentivirus is completed by Genomeditech (Shanghai).

The the designed and synthesized SLC3A2 sgRNA oligomeric single stranded oligo sequence is as follows (rat):
Primer-T2 CACCGAGCAGCAGCAGCGCCCAGC;
Primer-B2 AAACGCTGGGCGCTGCTGCTGCTC.

### 2) NRK-49F cells infected with Cas9-BlasticidinS virus

a) Evenly planting NRK-49F cells in a six-well cell plate at a rate of 10⁵ cells per well by using the cell culture medium (DMEM/F12+10% FBS) ; b) after the cells adhere to the wall and reach a density of 60%, replacing the culture medium with a serum-free medium containing a polybrene transfection auxiliary reagent (working concentration: 6µg/mL); adding the Cas9-Blasticidin S virus solution (MOI 1: 100) to infect the cells, and after 24 hours, replacing the used medium with the cell culture medium (DMEM/F12+10% FBS), wherein the blank control cells are given the the serum-free medium containing the same concentration of polybren for culturing; c) screening for cells infected with blasticidinS: 48 hours after the transfection, passaging the cells in a fresh culture medium containing 25µg/mLBlasticidinS at a working concentration; subsequently, replacing the used medium every 3-4 days with the fresh culture medium containing antibiotics until all cells in the blank control wells are dead;

### (3) NRK-49F with Cas9 working system infected with SLC3A2-sgRNA and CTL-sgRNA

a) Evenly planting the screened transfected NRK-49F cells with the expression of the Cas9 working system in a six-well cell plate at a rate of 10⁵ cells per well by using the cell culture medium (DMEM/F12+10% FBS); b) infecting the cells by SLC3A2-sgRNA and CTL-sgRNA viruses using the same method as 2b); c) screening for transfected cells using puromycin: 48 hours after the transfection, passaging cells in a fresh culture medium containing 8µg/mL puromycin at a working concentration; replacing the used medium every 3-4 days with the fresh culture medium containing antibiotics until all cells in the blank control wells are dead; d) verifying the knockdown efficiency of SLC3A2 in fibroblasts by using the western blot method.

### 4.3.9 Transfection using SLC3A2-siRNA and CTL-siRNA

SLC3A2 siRNA and CTL siRNA are synthesized by GeneBio (Shanghai).

The SLC3A2-siRNA (rat) sequence used is:
Primer-F: GAGGCAUAGCUGGUCUGAA;
Primer-R: UUCAGACCAGCUAUGCCUC.

Transfecting using SLC3A2-siRNA and CTL-siRNA according to the operating process of the Lipofectamine 3000, comprising the steps of:
1) Evenly planting NRK-49F cells in a six-well cell plate at a rate of 10⁵ cells per well by using the cell culture medium (DMEM/F12+10% FBS) ; 2) after the cells adhere to the wall and grow steadily, replacing the cell culture medium with the serum-free medium (1.5mL per well); 3) dissolving the siRNA in DEPC water and preparing a storage solution with a concentration of 20µM; 4) preparing a siRNA working solution in an amount of 500µL Opti-MEM + 10µL siRNA storage solution+6µL Lipofectamine 3000 per well, wherein the working concentration of siRNA is 100nM; adding 500µL of the working solution containing 100nM siRNA to each well; 5) reacting for 48 hours, replacing the working solution, and performing a next step;

### 4.3.10 Cell mitochondrial stress test (Seahorse XF)

1) Planting cells: planting NRK-52E cells into a seahorse XF cell culture microplate at a rate of 5000 cells per well using the cell culture medium (DMEM/F12+10% FBS);
2) Stimulating cells: after the cells adhere to the wall (about 6 hours), replacing the cell culture medium with the serum-free medium, adding corresponding stimuli (NC, 40ng/mL Cf48, 1ng/mL TGF-β1, 1ng/mL TGF-β1+40ng/mL Cf48) to each well according to the grouping of the experiment, and stimulating for a total of 48 hours, wherein the method for transfecting CTL-siRNA and SLC3A2-siRNA is the same as 4.3.9;
(3) Performing the cell mitochondrial stress test according to the operating instructions of the Agilent Seahorse XF, comprising the steps of:
   a) Hydrating a sensor: placing a sensor probe plate in ddH20 and hydrating overnight in a CO₂₋free incubator at a temperature of 37°C;
   b) Calibrating the sensor: after the overnight hydration of the sensor, placing the probe in a calibration solution and letting it sit for 45-60 minutes in a CO₂₋free incubator at a temperature of 37°C for calibration;
   c) Preparing a basic detection solution: adding additives to the RPMI culture medium to prepare a basic detection solution containing 1mmol/L sodium pyruvate, 2mmol/L glutamine and 10mmol/L glucose;
   d) Preparing working solutions: dissolving with the basic detection solution to prepare working solutions: oligomycin, FCCP, and rotenone/antimycin A;
   e) Adding into microplate: respectively adding oligomycin, FCCP, and rotenone/antimycin A to the calibrated sensor probe plate following the sequence of wells A, B, and C, thereby achieving the solutions with final concentrations of 2µM, 1µM, and 0.5µM in the wells during the reaction;
   f) Performing the test following the cell mitochondrial pressure test process.

### 4.4 Experimental results

### 4.4.1 Secretory Cf48 promotes the fibroblast activation

In the process of synthesizing the Cf48 small peptide in vitro, two types of recombinant Cf48 small peptides are obtained by means of the enrichment and purification from the culture medium supernatant and cell lysate of tool cells: sCf48 (secretory Cf48) from the culture medium supernatant and cCf48 (cellular Cf48) from the cell lysate.

To explore the possible effects of the synthesized Cf48 on fibroblasts, two types of recombinant Cf48 are added to rat fibroblasts (NRK-49F) in the presence or absence of TGF-β1 (1ng/mL) for stimulating the fibroblasts for 48 hours. As shown in Figure 4-1, in the absence of TGF-β1, the expression level of α-SMA in fibroblasts stimulated with the secretory Cf48 (sCf48) is increased, while the expression level of α-SMA in fibroblasts stimulated with the cellular Cf48 (cCf48) does not vary significantly. After adding the TGF-β1, the expression level of α-SMA in the control group is increased, indicating that TGF-β1 is capable of promoting the fibroblast activation. In the presence of TGF-β1, the expression level of α-SMA in fibroblasts stimulated by sCf48 is further increased, while there is no significant difference between fibroblasts stimulated with cCf48 and the control group. It is consistent with the proved result that the Cf48 over-expression medium is capable of promoting the fibroblast activation, and it further proves the biological effect of the secretory Cf48 in promoting the fibroblast activation. Therefore, the secretory Cf48 (sCf48) is chosen to obtain from the cell culture medium supernatant in a next experiment.

### 4.4.2 Secretory Cf48 promotes the fibroblast activation in a concentration dependent manner

Furthermore, the sCf48 with different concentrations is added to NRK-49F cells in the presence or absence of TGF-β1, and the expression of α-SMA is detected by using the western blot. As shown in Figure 4-2A, in the absence of TGF-β1, the sCf48 with a concentration below 5ng/ml does not cause any changes in the expression level of α-SMA. Along with the increase of the concentration of sCf48, the expression level of α-SMA is gradually increased. The sCf48 with a high concentration achieves a promoting effect on the fibroblast activation and achieves a significant promoting effect at 40ng/mL. In the presence of TGF-β1, the promoting effect of the sCf48 on the fibroblast activation is significantly enhanced. The expression level of α-SMA is significantly increased when the concentration of the sCf48 is above 5ng/mL, indicating an interaction between the sCf48 and TGF-β1 in promoting fibrosis. Through three independent experiments, as shown in Figures 4-2 B and 4-2C, 40ng/mL sC4orf48 significantly increases the expression of α-SMA in the presence or absence of TGF-β1, indicating a statistical difference. Therefore, the sCf48 with a concentration of 40ng/mL is chosen for further experiments on fibroblasts.

### 4.4.3 Identification of Cf48 co-immunoprecipitated mixture

To further explore the molecular mechanism of Cf48 in promoting fibrosis, the plasmid with Cf48 expression is transfected into 293T cells, and potential molecules capable of interacting with the Cf48 are identified by means of the co-immunoprecipitation and protein mass spectrometry. According to the image of the SDS-PAGE gel in the protein mixture interacting with the Cf48, it is found that the consistency of the three biological experiments is high, and the distribution of the protein mixture is relatively consistent. The target protein Cf48 is detected in all three rounds of mixture identification, indicating a successful transfection of Cf48 and a successful removal during the co-immunoprecipitation. The protein molecules that undergo the co-immunoprecipitation are identified by using the protein mass spectrometry.

The Cf48 binds to the cell-surface receptors and transmits information into the cells, thereby causing a series of biological effects. To screen out the Cf48 receptors, molecules with cytoplasmic domain, transmembrane domain and extracellular domain are taken as the screening criteria. Both TFRC and SLC3A2 proteins are detected in three rounds of mixture identification, which meets the screening criteria and achieves high protein scores. The concentration gradient binding curves of Cf48 with TFRC and SLC3A2 detected by using the surface plasmon resonance (SPR) show that the dissociation equilibrium constants (also known as affinity constants) [KD (M)] of Cf48 with TFRC and SLC3A2d are respectively 5.71e-7 and 3.52e-8.

The TFRC is a transferrin receptor protein 1 that mediates the ferric absorption and ferroptosis. The reduction of the expression of TFRC is capable of alleviating the fibrosis caused by diabetic nephropathy induced by UUO and STZ (Yasumura et al. Hypertension. 2020 Feb; 75(2): 413-421).

The SLC3A2 is a heavy chain of the amino acid transport channel protein located on the cell membrane. It binds to various light chains through disulfide bonds, playing a role in stably anchoring the amino acid transport channel protein to the plasma membrane. The amino acid transmembrane transport plays an important role in cellular energy metabolism and the maintenance of other normal cellular functions. The SLC3A2 is extensively expressed in renal tissues especially in renal tubular epithelial cells and participates in the reabsorption function of renal tubules. The aforesaid suggests that the TFRC and SLC3A2 are involved in the action mechanism of the Cf48, and the TFRC and SLC3A2 may serve as receptors and/or targets of the Cf48.

### 4.4.4 Secretory Cf48 reduces the expression level of SLC3A2 in fibroblasts

To explore the possible effect of the Cf48 on the SLC3A2, the sCf48 at concentrations of 0, 5ng/mL, 10ng/mL and 20ng/mL are respectively added into fibroblasts for stimulating them for 48 hours, thereby observing its effect on the SLC3A2. As shown in Figure 4-3, by means of the western blot, it is found that the sCf48 is capable of decreasing the expression level of SLC3A2 in a concentration dependent manner and reducing the SLC3A2 expression at a lower concentration (5ng/mL).

### 4.4.5 Down-regulation of SLC3A2 expression promotes fibroblast activation

To determine the effect of decreased expression of the SLC3A2 on fibroblasts, the expression of SLC3A2 is silenced by means of two methods: lentiviral infection and siRNA transfection. To observe its effect on fibroblasts, as shown in Figure 4-4A, infection with the SLC3A2-gRNA is capable of partially knocking down the expression of SLC3A2 compared to infection with the control virus. Fibroblasts infected with SLC3A2-sgRNA show an increase in the expression of α-SMA along with the decrease of the expression of SLC3A2.

Subsequently, the expression of SLC3A2 is knocked down by transfecting the control siRNA and SLC3A2-siRNA at different concentrations. As shown in Figure 4-4B, as the concentration of the SLC3A2-siRNA increases, the expression of SLC3A2 gradually decreases. At a concentration of 100nmol/L of the SLC3A2-siRNA, the complete knockout of the SLC3A2 is achieved. Along with the decrease of the expression of the SLC3A2, the expression level of the α-SMA in fibroblasts gradually increases. It can be seen from the aforesaid that a decrease in the expression level of the SLC3A2 increases the expression of the α-SMA in fibroblasts and promotes the fibroblast activation.

### 4.4.6 Down-regulation of SLC3A2 expression enhances the promoting effect of secretory Cf48 on fibroblast activation

The expression of α-SMA in fibroblasts transfected with CTL sgRNA and SLC3A2-sgRNA is observed with or without the sCf48 stimulation. The results are shown in Figure 4-5. The results show that adding the sCf48 to stimulate fibroblasts transfected with CTL-sgRNA leads to a decrease in SLC3A2 expression and an increase in α-SMA expression, which are consistent with the previous results. The fibroblasts infected with SLC3A2-sgRNA have a decreased expression of SLC3A2 and increased expression of α-SMA. The addition of sCf48 further decreases the expression of SLC3A2 and increases the expression of α-SMA. This suggests that the down-regulation of SLC3A2 expression is capable of further enhancing the promoting effect of sCf48 on fibroblast activation, indicating an interaction between the down-regulation of SLC3A2 expression and promoting effect of sCf48 on fibroblast activation.

### 4.4.7 Down-regulation of SLC3A2 expression enhances the promoting effect of TGF-β1 on fibroblast activation

Due to the promoting effect of TGF-β1 on fibroblast activation, to determine whether the down-regulation of SLC3A2 expression has a synergistic effect with the TGF-β1, the TGF-β1 is respectively added or not into fibroblasts transfected with CTL-sgRNA and SLC3A2-sgRNA to observe its promoting effect on fibroblast activation.

As shown in Figure 4-6, when the TGF-β1 is added to fibroblasts transfected with CTL-sgRNA, the expression level of α-SMA is increased, indicating that TGF-β1 itself is capable of promoting the fibroblast activation. Compared with fibrolasts transinfected with CTL-sgRNA, fibroblasts transfected with SLC3A2-sgRNA show a decrease in SLC3A2 expression and an increase in α-SMA expression, which are consistent with the previous results. Knocking down the expression of SLC3A2 and stimulating the fibroblasts with TGF-β1 further increase the expression of α-SMA. It can be seen that the down-regulation of the SLC3A2 expression further enhances the promoting effect of TGF-β1 on the fibroblast activation, suggesting that the decreased SLC3A2 expression enhances the promoting effect of TGF-β1 on fibrosis.

### 4.4.8 Secretory Cf48 promotes tubular cell transdifferentiation

To observe the effects of the secretory Cf48 on other cells, the sCf48 at different concentrations are respectively added to rat renal tubular epithelial cells (NRK-52E) in the presence and absence of TGF-β1 (1ng/mL) for 48 hours of stimulation. The expression of α-SMA in tubular cells is observed by using the cell immunofluorescence staining.

The results show that, in the absence of the TGF-β1, the expression of α-SMA does not exist in tubular cells in the control group, indicating that tubular cells do not undergo the epithelial-mesenchymal transition under normal conditions. After 48 hours of stimulation with sCf48, the α-SMA expression is observed in tubular cells at a concentration of 20ng/mL. As the concentration of the sCf48 increases, the number of tubular cells having the expression of α-SMA is also increased, indicating that the sCf48 is capable of promoting the epithelial-mesenchymal transition of tubular cells in a concentration dependent manner. After 48 hours of stimulation with 1ng/mL TGF-β1, it is observed that the tubular cells have the expression of α-SMA, indicating that the TGF-β1 is capable of promoting the epithelial-mesenchymal transition of tubular cells. Simultaneously adding the sCf48 at different concentrations (1ng/mL, 5ng/mL, 10ng/mL, 15ng/mL and 20ng/mL) is capable of further increasing the expression level of α-SMA in tubular cells, indicating that the sCf48 enhances the epithelial-mesenchymal transition effect of the TGF-β1 on tubular cells in a concentration dependent manner.

### 4.4.9 Cf48 transgenic mice with folic acid nephropathy have significantly increased number of transitioned tubular cells

According to the in vitro observation, the Cf48 is capable of promoting the epithelial-mesenchymal transition of tubular cells. To further determine the promoting effect of the Cf48, in the in vivo models of control mice with folic acid nephropathy and mice with Cf48 over-expression folic acid nephropathy, the Pax8 staining is used to label the tubular cells, and the α-SMA staining is used as a mesenchymal cell marker. Because the Pax8 serves as a nuclear marker for tubular cells, the co-localization of Pax8 and DAPI is used for identifying tubular cells in renal tissues.

Experiments results show that the wild-type mice with folic acid nephropathy have a small amount of Pax8 expression in tubular cells, and the expression of α-SMA is seen in the cytoplasm, indicating that the tubular cells have undergone the epithelial-mesenchymal transition. However, in mice with Cf48 over-expression folic acid nephropathy, the number of tubular cells having expression of both Pax8 and α-SMA is significantly increased. This indicates that the up-regulation of the Cf48 expression in vivo is capable of promoting the epithelial-mesenchymal transition of tubular cells in a disease state, which is consistent with the results of in vitro experiments.

### 4.4.10 Secretory Cf48 reduces the expression level of SLC3A2 in tubular cells

To determine the effect of the secretory Cf48 on the expression level of SLC3A2 in tubular cells, the sCf48 at different concentrations are added into NRK-52E cells for stimulation for 5 days to observe the variation of SLC3A2 expression level. By means of the western blot, it is found that the sCf48 is capable of reducing the expression level of SLC3A2 in a concentration dependent manner and achieving a more significant effect at a concentration of 40ng/mL. Meanwhile, as shown in Figure 4-7, it is observed that, as the concentration of the sCf48 increases, the expression level of α-SMA is gradually increased along with the decrease of the SLC3A2 expression, which is consistent with the previous results.

### 4.4.11 Down-regulation of SLC3A2 expression promotes tubular cell epithelial-mesenchymal transition

To explore the effect of down-regulating the SLC3A2 on the epithelial-mesenchymal transition of tubular cells, the control siRNA and SLC3A2-siRNA are transfected into NRK-52E cells to silence the expression of SLC3A2. As shown in Figure 4-8, after the cells are transfected with the SLC3A2-siRNA, the expression of SLC3A2 is decreased, the expression of E-cadherin serving as a marker of tubular cells is decreased, and the expression of α-SMA serving as a marker of interstitial cells is increased. The above results indicate that the decrease in the SLC3A2 expression leads to the epithelial-mesenchymal transition of tubular cells.

### 4.4.12 Down-regulation of SLC3A2 expression leads to inclined mitochondrial function of tubular cells

The expression of SLC3A2 in renal tubular epithelial cells is knocked down for detecting the mitochondrial function of the cells. After treating the rat renal tubular cells with CTL-siRNA and SLC3A2-siRNA for 48 hours, the mitochondrial function is detected by using the Seahorse method. The results are shown in Figure 4-9. The OCR curve of tubular cells with knocked-down SLC3A2 expression shows that the mitochondrial function significantly declines. The basal respiratory rate, maximum respiratory rate, ATP production capacity and reserve respiratory capacity that are indicators for evaluating the mitochondrial function are significantly decreased in tubular cells with reduced SLC3A2 expression. The SLC3A2 participates in the formation of amino terminal transport channels, and normal amino acid metabolism is a necessary condition for energy supply. Due to the high energy demand and dependence on aerobic metabolism of renal tubular cells, the decreased expression of the SLC3A2 leads to the abnormal energy metabolism ability of tubular cells.

### 4.4.13 Secretory Cf48 leads to inclined mitochondrial function of tubular cells

The sCf48 is directly added into tubular cells for detecting the mitochondrial function in the presence or absence of TGF-β1. It can be seen from the results that, compared with the normal control (NC), the OCR curve of tubular cells stimulated with sCf48 shows that the mitochondrial function is significantly declined, and the basal respiratory rate, maximum respiratory rate, ATP production capacity and reserve respiratory capacity are all significantly decreased, indicating a statistical difference. The OCR curve of tubular cells stimulated by TGF-β1 shows that the mitochondrial function is significantly declined compared to that of the NC and the level is basically the same as that of tubular cells stimulated with sCf48, indicating that the sCf48 and TGF-β1 have similar effects on the declination of mitochondrial function of tubular cells. According to the OCR curve, under the stimulation of the TGF-β1, the addition of the sCf48 further declines the mitochondrial function of tubular cells, and the basal respiratory rate, ATP production capacity and reserve respiratory capacity are significantly decreased. As shown in Figure 4-10, the above results indicate that the sCf48 leads to a decline in mitochondrial function of tubular cells and further exacerbates the mitochondrial dysfunction caused by the TGF-β1.

### 4.4.14 Decreased expression of SLC3A2 in mice with folic acid nephropathy

The effect of the Cf48 small peptide exerting through the SLC3A2 is observed in in-vitro experiments. To further explore the relationship between the Cf48 small peptide and the SLC3A2 in in-vivo experiments, the immunofluorescence staining of the SLC3A2 is performed on renal tissues of mice with folic acid nephropathy and normal control mice to observe the expression of the SLC3A2 in vivo. The experimental results show that high expression of the SLC3A2 is found in the glomeruli and tubules of normal control mice, while in renal tissues of the FAN mice, the expression level of the SLC3A2 is greatly reduced in the glomeruli and tubules.

### 4.4.15 Down-regulation of SLC3A2 expression exacerbates fibrosis in Cf48 over-expression mice

To observe the relationship between the expression of the Cf48 and SLC3A2 as well as that between the expression of Cf48 and the fibrosis degree in vivo, the expression of the SLC3A2 and the fibrosis index α-SMA are detected in the model of Cf48 transgenic mice with folic acid nephropathy. From the results of the western blot shown in Figure 4-11, it can be seen that, in normal control mice, the expression of the Cf48 is increased in Tg mice compared to WT mice, and meanwhile, the expression of the SLC3A2 is decreased and the expression of the α-SMA is slightly increased. In the FAN model of WT mice, an increase in the expression level of the Cf48 is observed, while the expression level of the SLC3A2 is greatly decreased compared to normal control mice. Meanwhile, the expression level of the α-SMA is significantly increased, which is consistent with the previous results. Compared with the WT-FAN mice, the Tg-FAN mice show a further increase in the Cf48 expression. Meanwhile, the expression of the SLC3A2 is significantly decreased and the expression of the α-SMA is greatly increased.

### 4.4.16 Increased expression of SLC3A2 in Cf48 gene knockout mice reduces fibrosis degree

The expression levels of the SLC3A2 and fibrosis index α-SMA in the UUO model of Cf48 gene knockout mice are further detected. As shown in Figure 4-12, in the sham group of mice, the expression of the SLC3A2 is slightly increased in the Cf48-KO mice compared to the WT mice. After undergoing the UUO surgery, a significant decrease in the expression of the SLC3A2 and a significant increase in the expression of the α-SMA are observed in the WT mice. Compared with the WT-UUO mice, the KO-UUO mice show an increase in the expression of the SLC3A2 and a decrease in the expression of the α-SMA, resulting in the reduced degree of the renal fibrosis in the KO-UUO mice.

### 4.4.17 TFRC mediates Cf48 to reduce SLC3A2 expression and promote fibrosis

To further determine the role of the TFRC in the biological effects caused by the Cf48, the TFRC is knocked down by adopting the siRNA technology in rat renal fibroblasts. As shown in Figure 4-13, the results show that knocking down the TFRC greatly reduces the expression of the TGF-β1-induced α-SMA promoted by the Cf48, indicating that the profibrotic response caused by the Cf48 is significantly weakened and the TFRC is capable of serving as the receptor mediating the profibrotic response of the Cf48.

4.5 The mice with progressive diabetic nephropathy induced by streptozotocin obtained in this embodiment are treated with the Cf48 locked nucleic acid. After 4 weeks of STZ induction in mice with diabetic nephropathy, the mice are intraperitoneally injected with the LNA-CTL or LNA-c4orf48 (20mg/kg) once a week for 6 consecutive weeks, and then the mice are euthanized. Age-matched normal mice are also treated with the LNA-CTL or LNA-c4orf48 (20mg/kg) as controls. The results show that the Cf48 locked nucleic acid treatment knocks down the expression of the endogenous Cf48 in mice and delays the occurrence and development of the progressive diabetic nephropathy in mice. It can be seen that the kidney injury may be inhibited or treated by means of inhibiting the expression of the Cf48 in a target object's kidney. It achieves the targeted inhibition of the production of Cf48 in the target object's kidney, so that the drug treatment effect is realized.

### 4.6 Conclusion

The maintenance of normal renal function depends on the complex and precise interactions among numerous renal parenchymal cells. This delicate balance among cells primarily relies on the action of communication cellular factors. When this balance is disrupted by factors such as metabolic disorders, hemodynamic abnormalities and toxic factors, renal tubular cells are often the first to be hit, resulting in the abnormal secretion of communication cellular factors. If the injuring factors continuously exist, the progressive action of communication cellular factors irreversibly injures the renal parenchymal cells and ultimately leads to the renal fibrosis.

It is confirmed by in-vitro experiments that the Cf48 small peptide secreted by renal tubular cells under an injured condition is capable of serving as a communication cellular factor. The secretory Cf48 (sCf48) acts on fibroblasts through the paracrine channel, thereby increasing their expression of α-SMA and activating them into myofibroblasts capable of producing the ECM. The secretory Cf48 also promotes the epithelial-mesenchymal transition (EMT) of tubular cells through the autocrine channel, thereby accelerating the progression of fibrosis. Through the in-vivo experiments, it is also observed that the up-regulation of the Cf48 expression is capable of greatly increasing the number of transitioned tubular cells in mice in a disease state.

The action mechanism of the Cf48 small peptide is similar to that of the TGF-β1 that has been extensively researched. Both of them are capable of being produced by injured tubular cells and acting on renal parenchymal cells through the humoral communication for promoting the fibrosis. The TGF-β1 is expressed in many tissues and cells and participates in numerous biological signal channels. Many preclinical researches have shown that the treatment aiming at the TGF-β1 channel achieves an anti-fibrosis effect. However, due to its participation in numerous signal channels, it also has other adverse effects on the kidney and cardiovascular system while combating the fibrosis. The TGF-β1 signal transduction blocker does not achieve benefits on the renal function protection in clinical experiments of focal segmental glomerulosclerosis and diabetic nephropathy. Therefore, the clinical development of anti-TGF-β1 neutralizing antibody in renal diseases has been suspended. Differing from the TGF-β1, the Cf48 small peptide has a small molecular weight and high specificity for targeted antibodies. No significant phenotypic abnormalities have been found in the Cf48 gene knockout mice. Because the Cf48 small peptide plays a role similar to the TGF-β1 in activating fibroblasts and promoting the epithelial-mesenchymal transition (EMT) in tubular cells, the application of targeted treatment drugs aiming at the Cf48 has great potential.

Through the co-immunoprecipitation protein mass spectrometry identification, it is found that protein molecules interacting with the Cf48 include the TFRC and SLC3A2. The KD values of Cf48 and TFRC, as well as Cf48 and SLC3A2 obtained by the SPR detection are respectively 5.71e-7M and 3.52e-8M. The TFRC and SLC3A2 are receptors and/or targets of the Cf48, and the Cf48 small peptide is capable of reducing the expression of the SLC3A2 in fibroblasts and tubular epithelial cells. The SLC3A2 belongs to the SLC3 family of proteins, which form the heavy chains of amino acid transport channels. Amino acids are essential raw materials for the normal functioning of all living cells and organisms, and there are therefore special transport channels that mediate the transport of amino acids across the plasma membrane. The SLC3 and SLC7 families together form the heteromeric amino acid transporters (HAT). The HAT consists of light and heavy chains, wherein the SLC3 family members form the heavy chains and the SLC7 family members form the light chains. The SLC3A2 binds to various light chains through disulfide bonds, thereby anchoring the HAT to the plasma membrane to maintain the stability of the amino acid transport channel structure. The HAT is an exchange channel for many amino acid substrates. Therefore, it is crucial for the reabsorption function of the kidneys and small intestine, as well as for redox reactions of cells. The LC3A2 serving as a heavy chain for amino acid transport channels plays an important role in cellular energy supply and tissue metabolic balance. Through in-vivo experiments, it is found that the expression of the SLC3A2 is greatly reduced in renal tissues of mice with folic acid nephropathy. Through in-vitro experiments, it is found that silencing the expression of the SLC3A2 in fibroblasts is capable of activating them into myofibroblasts expressing α-SMA. Similarly, silencing the expression of the LC3A2 in renal tubular epithelial cells leads to the EMT in tubular cells. Therefore, the SLC3A2 itself is capable of participating in the occurrence and development of fibrosis. Then, through what mechanism does the SLC3A2 mediate the fibrotic changes in renal parenchymal cells?

The kidney is a high energy consuming organ in normal physiological processes, and its significant energy demand is mainly from the reabsorption of the proximal tubules, including Na⁺, K⁺, glucose and amino acids in the original urine, wherein the transport of the sodium requires a close coordination between the energy supply and demand. Research results show that the energy metabolism defects promote the occurrence and development of renal fibrosis, and the progression of CKD is related to abnormal fatty acid oxidation (FAO) and impaired glucose metabolism. A lot of researches have confirmed that the insufficient energy supply to tubular cells leads to difficulties in maintaining their tubular morphology and function, resulting in the epithelial-mesenchymal transition. Silencing the enzyme AMPK (AMP-activated protein kinase) associated with the energy metabolism in tubular cells is capable of inhibiting the energy metabolism, thereby promoting the EMT and exacerbating the fibrosis. Research results show that, when the expression of the amino acid channel SLC3A2 is inhibited, the mitochondrial function of tubular cells significantly declines, resulting in the insufficient cellular energy supply and leading to the occurrence of the EMT. The Cf48 small peptide is capable of inhibiting the mitochondrial function of tubular cells and further exacerbating the TGF-β1-mediated decline in mitochondrial function, thereby inhibiting the energy metabolism in tubular cells. Because the Cf48 small peptide is capable of decreasing the SLC3A2 expression, it can be concluded that the Cf48 small peptide is capable of promoting the occurrence of the EMT and the progression of fibrosis by reducing the expression of the amino acid channel SLC3A2 and inhibiting the energy metabolism in tubular cells.

Experimental results show that the Cf48 small peptide also leads to a decrease in the SLC3A2 expression in fibroblasts, and as the expression level of SLC3A2 in fibroblasts decreases, the expression level of the α-SMA gradually increases. Therefore, the Cf48 small peptide is capable of activating the fibroblasts by causing a decrease in the SLC3A2 expression.

In in-vivo experiments, it is observed that the expression of the SLC3A2 is greatly decreased in the model of mice with renal fibrosis, and through the genetic engineering of mice, it is observed that, as the expression of the Cf48 is up-regulated, the expression level of the SLC3A2 is decreased, and the degree of fibrosis in mice is increased. Similarly, as the expression of the Cf48 is down-regulated and the expression of the SLC3A2 is increased, the degree of fibrosis in mice is decreased.

In conclusion, in this embodiment, it is found that the Cf48 small peptide is capable of activating fibroblasts and promoting the EMT in tubular cells, thereby exacerbating the renal fibrosis. The Cf48 small peptide also reduces the expression of the amino acid channel SLC3A2 in fibroblasts and promotes the fibroblast activation. The Cf48 small peptide is capable of reducing the expression of the amino acid channel SLC3A2 in tubular cells, inhibiting the energy metabolism in tubular cells, and promoting the occurrence of the EMT.

Based on the above analysis, it can be concluded that:
a. The secretory Cf48 promotes the fibroblast activation and the epithelial-mesenchymal transition (EMT) of renal tubular epithelial cells;
b. The secretory Cf48 reduces the expression level of the SLC3A2 in fibroblasts and renal tubular epithelial cells;
c. The secretory Cf48 is capable of inhibiting the amino acid channel SLC3A2 and promoting the fibroblast activation;
d. The secretory Cf48 is capable of inhibiting the amino acid channel SLC3A2, thereby leading to a decline in mitochondrial function and promoting the EMT in tubular cells;
e. In in-vivo experiments, it is found that the up-regulation of the Cf48 expression is capable of leading to a decrease in the SLC3A2 expression and an exacerbation of fibrosis, and the down-regulation of the Cf48 expression is capable of leading to the increased expression of the SLC3A2 and the reduced degree of fibrosis.

### Embodiment 4

In this embodiment, a use of the Cf48 small peptide as a drug intervention target in the preparation of a drug for treating kidney injury is provided.

The kidney injury treatment drug is used to treat the kidney injury or delay the development of kidney injury by the targeted inhibition or elimination of the expression of the Cf48 small peptide.

More specifically, the kidney injury treatment drug is capable of inhibiting the expression of the Cf48 small peptide, thereby preventing the amino acid channel SLC3A in fibroblasts from being inhibited by the Cf48 small peptide such that the fibroblast activation is avoided.

The drug for treating kidney injury inhibits the expression of the Cf48 small peptide in a targeted mode, thereby avoiding the induction of the EMT and promotion of the kidney fibrosis caused by Cf48 inhibiting the amino acid channel SLC3A2 and the energy metabolism in tubular cells.

The experimental results also show that, by means of the targeted inhibition of the expression of the Cf48 small peptide, the drug for treating kidney injury is capable of preventing the synergistic effect of the Cf48 and other factors (e.g.,TGF-β1) that promote the formation of renal fibrosis.

It is worth mentioning that, the drug for inhibiting the expression of the Cf48 small peptide may be locked nucleic acid or other drugs capable of achieving the inhibition of the Cf48, which are briefly described herein.

### Embodiment 5

A system for evaluating the kidney injury condition on the basis of Cf48, comprising:
A data acquisition module, configured to acquire at least a content of the Cf48 in a biological sample of a target object to be detected, and
An evaluation module, configured to compare the content of the Cf48 in the biological sample with control parameters and give a level result corresponding to the kidney injury condition of the target object to be detected according to the comparison result.

More specifically, the evaluation module is provided with a parameter storage unit and an analysis unit, wherein the parameter storage unit stores a first reference value and a second reference value.

The analysis unit compares the content of the Cf48 in the detected biological sample transmitted by the data acquisition module with the first reference value and the second reference value. When the content of the Cf48 small peptide in the detected biological sample is greater than or equal to the first reference value, the analysis unit gives a first-level result, and the first-level result indicates that the kidney injury condition is serious and needs to be intervened.

When the content of the Cf48 small peptide in the detected biological sample is lower than the second reference value, the analysis unit gives a low-level result, and the low-level result indicates that the kidney injury condition is slight and an intervention is unnecessary, wherein the first reference value is greater than the second reference value. It is worth mentioning that, the first reference value, the second reference value, as well as a numerical range of them may be flexibly selected and set according to a specific use and occasion.

Further, the data acquisition module is configured to acquire whether the TGF-β1 exists in the biological sample to be detected;

The analysis unit compares the content of the Cf48 in the detected biological sample transmitted by the data acquisition module with the first reference value and the second reference value. When the content of the Cf48 small peptide in the detected biological sample is lower than the first reference value and greater than the second reference value, the analysis unit obtains a result of whether the TGF-β1 exists in the to-be-detected biological sample from the data acquisition module, and when the TGF-β1 exists in the biological sample to be detected, the analysis unit gives a second-level result, and the second-level result indicates that the kidney injury condition is serious and needs to be intervened.

The system for evaluating the kidney injury condition on the basis of Cf48 evaluates the kidney injury condition and gives a level result by acquiring the content of Cf48 in the biological sample of the target object to be detected, which achieves the benefits of convenient detection and accurate results.

By means of the system, it is possible to follow the registration and evaluation of various uses including the progression of nephropathy, kidney injury degree, target object prognosis prediction, renal fibrosis degree, renal fibrosis activity and kidney patient treatment effect monitoring. According to different requirements, a corresponding first reference value and a corresponding second reference value are set, so that the evaluation for multiple purposes is achieved.

### Embodiment 6

In this embodiment, the evaluation on the renal fibrosis activity is performed by means of the system for evaluating the kidney injury condition on the basis of Cf48 in embodiment 5. The first reference value and the second reference value stored in the parameter storage unit are respectively a first threshold and a second threshold.

The analysis unit compares the content of the Cf48 in the detected biological sample transmitted by the data acquisition module with the first threshold and the second threshold. When the content of the Cf48 small peptide in the detected biological sample is greater than or equal to the first threshold, the analysis unit prompts that the renal fibrosis activity is high and gives a first-level result. The first level result indicates that the renal fibrosis activity is high and needs to be intervened.

When the content of the Cf48 small peptide in the detected biological sample is lower than the second threshold, the analysis unit prompts that the renal fibrosis activity is weak and gives a low-level result. The low-level result indicates that the renal fibrosis activity is low, and the renal fibrosis does not exacerbate, wherein the first threshold is greater than the second threshold, and the first threshold and the second threshold may be set according to the purpose and object in actual use.

In the system for evaluating the kidney injury condition on the basis of Cf48, the data acquisition module further acquires whether the TGF-β1 exists in the biological sample to be detected.

When the content of the Cf48 small peptide in the detected biological sample is lower than the first threshold and greater than the second threshold, the analysis unit obtains a result of whether the TGF-β1 exists in the to-be-detected biological sample from the data acquisition module. When the TGF-β1 exists in the biological sample to be detected, the analysis unit prompts that the renal fibrosis activity is strong, and the analysis unit gives a second-level result. The second-level result indicates that the renal fibrosis activity is high and needs to be intervened.

The system for evaluating the kidney injury condition on the basis of Cf48 evaluates the renal fibrosis degree and gives a level result by acquiring the content of Cf48 in the biological sample of the target object to be detected, which achieves the benefits of convenient detection and accurate results.

### Embodiment 7

Below are several use scenarios of the marker of the present invention.

Li, 48 years old, male, has no obvious abnormal sensation in the kidney, failing to meet the clinical indications for kidney disease examination and treatment. In routine physical examinations, the detected content of Cf48 in the blood sample is below 0.1ng/mL, indicating that there is no risk of kidney injury.

Wang, 53 years old, male, has no obvious abnormal sensation in the kidney, failing to meet the clinical indications for kidney disease examination and treatment. In routine physical examinations, the detected content of Cf48 in the blood sample is higher than 10ng/mL but lower than 30ng/mL, indicating the presence of mild kidney injury. After a period of targeted treatment to reduce the content of Cf48, the re-detected content of Cf48 is significantly decreased, indicating that the treatment is effective.

Chen, 49 years old, female, has a high level of renal urinary albumin/creatinine but fails to meet the clinical indications for renal biopsy. The detected content of Cf48 in the blood sample is as high as 32ng/mL, indicating a severe kidney injury and an exacerbation of the kidney disease. The renal fibrosis degree is high, and the renal fibrosis activity is also high, which need to be inhibited as soon as possible. It indicates that the clinical treatment is necessary, and the treatment effect may be determined by detecting the content of Cf48 in the blood sample after a period of treatment.

### Embodiment 8

To verify the specific expression of C4orf48 in kidney disease and renal fibrosis, in this embodiment, the ELISA method is adopted to detect the C4orf48 in serum samples of healthy individuals (those who have undergone physical examinations at Zhongshan Traditional Chinese Medicine Hospital and Yichang Central Hospital and have normal results). The reagent is purchased from CUSABIO and the detection is performed according to the operating instructions. A standard curve is constructed by using standard samples at six different concentrations in Table 1 for obtaining the conversion formula between the optical density (OD) and concentration. The measured OD value of the samples are substituted into the formula to calculate the sample concentration value. The mean value obtained from the detected whole blood samples of 178 healthy individuals is 2.48ng/ml, and the standard deviation SD is 1.01ng/ml. Table 2 shows the detected results of samples 1-10 and 90-99 out of 178 samples. From the results, it can be seen that the content of Cf48 in the serum of healthy individuals is very low. By detecting the content of cf48, it is possible to distinguish between healthy individuals and patients with kidney injury.

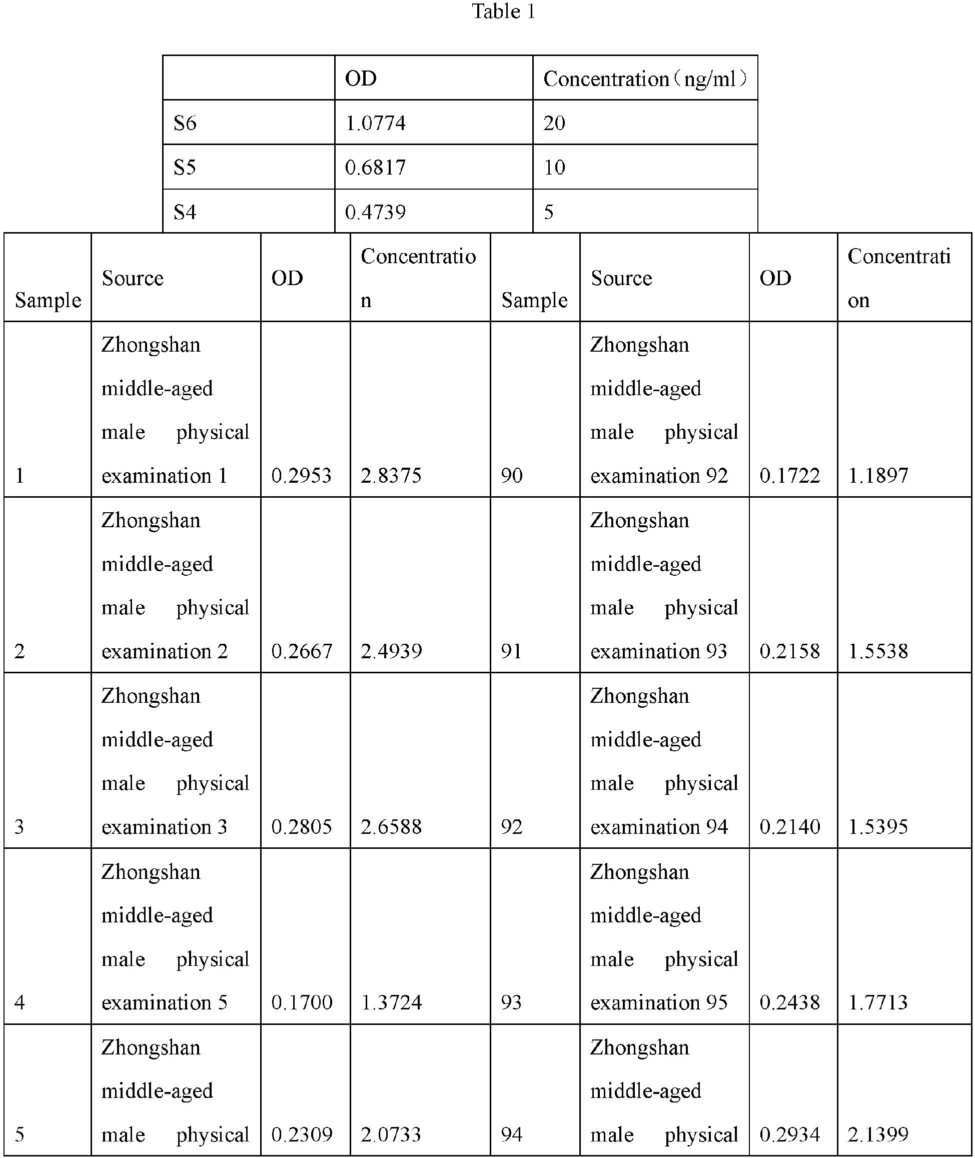

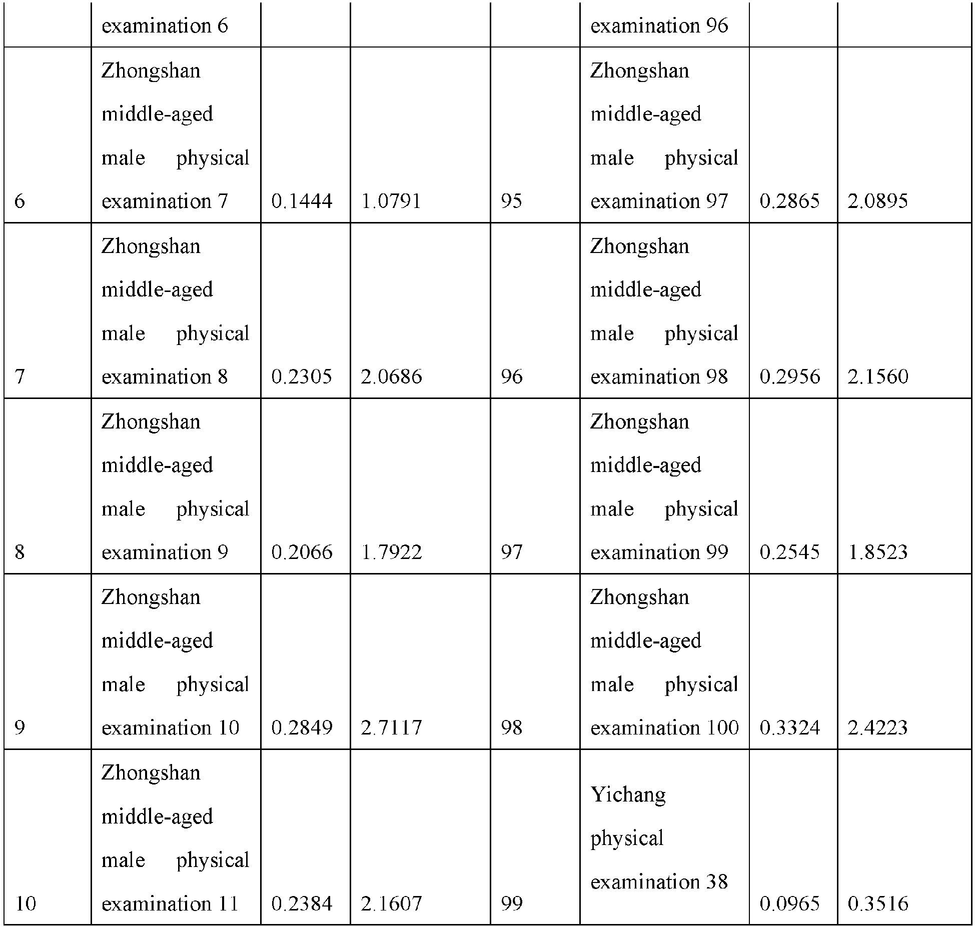

### Embodiment 9

To verify the specificity of C4orf48 in serum in the diagnosis of kidney disease and renal fibrosis, the same ELISA method as in embodiment 8 is adopted to detect the content of Cf48 in serum of lung disease patients having a tendency to deteriorate into pulmonary fibrosis, including 57 cases of interstitial lung disease, 33 cases of pulmonary infection, severe pneumonia, lung cancer, chronic obstructive pulmonary disease and other lung diseases. Table 3 shows the detection results of randomly-selected 10 cases of interstitial lung disease and 10 cases of other lung disease.

According to the detection results, the mean value of Cf48 in serum samples from patients with lung injury is 0.95ng/ml, and the standard deviation is 0.62ng/ml. The experimental results show that the content of Cf48 fail to represent the lung injury condition. It proves that the Cf48 is capable of serving as a specific marker for kidney diseases.

**Table 3**

| Sample | OD | Concentration |
|---|---|---|
| Interstitial lung disease 4 | 0.3021 | 1.9188 |
| Interstitial lung disease 8 | 0.2983 | 1.8940 |
| Interstitial lung disease 19 | 0.3499 | 2.2376 |
| Interstitial lung disease 25 | 0.1587 | 0.9982 |
| Interstitial lung disease 27 | 0.3265 | 2.0797 |
| Interstitial lung disease 30 | 0.2024 | 1.2815 |
| Interstitial lung disease 34 | 0.0898 | 0.4998 |
| Interstitial lung disease 35 | 0.1117 | 0.6707 |
| Interstitial lung disease 51 | 0.0620 | 0.2439 |
| Interstitial lung disease 56 | 0.0584 | 0.2047 |
| Pulmonary infection 1 | 0.2553 | 1.6175 |
| Pulmonary infection 2 | 0.1721 | 1.0863 |
| Pulmonary infection 3 | 0.1893 | 1.1976 |
| Pulmonary infection 4 | 0.2937 | 1.8641 |
| Pulmonary infection 5 | 0.0614 | 0.2375 |
| Pulmonary infection 6 | 0.0635 | 0.2597 |
| Pulmonary infection 7 | 0.1201 | 0.7322 |
| Pulmonary infection 8 | 0.1195 | 0.7279 |
| Pulmonary infection 9 | 0.1315 | 0.8133 |
| Pulmonary infection 10 | 0.2161 | 1.3687 |

The above embodiments are only used to illustrate the technical solution of the present invention and not to limit the scope of the present invention. Although the present invention has been described in detail with reference to the preferred embodiments, for those skilled in the art, the technical solution of the present invention may be modified or equivalently replaced without departing from the spirit and scope of the technical solution of the present invention.

## Claims

1. A use of a Cf48 small peptide as a marker for kidney injury condition, wherein the sequence encoding the the Cf48 small peptide is SEQ ID NO: 1.

2. The use of the Cf48 small peptide as a marker for kidney injury condition of claim 1, wherein the Cf48 small peptide serves as a marker for kidney disease diagnosis, or the Cf48 small peptide serves as a marker for kidney injury degree determination, or the Cf48 small peptide serves as a marker for prognosis prediction, or the Cf48 small peptide serves as a marker for renal fibrosis degree determination, or the Cf48 small peptide serves as a marker for renal fibrosis activity determination, or the Cf48 small peptide serves as one or more markers for monitoring treatment effect on patients with kidney disease.

3. A use of the Cf48 small peptide as a marker for kidney injury condition in a diagnosis kit.

4. The use of the Cf48 small peptide as a marker for kidney injury condition in a diagnosis kit of claim 3, wherein by detecting the expression level of the Cf48 small peptide in a biological sample, the expression level higher than a reference level indicates the presence of kidney injury, and wherein the sample may be kidney tissues, serum, plasma or whole blood.

5. The use of the Cf48 small peptide as a marker for kidney injury condition in a diagnosis kit of claim 3 or 4, wherein whether a target object has a kidney disease is determined by detecting the expression level of the Cf48 small peptide in the biological sample, or the kidney injury degree is determined by detecting the expression level of the Cf48 small peptide in the biological sample, or the prognosis prediction is performed by detecting the expression level of the Cf48 small peptide in the biological sample, or the renal fibrosis degree is determined by detecting the expression level of the Cf48 small peptide in the biological sample, or the renal fibrosis activity is determined by detecting the expression level of the Cf48 small peptide in the biological sample, or at least one method for monitoring the treatment effect on patients with kidney disease is performed by detecting the expression level of the Cf48 small peptide in the biological sample.

6. The use of the Cf48 small peptide as a marker for kidney injury condition in a diagnosis kit of claim 5, wherein whether there is renal tubular injury is determined based on the content of the Cf48 small peptide in a detected biological sample.

7. The use of the Cf48 small peptide as a marker for kidney injury condition in a diagnosis kit of claim 5, wherein the higher the content of the Cf48 small peptide in the detected biological sample, the higher the kidney injury degree, wherein the higher the content of the Cf48 small peptide in the detected biological sample, the more severe the prognosis prediction result, namely, the higher the development ability of the kidney disease, and wherein the higher the content of the Cf48 small peptide in the detected biological sample, the higher the renal fibrosis activity, namely, the higher the renal fibrosis degree.

8. The use of the Cf48 small peptide as a marker for kidney injury condition in a diagnosis kit of claim 7, wherein the content of the Cf48 small peptide in the detected biological sample is compared with a first threshold, wherein when the content of the Cf48 small peptide in the detected biological sample is greater than or equal to the first threshold, it indicates that the renal fibrosis activity is high and an intervention is needed, wherein when the content of the Cf48 small peptide in the detected biological sample is lower than the first threshold and greater than a second threshold, whether TGF-β1 exists in the biological sample is detected, and when TGF-β1 is found in the detected biological sample, it indicates that the renal fibrosis activity is high, wherein when the content of the Cf48 small peptide in the detected biological sample is lower than the second threshold, it indicates that the renal fibrosis activity is low and the development ability of kidney fibrosis is poor, and wherein the first threshold is greater than the second threshold.

9. A use of the Cf48 small peptide as a drug intervention target in the preparation of a drug for treating kidney injury.

10. The use of the Cf48 small peptide as a drug intervention target in the preparation of a drug for treating kidney injury of claim 9, wherein the drug for treating kidney injury treats the kidney injury or delays the development of kidney injury by the targeted inhibition or elimination of the expression of the Cf48 small peptide.

11. The use of the Cf48 small peptide as a drug intervention target in the preparation of a drug for treating kidney injury of claim 10, wherein the drug for treating kidney injury inhibits the expression of the Cf48 small peptide in a targeted mode, thereby preventing the amino acid channel SLC3A in fibroblasts from being inhibited by the Cf48 small peptide such that the fibroblast activation is avoided.

12. The use of the Cf48 small peptide as a drug intervention target in the preparation of a drug for treating kidney injury of claims 9-11, wherein the drug for treating kidney injury inhibits the expression of the Cf48 small peptide in a targeted mode, thereby avoiding the induction of the EMT and promotion of the kidney fibrosis caused by Cf48 inhibiting the amino acid channel SLC3A2 and the energy metabolism in tubular cells, and wherein the drug for treating kidney injury inhibits the binding of the Cf48 small peptide and transferrin receptor protein 1, thereby avoiding the induction of EMT and promotion of renal fibrosis caused by Cf48 activating downstream signal channels of TFRC.

13. The use of the Cf48 small peptide as a drug intervention target in the preparation of a drug for treating kidney injury of claim 12, wherein by means of the targeted inhibition of the expression of the Cf48 small peptide, the drug for treating kidney injury is capable of preventing the synergistic effect of the Cf48 and other factors that promote the formation of renal fibrosis.

14. A system for evaluating the kidney injury condition on the basis of Cf48, comprising:
a data acquisition module configured to acquire at least a content of the Cf48 in a biological sample of a target object to be detected, and
an evaluation module configured to compare the content of the Cf48 in the biological sample with control parameters and give a level result corresponding to the kidney injury condition of the target object to be detected according to the comparison result.

15. The system for evaluating the kidney injury condition on the basis of Cf48 of claim 14, wherein the evaluation module is provided with a parameter storage unit and an analysis unit, wherein the parameter storage unit stores a first reference value and a second reference value, wherein the analysis unit compares the content of the Cf48 in the detected biological sample transmitted by the data acquisition module with the first reference value and the second reference value, wherein when the content of the Cf48 small peptide in the detected biological sample is greater than or equal to the first reference value, the analysis unit gives a first-level result, and the first-level result indicates that the kidney injury condition is serious and needs to be intervened, wherein when the content of the Cf48 small peptide in the detected biological sample is lower than the second reference value, the analysis unit gives a low-level result, and the low-level result indicates that the kidney injury condition is slight and an intervention is unnecessary, and wherein the first reference value is greater than the second reference value.

16. The system for evaluating the kidney injury condition on the basis of Cf48 of claim 15, wherein the data acquisition module is configured to acquire whether the TGF-β1 exists in the biological sample to be detected, wherein the analysis unit compares the content of the Cf48 in the detected biological sample transmitted by the data acquisition module with the first reference value and the second reference value, wherein when the content of the Cf48 small peptide in the detected biological sample is lower than the first reference value and greater than the second reference value, the analysis unit obtains a result of whether the TGF-β1 exists in the to-be-detected biological sample from the data acquisition module, and wherein when the TGF-β1 exists in the biological sample to be detected, the analysis unit gives a second-level result, and the second-level result indicates that the kidney injury condition is serious and needs to be intervened.
